# EUROPÄISCHE PATENTSCHRIFT

(11) **EP 2 138 127 B1**
(45) Veröffentlichungstag und Bekanntmachung des Hinweises auf die Patenterteilung: **20.02.2013**
(21) Anmeldenummer: 08405165.5
(22) Anmeldetag: 27.06.2008
(51) Int. Cl.: A61C 19/00, A61G 15/00, A61L 2/02, A61L 2/16

(54) **Reinigungsapparatur**
Cleaning device
Appareil de nettoyage

(43) Veröffentlichungstag der Anmeldung: 30.12.2009
(73) Patentinhaber: Oro Clean Chemie AG, 8320 Fehraltorf (CH)
(72) Erfinder: Ionidis, Georgios, 8050 Zürich (CH)

(56) Entgegenhaltungen:
- EP-A- 0 111 249
- EP-A- 0 594 202
- WO-A-93/17726
- DE-A1- 19 913 962
- US-A- 6 117 285

## Beschreibung

### Technisches Gebiet

Die Erfindung betrifft einen Reinigungsapparat zum Reinigen und/oder Desinfizieren wenigstens eines medizintechnischen Geräts, insbesondere eines zahnmedizinischen Hohlinstruments wie ein Hand- und/oder ein Winkelstück, mit einer Reinigungsflüssigkeit, wobei der Reinigungsapparat wenigstens eine Steuereinheit und eine Zufuhrvorrichtung zur Zuleitung der Reinigungsflüssigkeit in einen Aufnahmebereich und/oder eine Kupplungsvorrichtung für das zu reinigende medizintechnische Gerät umfasst und wobei die Zufuhrvorrichtung über wenigstens eine erste Sensoreinheit zur Bestimmung eines physikalischen Zustands der Reinigungsflüssigkeit verfügt. Des Weiteren bezieht sich die Erfindung auf ein Verfahren zum Reinigen und/oder Desinfizieren eines medizintechnischen Geräts mit einer Reinigungsflüssigkeit.

### Stand der Technik

Medizintechnische Geräte, die während dem Gebrauch durch Zellen, Blut, Proteine, Bakterien, physiologische Flüssigkeiten oder dergleichen verunreinigt werden, müssen, je nach Gerätetyp, nach jeder Benutzung gereinigt, dekontaminiert, desinfiziert, sterilisiert und/oder gepflegt werden. Nur so können heutige Hygienestandards im medizinischen Bereich garantiert werden, um Keimübertragungen von Patient zu Patient wirkungsvoll zu verhindern.

Für die Reinigung und Desinfektion werden z. B. spezielle Reinigungs- und Desinfektionsflüssigkeiten eingesetzt. Ebenso ist es bekannt, die zu reinigenden Geräte mit heissem Dampf oder Gasen in Kontakt zu bringen, um eine effektive Sterilisation zu erzielen. Hierbei ist aber zu beachten, dass medizintechnische Geräte, welche empfindliche mechanische, elektronische und/oder optische Komponenten enthalten, in den meisten Fällen nicht mit aggressiven Mitteln, wie z. B. heissem Dampf, behandelt werden, da ansonsten eine Beschädigung der empfindlichen Komponenten auftreten kann. Bei derartigen medizintechnischen Geräten kann die Desinfektion bzw. die Sterilisation durch geeignete Reinigungs- und/oder Desinfektionsflüssigkeiten erreicht werden, welche eine schonende Behandlung ermöglichen.

Die Zahnmedizin ist im Besonderen von diesen Anforderungen betroffen. Empfindliche rotierende Instrumente (Hand und Winkelstücke) für Bohrer und ähnliche Werkzeuge müssen nach jeder Verwendung am Patienten gereinigt und desinfiziert bzw. aufbereitet werden.

Aber auch in anderen medizinischen Bereichen ist die Reinigung und Desinfektion von empfindlichen medizintechnischen Geräten von grosser Wichtigkeit. So weisen insbesondere Endoskope meist sehr empfindliche Elemente, z. B. optische Leiter, welche mit besonders materialschonenden Verfahren gereinigt und desinfiziert werden müssen, auf.

Um die Wirksamkeit der Reinigung und/oder Desinfektion von medizintechnischen Geräten zu garantieren, ist es unablässig, dass die Reinigungs- und/oder Desinfektionsvorgänge nach genau definierten Verfahren, welche teilweise auch gesetzlich vorgeschrieben sind, vorgenommen werden. Die durchzuführenden Verfahren umfassen beispielsweise genau definierte Verfahrensschritte, wie z. B. das vollständige Benetzen bzw. Eintauchen des zu reinigenden medizintechnischen Geräts, die Reinigung mit einer vorgeschriebenen Lösung eines oberflächenaktiven Stoffes, das Spülen und das nachfolgende in Kontakt bringen mit einer Desinfektionslösung während einer vordefinierten Mindestzeitdauer bzw. Mindesteinwirkzeit. Derartige Verfahren müssen mehr und mehr entsprechend internationalen, nationalen und/oder institutionellen Richtlinien durchgeführt werden.

Hierfür existieren bereits automatisierte Geräte, welche den Benutzer unterstützen und die Einhaltung von Prozessparametern vereinfachen wie z.B. das in US 6 117 285 offenbartes Reinigungsgerät.

In der EP 0 594 202 (Kaltenbach & Voigt GmbH & Co.) ist beispielsweise eine Vorrichtung mit einer elektrischen Steuereinheit beschrieben, welche der automatischen Reinigung und Desinfektion von ärztlichen oder zahnärztlichen Instrumenten dient. Diese werden mit Wasser, welches als Reinigungs- und Desinfektionsmittel wirkt, innenseitig und aussenseitig in einem vorbestimmten Verfahrensablauf bei unterschiedlichen Temperaturen in Kontakt gebracht. Zur Unterstützung des Reinigungsvorgangs dient zusätzlich ein Ultraschallschwinger. Die Steuerung des Reinigungs- und Desinfektionsvorgangs kann dabei vollautomatisch erfolgen.

Obschon die heute bekannten Reinigungsvorrichtungen die Einhaltung der Richtlinien vereinfachen, bestehen beispielsweise immer noch Gefahren durch Fehlmanipulationen und/oder fehlerhafte Reinigungs- und/oder Desinfektionsmittel. Aufgrund der grossen Vielfalt von unterschiedlichen Farbcodes auf den von verschiedenen Anbietern produzierten Behältern mit den Reinigungs- und/oder Desinfektionsmittel kann es nämlich leicht passieren, dass ein für die vorgesehene Anwendung nicht geeignetes Reinigungs-und/oder Desinfektionsmittel eingesetzt wird. Auch der Einsatz von Reinigungs- und/oder Desinfektionsmittel, welche z. B. bei der Produktion, beim Transport oder während der Lagerung unbemerkt verunreinigt oder mit biologischem Material kontaminiert wurden, stellt ein erhebliches Gefahrenpotential dar.

Es besteht daher nach wie vor Bedarf nach einer Reinigungsvorrichtung, welche die Einhaltung der geltenden Richtlinien betreffend die Reinigung und/oder Desinfektion von medizintechnischen Geräten vereinfacht bzw. besser gewährleistet und welche Reinigungsvorrichtung eine Validierung jedes einzelnen Reinigungs- und/oder Desinfektionsvorgangs ermöglicht.

### Darstellung der Erfindung

Aufgabe der Erfindung ist es daher, eine dem eingangs genannten technischen Gebiet zugehörende Vorrichtung zu schaffen, welche eine sichere, reproduzierbare und validierbare Reinigung und/oder Desinfektion von medizintechnischen Geräten ermöglicht.

Die Lösung der Aufgabe ist durch die Merkmale des Anspruchs 1 definiert. Gemäss der Erfindung weist die Zufuhrvorrichtung wenigstens eine zweite Sensoreinheit zur Bestimmung der stofflichen Beschaffenheit der Reinigungsflüssigkeit auf.

Der Begriff Reinigungsflüssigkeit bezieht sich im Rahmen dieser Patentanmeldung auf Flüssigkeiten, welche zur Reinigung und/oder Desinfektion geeignet sind.

Unter einem physikalischen Zustand der Reinigungsflüssigkeit wird ein durch Energieaustausch zwischen der Reinigungsflüssigkeit und seiner Umgebung veränderbarer Zustand der Reinigungsflüssigkeit verstanden. Die verschiedenen physikalischen Zustände der Reinigungsflüssigkeit sind daher insbesondere durch die Temperatur und den Druck der Reinigungsflüssigkeit charakterisiert.

Unter dem Begriff stoffliche Beschaffenheit werden in diesem Zusammenhang die physikalisch-chemischen Stoffeigenschaften der Reinigungsflüssigkeit verstanden, wobei die Reinigungsflüssigkeit als Reinstoff (als eine chemische Verbindung oder als ein chemisches Element) oder als Stoffgemisch aus wenigstens zwei Reinstoffen vorliegen kann. Die stoffliche Beschaffenheit bzw. die physikalisch-chemischen Stoffeigenschaften der Reinigungsflüssigkeit sind z. B. Dichte, elektrische Leitfähigkeit, Wärmekapazität, Viskosität, Oberflächenspannung, optische Aktivität oder Farbe bzw. Absorptions-und/oder Emissionsverhalten bezüglich elektromagnetischer Strahlung. Bei Stoffmischungen aus mehreren Reinstoffen hängen die Stoffeigenschaften im Allgemeinen vom Mischungsverhältnis der einzelnen Reinstoffe ab. Es hat sich dabei gezeigt, dass die stoffliche Beschaffenheit bzw. die physikalisch-chemischen Stoffeigenschaften der Reinigungsflüssigkeit im Bereich der hier relevanten physikalischen Zustände der Reinigungsflüssigkeit nur wenig variieren und daher als im Wesentlichen konstant betrachtet werden können.

Durch die zweite Sensoreinheit kann nun die stoffliche Beschaffenheit der für die Reinigung und/oder Desinfektion vorgesehenen Reinigungsflüssigkeit ermittelt werden. Damit kann insbesondere überprüft werden, ob die eingesetzte Reinigungsflüssigkeit auch tatsächlich die für den Reinigungs- und/oder Desinfektionsvorgang passende und zu verwendende Reinigungsflüssigkeit ist. Sollte die Reinigungsflüssigkeit z. B. mit unerwünschten Fremdstoffen verunreinigt und/oder mit biologischem Material kontaminiert sein, kann dies mit der zweiten Sensoreinheit ebenfalls festgestellt werden.

Durch das Zusammenwirken der ersten Sensoreinheit, welche zur Bestimmung eines physikalischen Zustands der Reinigungsflüssigkeit ausgebildet ist, und der zweiten Sensoreinheit, welche zur Bestimmung der stofflichen Beschaffenheit der Reinigungsflüssigkeit ausgebildet ist, kann die eingesetzte Reinigungsflüssigkeit umfassend charakterisiert werden. Damit kann sichergestellt werden, dass die für den Reinigungs- und/oder Desinfektionsvorgang passende Reinigungsflüssigkeit im entsprechend vorgeschriebenen physikalischen Zustand, z. B. bei vorgeschriebener Temperatur und Druck, vorliegt.

Die erste und/oder die zweite Sensoreinheit kann insbesondere auch dazu verwendet werden, die Funktionsfähigkeit des Reinigungsapparats zu überprüfen. Hierfür kann z. B. periodisch eine standardisierte Kalibrierungslösung eingesetzt werden, welche durch die Reinigungsapparatur und insbesondere die Sensoreinheiten geleitet wird. Dadurch lässt sich z. B. die Funktionsfähigkeit der Sensoreinheiten kontrollieren.

Damit sind die grundlegenden Voraussetzungen geschaffen, um eine wirksame und sichere Reinigung und/oder Desinfektion von medizintechnischen Geräten zu ermöglichen.

Bevorzugt ist die zweite Sensoreinheit zur Bestimmung eines pH-Werts und/oder einer elektrischen Leitfähigkeit und/oder einer Absorption von elektromagnetischer Strahlung der Reinigungsflüssigkeit ausgebildet.

Die Bestimmung des pH-Werts bzw. der H⁺-lonenkonzentration und/oder die Messung der elektrischen Leitfähigkeit der Reinigungsflüssigkeit kann z. B. durch Potentiometrie erfolgen. Diese an sich bekannte Methode hat sich in der Praxis als zuverlässig erwiesen und lässt sich zudem mit technisch relativ einfachen Mitteln realisieren. Dabei können Elektroden eingesetzt werden, welche direkt in die Reinigungsflüssigkeit eintauchen.

Der pH-Wert und die elektrische Leitfähigkeit der Reinigungsflüssigkeit haben sich zudem als durchaus charakteristische Eigenschaft der unterschiedlichen Reinigungsflüssigkeiten herausgestellt. Verunreinigungen und Kontaminationen im Reinigungsmittel beeinflussen dabei die gemessenen Werte, so dass auf die Reinheit der Reinigungsflüssigkeit geschlossen werden kann. Damit wird eine zuverlässige Aussage bezüglich der stofflichen Beschaffenheit des Reinigungsmittels ermöglicht. Insbesondere bei der gleichzeitigen Messung des pH-Werts und der elektrischen Leitfähigkeit kann die Eindeutigkeit der Zuordnung zu einer bestimmten Reinigungsflüssigkeit bzw. deren stofflicher Beschaffenheit weiter verbessert werden.

Die Messung der Absorption von elektromagnetischer Strahlung bzw. die Ausbildung der zweiten Sensoreinheit zur Messung der Absorption von elektromagnetischer Strahlung durch die Reinigungsflüssigkeit stellt eine äusserst vorteilhafte Massnahme vor. Dabei ist es möglich, die Absorption von elektromagnetischer Strahlung bei einer einzigen Wellenlänge oder aber bei mehreren Wellenlängen bzw. über einen Wellenlängenbereich zu bestimmen. Die Wellenlänge der hierfür verwendeten elektromagnetischen Strahlung liegt mit Vorteil im Infrarotbereich, im Bereich des sichtbaren Lichts und/oder im ultravioletten Bereich. Elektromagnetische Strahlung dieser Wellenlängen wechselwirkt in charakteristischer Weise mit den Molekülen in der Reinigungsflüssigkeit.

Die Absorption von elektromagnetischer Strahlung im Infrarotbereich wird insbesondere durch Molekülschwingungen der in der Reinigungsflüssigkeit vorhandenen Moleküle bestimmt. Da die meisten Moleküle aufgrund von funktionellen Gruppen charakteristische Schwingungsbanden aufweisen, lassen sich unterschiedliche Moleküle in der Reinigungsflüssigkeit und/oder unterschiedliche Reinigungsflüssigkeiten in einfacher Weise unterscheiden. Damit können beispielsweise auch die relativen Anteile der verschiedenen Moleküle in der Reinigungsflüssigkeit bestimmt werden.

Die Absorption von elektromagnetischer Strahlung im sichtbaren oder im ultravioletten Bereich wird vorwiegend durch elektronische Übergänge zwischen den verschiedenen elektronischen Zuständen der Moleküle in der Reinigungsflüssigkeit bestimmt. Da auch die elektronischen Niveaus in Molekülen charakteristische Energien aufweisen, lassen sich unterschiedliche Moleküle in der Reinigungsflüssigkeit und/oder unterschiedliche Reinigungsflüssigkeiten auch im sichtbaren oder im ultravioletten Bereich in einfacher Weise unterscheiden oder die Anteile der verschiedenen Moleküle in der Reinigungsflüssigkeit lassen sich bestimmen.

Hierbei ist jedoch zu beachten, dass die Energie der elektromagnetischen Strahlung insbesondere im ultravioletten Bereich relativ hoch ist, so dass unter Umständen chemische Reaktionen in der Reinigungsflüssigkeit induziert werden können, welche unter Umständen zu unerwünschten Veränderungen der Reinigungsflüssigkeit führen können. Vor diesem Gesichtspunkt bietet die Verwendung von elektromagnetischer Strahlung im Infrarotbereich eine optimale Wahl.

Insbesondere die Absorption von elektromagnetischer Strahlung bei mehreren Wellenlängen bzw. über einen Wellenlängenbereich hat sich als äusserst charakteristische Eigenschaft der unterschiedlichen Reinigungsflüssigkeiten herausgestellt. Die Absorption von elektromagnetischer Strahlung über einen Wellenlängenbereich kann auch als Spektrum bezeichnet werden. Verunreinigungen und Kontaminationen im Reinigungsmittel beeinflussen dabei die gemessenen Werte, so dass durch die Absorption von elektromagnetischer Strahlung auf die Reinheit der Reinigungsflüssigkeit geschlossen werden kann, womit eine äusserst zuverlässige Aussage bezüglich der stofflichen Beschaffenheit des Reinigungsmittels erhalten wird.

Um die Sicherheit bei der Bestimmung der stofflichen Beschaffenheit der Reinigungsflüssigkeit weiter zu erhöhen, können die Absorption von elektromagnetischer Strahlung und die elektrische Leitfähigkeit der Reinigungsflüssigkeit und/oder der pH-Wert der Reinigungsflüssigkeit gemeinsam gemessen werden.

Grundsätzlich ist es auch denkbar, die Absorption der elektromagnetischen Strahlung im Mikrowellenbereich zu messen, welche durch Übergänge zwischen verschiedenen Rotationsniveaus in den Molekülen der Reinigungsflüssigkeit bestimmt ist. Hierbei ist zu beachten, dass diese aufgrund von Molekülwechselwirkungen in Flüssigkeiten stark verbreitert sind, so dass eine Zuordnung zu den einzelnen Molekülen unter Umständen schwieriger ist.

Ebenso können mit der zweiten Sensoreinheit anstelle oder zusätzlich zur Bestimmung des pH-Werts, der elektrischen Leitfähigkeit und/oder der Absorption von elektromagnetischer Strahlung auch andere physikalisch-chemische Stoffeigenschaften bestimmt werden, welche vorstehend bereits genannt wurden. Dies können z. B. die Emission von elektromagnetischer Strahlung, die Viskosität, die Dichte oder die optische Aktivität der Reinigungsflüssigkeit sein.

Die erste Sensoreinheit ist insbesondere zur Bestimmung einer Temperatur und/oder eines Drucks der Reinigungsflüssigkeit ausgebildet.

Die Temperatur spielt bei der Reinigung und/oder Desinfektion eine entscheidende Rolle und sollte daher so genau wie möglich eingehalten werden. Im Allgemeinen hängen die Geschwindigkeiten von physikalisch-chemischen Vorgängen, wie z. B. Lösungsprozesse von Verunreinigungen auf Oberflächen, stark von der Temperatur ab. Um eine wirksame Reinigung eines medizinischen Geräts zu erreichen, ist es daher insbesondere vorteilhaft, eine gewisse Mindesttemperatur einzuhalten. Jedoch sollte die Temperatur gewisse Maximalwerte nicht übersteigen, um das medizinische Gerät und/oder das Reinigungsmittel aufgrund von Hitzeeinwirkungen nicht zu beschädigen oder zu beeinträchtigen. Dies kann durch eine entsprechend zur Temperaturbestimmung ausgebildete erste Sensoreinheit garantiert werden.

Als Temperaturmesser sind z. B. Thermoelemente, umfassend zwei an ihren Enden verbundene unterschiedliche Metalle, geeignet, welche in der Zufuhrvorrichtung direkt in die Reinigungsflüssigkeit hineinragend angeordnet werden können. Auch denkbar ist die Verwendung eines Widerstandsthermometers bzw. eines Thermistors, wobei die Temperatur über die Temperaturabhängigkeit eines elektrischen Widerstands gemessen wird. Als elektrischer Widerstand kann z. B. Platin, eine Heissleiter (NTC-Widerstand) oder ein Kalteiter (PTC-Widerstand) verwendet werden.

Ebenso ist die Einhaltung eines bestimmten Drucks der Reinigungsflüssigkeit bei der Reinigung von medizintechnischen Geräten vorteilhaft. Der Druck der Reinigungsflüssigkeit bestimmt nämlich, im Zusammenspiel mit den Dimensionen des Zufuhrsystems, insbesondere die Geschwindigkeit, mit welcher die Reinigungsflüssigkeit beispielsweise auf die Oberfläche oder durch die inneren Kanäle des medizintechnischen Instruments geleitet wird. Die Fliess- oder Spritzgeschwindigkeit der Reinigungsflüssigkeit hat dabei einen nicht zu vernachlässigenden Einfluss auf den Reinigungsprozess, da durch die kinetische Energie der Reinigungsflüssigkeit eine mechanische Entfernung von Verunreinigungen erreicht werden kann.

Zur Messung des Drucks können z. B. piezoelektrische Sensoren eingesetzt werden, bei welchen aufgrund des auf einen Kristall einwirkenden Drucks eine Ladungstrennung und damit eine elektrische Spannung erzeugt wird.

In einer vorteilhaften Variante ist die erste Sensoreinheit daher insbesondere zur gleichzeitigen Bestimmung einer Temperatur und eines Drucks der Reinigungsflüssigkeit ausgebildet.

Bevorzugt verfügt die Zufuhrvorrichtung über wenigstes eine dritte Sensoreinheit zur Bestimmung eines Strömungsparameters der Reinigungsflüssigkeit, wobei die dritte Sensoreinheit insbesondere ein Durchflussmessgerät umfasst.

Unter einem Strömungsparameter wird in diesem Zusammenhang ein Parameter verstanden, welcher die Strömung der Reinigungsflüssigkeit in der Zufuhrvorrichtung charakterisiert. Dies können z. B. eine Fliessgeschwindigkeit, ein dynamischer Druck, ein Volumenstrom bzw. eine Durchflussmenge, ein Massenstrom bzw. ein Durchsatz und/oder eine Wandschubspannung der Reinigungsflüssigkeit sein.

Die Bestimmung eines Strömungsparameters ist insbesondere im Hinblick auf die Zufuhr bzw. die Förderung der Reinigungsflüssigkeit in der Zufuhrvorrichtung relevant. Damit kann sichergestellt werden, dass die Reinigungsflüssigkeit auch tatsächlich durch die Zufuhrvorrichtung bewegt wird bzw. dass die Reinigungsflüssigkeit in den Aufnahmeraum und/oder die Kupplungsvorrichtung für das zu reinigende medizintechnische Gerät gefördert wird.

Durch ein Durchflussmessgerät können z. B. das Volumen der aktuell pro Zeiteinheit durch die Zufuhrvorrichtung fliessenden Reinigungsflüssigkeit und/oder das Gesamtvolumen der während des gesamten Reinigungsvorgangs durch die Reinigungsapparatur geflossenen Reinigungsflüssigkeit bestimmt werden. Dies stellt besonders aussagekräftige Strömungsparameter dar. Es kann aber auch eine andere Vorrichtung angeordnet werden, welche es erlaubt einen Strömungsparameter zu messen.

Insbesondere im Zusammenspiel mit der ersten Sensoreinheit und der zweiten Sensoreinheit kann mit der dritten Sensoreinheit die korrekte Durchführung des Reinigungs- und/oder Desinfektionsverfahrens überwacht werden, womit bezüglich der Reinigung und/oder Desinfektion von medizintechnischen Geräten eine bestmögliche Sicherheit gewährleistet ist.

Grundsätzlich kann die dritte Sensoreinheit aber auch an einer anderen Stelle als im Bereich der Zufuhrvorrichtung angeordnet sein. So kann die dritte Sensoreinheit zur Bestimmung eines Strömungsparameters der Reinigungsflüssigkeit beispielsweise auch in einem Auffangbereich angeordnet sein, welcher dem medizintechnischen Gerät nachgeordnet ist und die Reinigungsflüssigkeit nach dem Kontakt mit dem medizintechnischen Gerät wieder auffängt. Auch möglich ist der vollständige Verzicht auf die dritte Sensoreinheit.

In einer weiteren bevorzugten Ausführungsform ist die Reinigungsapparatur derart ausgebildet, dass die Funktionsfähigkeit sämtlicher Sensoreinheiten überprüft und/oder validiert werden kann. Insbesondere können die Sensoreinheiten der Reinigungsapparatur dabei auch kalibriert werden. Hierfür kann die Reinigungsapparatur mit einer oder mit mehreren standardisierten Kontrolllösungen beaufschlagt werden. Die Kontrolllösungen verfügen dabei z. B. über genau bekannte stoffliche Beschaffenheiten. Gelangt die Kontrolllösung in eine zu überprüfende Sensoreinheit, sollten die von den einzelnen Sensoren der Sensoreinheit gemessenen Messwerte in einem vorgegebenen Messwertbereich liegen. Liegen einer oder mehrere der Messwerte ausserhalb des vorgegebenen Bereichs, so können die entsprechenden Sensoren neu kalibriert werden. Dies kann z. B. mit einer oder mit mehreren speziellen Kalibrierlösung erfolgen, welche auf die einzelnen Sensoren abgestimmt sind. Somit können beispielsweise die Genauigkeit, die Präzision, die analytische Sensitivität, die analytische Spezifität, die Reproduzierbarkeit und/oder andere für die Validierung der Sensoreinheiten relevante Eigenschaften bestimmt und angepasst werden.

Die Reinigungsapparatur, insbesondere eine Steuereinheit der Reinigungsapparatur, kann dabei so ausgebildet sein, dass bei einer nicht erfolgreich durchgeführten Überprüfung und/oder Kalibrierung der Sensoreinheiten bzw. bei defekten Sensoreinheiten, die Reinigungsapparatur in einen blockierten Zustand übergeht und dabei eine weitere Verwendung der Reinigungsapparatur verunmöglicht wird. Damit wird verhindert, dass medizintechnische Geräte mit einer fehlerhaften Reinigungsapparatur gereinigt werden können, was die Sicherheit der Reinigungs- und/oder Desinfektionsvorgänge erhöht. Zur Weiterverwendung der Reinigungsapparatur können die defekten Sensoreinheiten oder die einzelnen defekten Sensoren der Sensoreinheiten ausgetauscht werden.

Die Reinigungsapparatur und/oder die Steuereinheit der Reinigungsapparatur kann insbesondere so ausgebildet sein, dass periodisch eine Validierung bzw. Überprüfung der Sensoreinheiten durchgeführt werden muss, damit die Reinigungsapparatur funktionstüchtig bleibt. Hierfür kann z. B. das Datum der nächsten durchzuführenden Validierung bzw. Überprüfung der Sensoreinheiten in einem Datenspeicher abgelegt werden. Erfolgt bis zu diesem Datum keine Validierung bzw. Überprüfung der Sensoreinheiten, blockiert die Reinigungsapparatur und verunmöglicht dabei eine weitere Verwendung der Reinigungsapparatur.

Besonders vorteilhaft weist die Zufuhrvorrichtung eine Einrichtung zur Veränderung des physikalischen Zustands der Reinigungsflüssigkeit auf, insbesondere eine Pumpe und/oder eine Heizung. Durch eine Pumpe kann die Reinigungsflüssigkeit einerseits in den Aufnahmebereich und/oder die Kupplungsvorrichtung für die medizintechnischen Geräte gefördert werden. Andererseits kann die Pumpe aber auch zur Druckerzeugung eingesetzt werden. In beiden Fällen wird der Reinigungsflüssigkeit Energie durch mechanische Arbeit der Pumpe zugeführt. Die Pumpe kann z. B. eine Membranpumpe oder eine Rotationskolbenpumpe sein. Im Prinzip können aber sämtliche zur Förderung von Flüssigkeit geeigneten Pumpen eingesetzt werden, wobei aber mit Vorteil selbstansaugende Pumpen verwendet werden, so dass die Lage des Behälters bzw. der Reservoirs mit der Reinigungsflüssigkeit in weiten Bereichen beliebig gewählt werden kann.

Es ist aber auch möglich, anstelle und/oder zusätzlich zur Pumpe für die Druckerzeugung und/oder die Förderung der Reinigungsflüssigkeit eine Verbindung mit einer externen Druckleitung, z. B. eine Wasserleitung, und/oder eine Druckluftleitung vorzusehen.

Grundsätzlich ist es auch denkbar, auf eine Pumpe zu verzichten und die Reinigungsflüssigkeit durch die Wirkung der Schwerkraft aus einem oberhalb gelegenen und in vertikaler Richtung verschiebbaren Behälter oder Reservoir in die Zufuhrvorrichtung einzubringen. Je nach vertikaler Lage des Behälters oder des Reservoirs kann dabei der Druck der Reinigungsflüssigkeit in der Zufuhrvorrichtung reguliert werden.

Eine Heizung in der Zufuhrvorrichtung ermöglicht in einfacher Weise eine Anpassung bzw. eine Energiezufuhr und damit eine Erhöhung der Temperatur der Reinigungsflüssigkeit. Die Heizung ist zweckmässigerweise als elektrische Widerstandsheizung ausgebildet, da sich diese in einfacher Weise steuern lässt. Prinzipiell kann die Heizung auch mit einer Kühlung kombiniert werden, so dass die Temperatur der Reinigungsflüssigkeit in optimaler Weise reguliert werden kann. Hierfür sind beispielsweise Peltier-Elemente geeignet. Je nach Stromrichtung kann ein Peltier-Element zur Heizung oder zur Kühlung eingesetzt werden und zeichnet sich des Weiteren durch eine Platz sparende Bauweise aus.

Zusätzlich oder anstelle einer elektrischen Heizung kann beispielsweise auch eine elektromagnetische Heizung vorgesehen sein. Insbesondere Mikrowellen- und/oder Infrarotstrahler sind hierbei geeignet. Dabei wird die zur Erwärmung der Reinigungsflüssigkeit benötigte Energie in Form von elektromagnetischer Strahlung im Mikrowellen- bzw. Infrarotbereich in die Reinigungsflüssigkeit eingestrahlt und von den Molekülen in der Reinigungsflüssigkeit absorbiert.

Es liegt aber auch im Rahmen der Erfindung, auf eine Heizung zu verzichten und z. B. eine exakt vorgewärmte Reinigungsflüssigkeit einzusetzen oder die Reinigungsflüssigkeit bei Raumtemperatur zu verwenden.

Mit Vorteil ist die Steuereinheit derart ausgebildet, dass die Einrichtung zur Veränderung des physikalischen Zustands der Reinigungsflüssigkeit aufgrund eines Signals der ersten Sensoreinheit zur Bestimmung des physikalischen Zustands der Reinigungsflüssigkeit steuerbar ist. Damit kann beispielsweise ein in der Steuereinheit abgelegter Vergleichswert bzw. Sollwert bezüglich dem physikalischen Zustand der Reinigungsflüssigkeit eingestellt und geregelt werden. Sollte sich z. B. die Temperatur und/oder der Druck der geförderten Reinigungsflüssigkeit im Laufe des Reinigungs-und/oder Desinfektionsvorgangs verändern, kann dies in einfacher Weise nachgeregelt werden. Hierfür umfasst die Steuereinheit bevorzugt einen rückgekoppelten Regelkreis, welcher die Einhaltung eines bestimmten physikalischen Zustands der Reinigungsflüssigkeit sicherstellt.

Prinzipiell ist es aber auch denkbar, dass die Einrichtung zur Veränderung des physikalischen Zustands manuell gesteuert wird. Hierfür kann der von der ersten Sensoreinheit ermittelte physikalische Zustand der Reinigungsflüssigkeit dem Benutzer z. B. über eine visuelle und/oder tongestützte Ausgabevorrichtung angezeigt werden. Darauf kann der Benutzer die Vorrichtung zur Veränderung des physikalischen Zustands manuell bedienen.

In einer weiteren bevorzugten Ausführungsform ist die Steuereinheit derart ausgebildet, dass die Funktionsfähigkeit der Reinigungsapparatur und insbesondere der Sensoreinheiten periodisch geprüft, kalibriert und/oder validiert wird. Die Sensoreinheit kann zudem mit geeigneten Messfühlern verbunden sein, so dass beispielsweise ein Flüssigkeitsstand in einem der Flüssigkeitsbehälter und/oder einem Auffanggefäss überprüft werden kann. Die Steuereinheit kann insbesondere auch so ausgebildet sein, dass ein an einer Verbindungsvorrichtung angekoppeltes und zu reinigenden medizintechnischen Geräts erkannt werden kann und/oder dass die korrekte Ankoppelung des medizintechnischen Geräts durch die Steuereinheit überprüft werden kann. Bei einem nicht korrekt angekoppelten medizintechnischen Gerät kann die Steuereinheit z. B. ein Ventil in der Verbindungsvorrichtung verschliessen. Ebenso kann das Ventil in der Verbindungsvorrichtung bei einem nicht angekoppelten medizintechnischen Gerät durch die Steuereinheit automatisch verschlossen werden. Im Falle einer nicht korrekten Ankoppelung des medizintechnischen Geräts kann die Steuereinheit beispielsweise den Reinigungs- bzw. Desinfektionsvorgang abbrechen und/oder eine entsprechende Mitteilung an den Benutzer ausgeben, so dass eine ungenügende Reinigung des betreffenden medizintechnischen Geräts vermieden wird.

Ebenfalls kann die Steuereinheit so ausgestaltet sein, dass z. B. die Lage eines Verschlussdeckels oder einer Verschlussklappe kontrolliert werden kann. Damit wird insbesondere sichergestellt, dass während dem Reinigungs- und/oder Desinfektionsvorgang keine Reinigungsflüssigkeit aus der Reinigungsapparatur heraus spritzt.

In einer weiteren Ausführungsform kann die Steuereinheit derart ausgebildet sein, dass je nach Benutzer unterschiedliche Funktionen der Reinigungsapparatur aktiviert und/oder deaktiviert werden. Die Identifikation des Benutzers kann z. B. über eine mit der Steuereinheit verbundene Eingabevorrichtung, z. B. eine Tastatur, beim Einschalten des Geräts erfolgen. Die Steuereinheit kann z. B. so ausgestaltet sein, dass einem gewöhnlichen Benutzer die Überprüfung und/oder Kalibrierung der Sensoreinheiten verunmöglicht wird, während einer Aufsichtperson der Zugriff auf sämtliche Funktionen der Reinigungsapparatur ermöglicht wird. Dies verbessert ebenfalls die Sicherheit der Reinigungs- und/oder Desinfektionsvorgänge, da sichergestellt werden kann, dass nur Personen mit ausreichendem Fachwissen, z. B. die Aufsichtsperson, auf sicherheitskritische Funktionen zugriff haben.

Insbesondere verfügt die Steuereinheit über eine Zeitmesseinrichtung und/oder ist mit einer Zeitmesseinrichtung verbunden. Die Wirksamkeit eines Reinigungs- und/oder Desinfektionsvorgangs hängt im Allgemeinen auch von den Einwirkzeiten bzw. der Zeitdauer, während welcher die Reinigungsflüssigkeit mit dem zu reinigenden medizintechnischen Gerät in Kontakt steht, ab. Bevorzugt wird daher die Aussenseite und/oder die Innenseite des medizintechnischen Geräts während einer durch die Steuereinheit kontrollierten und vorbestimmten Zeitdauer mit der Reinigungsflüssigkeit in Kontakt gebracht. Zusätzlich kann durch die Steuereinheit kontrolliert werden, dass die stoffliche Beschaffenheit der Reinigungsflüssigkeit und/oder der physikalische Zustand der Reinigungsflüssigkeit und/oder der Strömungsparameter der Reinigungsflüssigkeit während der gesamten, für eine wirksame Reinigung und/oder Desinfektion erforderlichen Zeitdauer eingehalten wird bzw. in einem zulässigen Wertebereich liegt.

Mit einer Steuereinheit, welche über eine Zeitmesseinrichtung verfügt und/oder mit einer Zeitmesseinrichtung verbunden ist, können auch zeitgesteuert Anweisungen an den Benutzer, z. B. über eine visuelle und/oder tongestützte Ausgabevorrichtung, abgegeben werden. Der Benutzer kann z. B. über den zeitlichen Ablauf der einzelnen Schritte eines Reinigungs- und/oder Desinfektionsvorgangs informiert werden und gegebenenfalls aufgefordert werden, den physikalischen Zustand der Reinigungsflüssigkeit nach einer vorgegebenen Zeitdauer zu verändern und/oder die Reinigungsflüssigkeit auszutauschen.

Es kann auch vorteilhaft sein, die stoffliche Beschaffenheit der Reinigungsflüssigkeit und/oder den physikalischen Zustand der Reinigungsflüssigkeit und/oder den Strömungsparameter der Reinigungsflüssigkeit direkt zeitaufgelöst zu messen und/oder als eine Funktion der Zeit in der Steuereinheit zu erfassen. Diese Daten können dann z. B. zur Protokollierung bei der Qualitätssicherung herangezogen werden. Generell ist es aber auch möglich, auf eine Zeitmesseinrichtung für die Steuereinheit zu verzichten. In diesem Fall kann der Benutzer die Einhaltung des zeitlichen Ablaufs beispielsweise manuell durch eine externe Uhr kontrollieren.

Sämtliche von der Steuereinheit erfassten Daten und/oder ausgesendeten Steuerbefehle werden vorzugsweise in einem Speicherbereich der Steuereinheit in elektronischer Form abgespeichert. Es kann aber auch vorteilhaft sein, die besagten Daten in einer externen Speichereinrichtung, wie z. B. einem USB-Stick, einer Harddisk, abzulegen. Ebenso ist es denkbar, die besagten Daten direkt auf einem internen und/oder externen Drucker auf Papier auszugeben.

Es liegt dabei ebenfalls im Rahmen der vorliegenden Erfindung, die Daten vor dem Abspeichern aus Sicherheitsgründen zu verschlüsseln bzw. mit einem Passwort zu schützen.

Um einen vollautomatischen Betrieb der Reinigungsapparatur zu ermöglichen, kann die erste Sensoreinheit zur Bestimmung des physikalischen Zustands der Reinigungsflüssigkeit über eine erste Signalleitung und die zweite Sensoreinheit zur Bestimmung der stofflichen Beschaffenheit der Reinigungsflüssigkeit über eine zweite Signalleitung und, falls vorhanden, die dritte Sensoreinheit zur Bestimmung eines Strömungsparameters der Reinigungsflüssigkeit über eine dritte Signalleitung mit der Steuereinheit verbunden werden. Entsprechend kann die Einrichtung zur Veränderung des physikalischen Zustands der Reinigungsflüssigkeit über eine vierte Signalleitung bzw. eine Steuerleitung mit der Steuereinheit verbunden werden.

Bevorzugt ist die Kupplungsvorrichtung für das zu reinigende medizintechnische Gerät sowohl von der Reinigungsapparatur selbst als auch von dem zu reinigenden medizintechnischen Gerät an- und abkoppelbar ausgebildet. Besonders bevorzugt ist die Kupplungsvorrichtung von Hand und ohne zusätzliches Werkzeug an- und abkoppelbar ausgebildet. Dies kann z. B. Rastklinken und/oder Rastkerben, welche an der Kupplungsvorrichtung angebracht sind, erreicht werden. Die Kupplungsvorrichtungen sind im Kupplungsbereich für die medizintechnischen Geräte an diese angeformt. Durch die Verwendung von an- und abkoppelbaren Kupplungsvorrichtungen können in einfacher Weise unterschiedliche medizintechnische Geräte in den erfindungsgemässen Reinigungsapparaturen verwendet werden. Auf ein kompliziertes Umrüsten der Reinigungsapparatur kann somit verzichtet werden. Zudem ist es durch an- und ankoppelbare Kupplungsvorrichtungen problemlos möglich, gleichzeitig mehrere unterschiedliche medizintechnische Geräte zu reinigen und/oder desinfizieren. Grundsätzlich ist es aber auch möglich eine fix angeordnete Kupplungsvorrichtung bzw. ein fix angeordnetes Verbindungsstück vorzusehen, was aber die Flexibilität der Reinigungsapparatur vermindet.

Die Verbindungsvorrichtungen verfügen mit Vorteil über einen Kupplungssensor zur Erkennung eines angekoppelten medizintechnischen Geräts. Die Verbindungsvorrichtung mit dem Kupplungssensor ist dabei bevorzugt so ausgebildet, dass zwischen einem korrekt angekoppelten und einem falsch angekoppelten medizintechnischen Gerät unterschieden werden kann, so dass unzureichende Reinigungs- und/oder Desinfektionsvorgänge verhindert werden können. Auf den Kupplungssensor kann prinzipiell verzichtet werden. Die Überprüfung der korrekten Ankopplung des medizintechnischen Geräts erfolgt in diesem Fall z. B. manuell durch den Benutzer.

Die Steuereinheit ist dabei bevorzugt so ausgebildet, dass aufgrund eines Signals des Kupplungssensors die Zuleitung von Reinigungsflüssigkeit oder anderen Fluiden zu einem Verbindungsstück bzw. einer Kupplungsvorrichtung mit einem nicht korrekt angekoppelten oder zu einem Verbindungsstück ohne angekoppeltes medizintechnisches Gerät unterbunden wird. Dies kann z. B. durch ein steuerbares Ventil in den entsprechenden Zuleitungen bewirkt werden. Damit wird sichergestellt, dass nicht unnötig Reinigungsflüssigkeit verbraucht wird, was insbesondere ökonomischer ist. Die Ansteuerung eines entsprechenden Ventils kann grundsätzlich aber auch manuell durch den Benutzer erfolgen.

Mit Vorteil verfügt wenigstens eine Verbindungsvorrichtung bzw. eine Kupplungsvorrichtung über mehrere Fluidleitungen, welche insbesondere zur Kommunikation mit unterschiedlichen Fluidkanälen des zu reinigenden medizintechnischen Geräts, insbesondere eines medizintechnischen Hohlinstruments, vorgesehen sind. Damit können beispielsweise unterschiedliche Fluide, wie z. B. Reinigungsflüssigkeit und/oder Druckluft, getrennt in die unterschiedlichen Fluidkanäle der zu reinigenden medizintechnischen Geräte geleitet werden. Es ist aber auch möglich, lediglich eine gemeinsame Fluidleitung als Zuleitung vorzusehen.

In einer Weiteren vorteilhaften Ausführungsform verfügen die Verbindungstücke zudem über mechanische Antriebe zur Bewegung von drehenden Teilen der medizintechnischen Geräte während dem Reinigungs- und/oder Desinfektionsvorgang. Drehende Teile sind z. B. Rotoren bei zahnärztlichen Hand- oder Winkelstücken. Die mechanischen Antriebe können z. B. in Form eines als Elektromotors oder in Form von pneumatisch angetriebenen Rotoren und/oder Turbinen vorliegen. Durch das Bewegen der drehenden Teile der zu reinigenden medizintechnischen Geräte kann die Reinigungs- und/oder Desinfektionswirkung verbessert oder sogar erst ermöglicht werden. Es ist aber auch möglich, auf derartige Antriebe zu verzichten und die drehenden Teile z. B. manuell zu bewegen oder auf eine Bewegung der Teile zu verzichten. In letzterem Fall kann aber die Wirksamkeit des Reinigungs- und/oder Desinfektionsvorgangs bei medizintechnischen Geräten mit drehenden Teilen beeinträchtigt werden.

In einer weiteren vorteilhaften Ausführungsform verfügt die Kupplungsvorrichtung über eine Anschlussstelle für ein Adapterstück, welches die Kupplungsvorrichtung und das zu reinigende medizintechnische Gerät miteinander verbindet. Das Adapterstück kann dabei auch als Einwegprodukt ausgebildet sein, welches nach einem einmaligen Gebrauch weggeworfen werden kann. Die Adapterstücke, welche sich relativ kostengünstig herstellen lassen, können an praktisch alle auf dem Markt erhältlichen medizintechnischen Geräte angepasst werden. Damit weisen die erfindungsgemässen Reinigungsapparaturen sehr breite Anwendungspektren auf. Prinzipiell kann aber auch auf die Adapterstücke verzichtet werden.

In einer weiteren vorteilhaften Ausführungsform verfügt die Reinigungsapparatur über einen geschlossenen Flüssigkeitskreislauf für die Reinigungsflüssigkeit, so dass die Reinigungsflüssigkeit während dem Reinigungs- und/oder Desinfektionsvorgang im Flüssigkeitskreislauf zirkulieren kann. Damit kann die Reinigungsflüssigkeit so lange sie für die Reinigungs- und/oder Desinfektionszwecks geeignet ist, weiter verwendet werden. Dies ist insbesondere ökonomischer. Es ist aber auch möglich ein Flüssigkeitsbehälter mit frischer Reinigungsflüssigkeit und ein Auffanggefäss für die verbrauchte Reinigungsflüssigkeit vorzusehen und dabei auf einen geschlossenen Flüssigkeitskreislauf zu verzichten.

Bevorzugt ist zur Kontrolle eines Füllstandes der Reinigungsflüssigkeit im Aufnahmebereich ein Zwischenreservoir im Flüssigkeitskreislauf angeordnet. Soll der Aufnahmebereich während dem Reinigungs- bzw. Desinfektionsvorgang vollständig mit Reinigungsflüssigkeit gefüllt sein, so dass z. B. die zu reinigenden medizintechnischen Geräte vollständig eintauchen, kann der Füllstand im Zwischenreservoir gesenkt werden. Zum Einbringen und Entfernen der medizintechnischen Geräte und/oder während einem speziellen Reinigungsschritt während dem Rienigungs- und/oder Desinfektionsvorgang kann der Füllstand des Zwischenreservoirs angehoben werden, wobei als Konsequenz davon der Füllstand im Aufnahmebereich abgesenkt und/oder der Aufnahmebereich vollständig entleert wird. Grundsätzlich kann aber auch auf das Zwischenreservoir verzichtet werden, wobei die vorstehend beschriebene Befüllung und Entleerung des Aufnahmebereich z. B. manuell oder automatisch über eine Zufuhrleitung und/oder eine Abflussleitung zu einem externen Reservoir und/oder Abfluss verändert werden kann.

Bezüglich des erfindungsgemässen Verfahrens wird zur Reinigung und/oder Desinfektion eines medizintechnischen Geräts, insbesondere eines zahnmedizinischen Hohlinstruments wie ein Hand- und/oder ein Winkelstück, die Reinigungsflüssigkeit über die Zufuhrvorrichtung in den Aufnahmebereich und/oder die Kupplungsvorrichtung für das zu reinigende medizintechnische Gerät geleitet und über die wenigstens eine und erste Sensoreinheit der Zufuhrvorrichtung der physikalische Zustand der Reinigungsflüssigkeit gemessen und in der Steuereinheit des Reinigungsapparats erfasst, wobei die Reinigungsflüssigkeit mit der Aussenseite und/oder der Innenseite des medizintechnischen Geräts in Kontakt gebracht wird und die stoffliche Beschaffenheit der Reinigungsflüssigkeit in der Zufuhrvorrichtung durch die zweite Sensoreinheit gemessen wird.

Bevorzugt wird dabei ein durch die zweite Sensoreinheit gemessenes Messsignal in der Steuereinheit des Reinigungsapparats mit wenigstens einem in der Steuereinheit abgelegten ersten Vergleichswert verglichen. In der Steuereinheit können insbesondere mehrere und unterschiedlichen Reinigungsflüssigkeiten zugeordnete Vergleichswerte abgelegt sein. Je nach eingesetzter Reinigungsflüssigkeit kann der Benutzer dabei den der verwendeten Reinigungsflüssigkeit zugeordneten Vergleichswert selektieren oder einstellen. Dies kann z. B. über eine Eingabeeinheit, z. B. eine Zahlen- und/oder Buchstabentastatur, erfolgen.

Aufgrund des Vergleichs des durch die zweite Sensoreinheit gemessenen Messsignals und des ersten Vergleichswerts kann in der Steuereinheit ein Zustand der Reinigungsflüssigkeit ermittelt werden. Je nach Abweichung zwischen Messsignal und Vergleichswert kann z. B. auf eine nicht passende Reinigungsflüssigkeit und/oder eine verunreinigte Reinigungsflüssigkeit geschlossen werden.

Grundsätzlich ist es aber auch denkbar, dass das von der zweiten Sensoreinheit gemessene Messsignal dem Benutzer lediglich durch eine visuelle und/oder tongestützte Ausgabeeinheit angezeigt bzw. mitgeteilt wird. Auf einen Vergleich mit einem Vergleichswert in der Steuereinheit kann in diesem Fall verzichtet werden. Der Benutzer kann in diesem Fall den Vergleich mit einem Vergleichswert beispielsweise manuell vornehmen.

Die Messung der stofflichen Beschaffenheit der Reinigungsflüssigkeit und der Vergleich mit dem in der Steuereinheit abgelegten ersten Vergleichswert erfolgt vorteilhaft mehrmals, insbesondere kontinuierlich, während der Zuleitung der Reinigungsflüssigkeit. Damit wird die Sicherheit erhöht. Sollte sich die zugeleitete Reinigungsflüssigkeit nämlich während der Zuleitung bezüglich ihrer stofflichen Beschaffenheit verändern, besteht so die Möglichkeit dies zu erkennen und gegebenenfalls entsprechend notwendige Massnahmen, wie z. B. die Ausgabe einer Warnung an den Benutzer, zu treffen. Die kontinuierliche Messung der stofflichen Beschaffenheit der Reinigungsflüssigkeit und der Vergleich mit dem in der Steuereinheit abgelegten ersten Vergleichswert bieten dabei eine bestmögliche Sicherheit, welche wirksam verhindert, dass während dem Reinigungs- und/oder Desinfektionsvorgang ungeeignete Reinigungsflüssigkeit verwendet wird.

Es ist grundsätzlich aber auch möglich, die Messung der stofflichen Beschaffenheit der Reinigungsflüssigkeit und den Vergleich mit dem in der Steuereinheit abgelegten ersten Vergleichswert lediglich einmal in Form einer Stichprobe durchzuführen.

Vorteilhafterweise unterbindet die Zufuhrvorrichtung bei einer vorgegebenen Abweichung des durch die zweite Sensoreinheit gemessenen Messsignals vom ersten Vergleichswert die Zuleitung der Reinigungsflüssigkeit in den Aufnahmebereich und/oder in die Kupplungsvorrichtung für das zu reinigende medizintechnische Gerät. Damit wird sichergestellt, dass der Reinigungs- und/oder Desinfektionsvorgang bei der Zufuhr einer fehlerhaften und/oder ungeeigneten Reinigungsflüssigkeit automatisch abgebrochen wird. In diesem Fall kann, falls dies möglich ist, die Reinigungsflüssigkeit in geeigneter Weise modifiziert werden, z. B. durch Zugabe eines Wirkstoffkonzentrats und/oder Filterung von Fremdstoffen. Ansonsten kann die Reinigungsflüssigkeit auch ausgetauscht werden. Die Unterbindung der Zuleitung der Reinigungsflüssigkeit kann dem Benutzer insbesondere über eine visuelle und/oder tongestützte Ausgabevorrichtung angezeigt werden.

Es ist aber prinzipiell auch möglich, dass die Zuleitung der Reinigungsflüssigkeit nicht automatisch unterbunden wird, sondern, dass beispielsweise lediglich eine Statusmeldung an den Benutzer, z. B. über eine visuelle und/oder tongestützte Ausgabevorrichtung, ausgegeben wird.

Besonders bevorzugt werden der gemessene physikalische Zustand der Reinigungsflüssigkeit in der Steuereinheit mit einem zweiten Vergleichswert und/oder der gemessene Strömungsparameter der Reinigungsflüssigkeit in der Steuereinheit mit einem dritten Vergleichswert verglichen. Insbesondere wird bei einer vorgegebenen Abweichung des gemessenen physikalischen Zustands der Reinigungsflüssigkeit vom zweiten Vergleichswert und/oder bei einer vorgegebenen Abweichung des gemessenen Strömungsparameters der Reinigungsflüssigkeit vom dritten Vergleichswert durch die Steuereinheit ein Betriebszustand der Vorrichtung zur Veränderung des physikalischen Zustands der Reinigungsflüssigkeit, insbesondere der Pumpe und/oder der Heizung, angepasst.

Dabei kann z. B. ein Regelkreis in der Steuereinheit vorgesehen werden, welcher die erforderlichen physikalischen Parameter und/oder der Strömungsparameter der Reinigungsflüssigkeit kontrolliert und konstant hält. Sollten sich die erforderlichen physikalischen Parameter und/oder Strömungsparameter durch die Vorrichtung zur Veränderung des physikalischen Zustands der Reinigungsflüssigkeit nicht einstellen lassen, kann der Reinigungs- und/oder Desinfektionsvorgang abgebrochen werden und/oder der Benutzer wird entsprechend, z. B. durch eine visuelle und/oder tongestützte Ausgabevorrichtung, informiert.

Damit wird die Einhaltung von Reinigungsvorschriften stark vereinfacht, was zudem die Sicherheit bezüglich eines wirksamen Reinigungs- und/oder Desinfektionsvorgangs erhöht.

Bevorzugt sind in der Speichereinheit mehrere unterschiedliche und unterschiedlichen Reinigungsflüssigkeiten zugeordnete Steuerprogramme abgelegt, welche insbesondere durch den Benutzer ausgewählt werden können. Die Steuerprogramme ermöglichen insbesondere einen vollautomatischen Betrieb der Reinigungsapparatur.

Grundsätzlich ist es aber auch möglich, auf Steuerprogramme zu verzichten, wobei die Steuerung der Reinigungsapparatur beispielsweise durch manuell ausgelöste Ein- und Ausschaltvorgänge der verschiedenen Komponenten der Reinigungsapparatur erfolgen kann.

Vorteilhafterweise werden zur Qualitätskontrolle sämtliche von den Sensoren erfassten Messwerte, insbesondere zeitaufgelöst, in einem Speicher der Steuereinrichtung bzw. in einer Protokolldatei abgelegt. Hierfür verfügt die Steuereinheit insbesondere über eine Speichereinheit.

Die von den Sensoren gemessenen Werte können aber auch direkt bzw. ohne Zwischenspeicherung in der Reinigungsapparatur über eine mit der Steuereinheit verbundene Kommunikationsschnittstelle an ein Datenerfassungssystem übermittelt und dort abgelegt bzw. protokolliert werden. Hierfür verfügt die Reinigungsapparatur bevorzugt über eine Kommunikationsschnittstelle, welche insbesondere zum Datenaustausch mit einem Computer und/oder einer einem externen Datenspeicher vorgesehen ist.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: Eine schematische Darstellung einer ersten Reinigungsapparatur;
- Fig. 2: Eine Detailansicht von Fig. 1, welche die Sensoreinheit zur Bestimmung der stofflichen Beschaffenheit der Reinigungsflüssigkeit zeigt;
- Fig. 3: Eine Detailansicht von Fig. 1, welche die Sensoreinheit zur Bestimmung des physikalischen Zustands der Reinigungsflüssigkeit zeigt;
- Fig. 4: Eine schematische Darstellung eines auf der ersten Reinigungsapparatur durchgeführten Reinigungsverfahrens;
- Fig. 5: Ein zweite Reinigungsapparatur, welche über ein Kreislaufsystem für das Reinigungsmittel verfügt;
- Fig. 6: Eine dritte Reinigungsapparatur mit einem von vorne zugänglichen Aufnahmebereich für sechs medizintechnische Hohlinstrumente;
- Fig. 7: Eine Ansicht der dritten Reinigungsapparatur aus Fig. 6 von vorne, mit aufgeschnittener Frontseite;
- Fig. 8: Eine Ansicht der dritten Reinigungsapparatur aus Fig. 6 von hinten, mit abmontierter Rückwand;
- Fig. 9: Eine Ansicht der dritten Reinigungsapparatur aus Fig. 6 von der Seite, mit abmontierter Seitenwand;
- Fig. 10: Eine Detailansicht der Schlauchstücke und Verbindungsstücke aus Fig. 6;
- Fig. 11: Eine vierte Reinigungsapparatur, welche zur gleichzeitigen Reinigung von medizintechnischen Hohlinstrumenten und anderen medizintechnischen Geräten ausgebildet ist;
- Fig. 12: Eine Ansicht auf die vierte Reinigungsapparatur aus Fig. 11 von vorne, bei abmontierter Frontseite;
- Fig. 13: Eine fünfte Reinigungsapparatur, welche über ein Kreislaufsystem und ein Zwischenreservoir für das Reinigungsmittel verfügt.

Grundsätzlich sind in den Figuren gleiche Teile mit gleichen Bezugszeichen versehen.

### Wege zur Ausführung der Erfindung

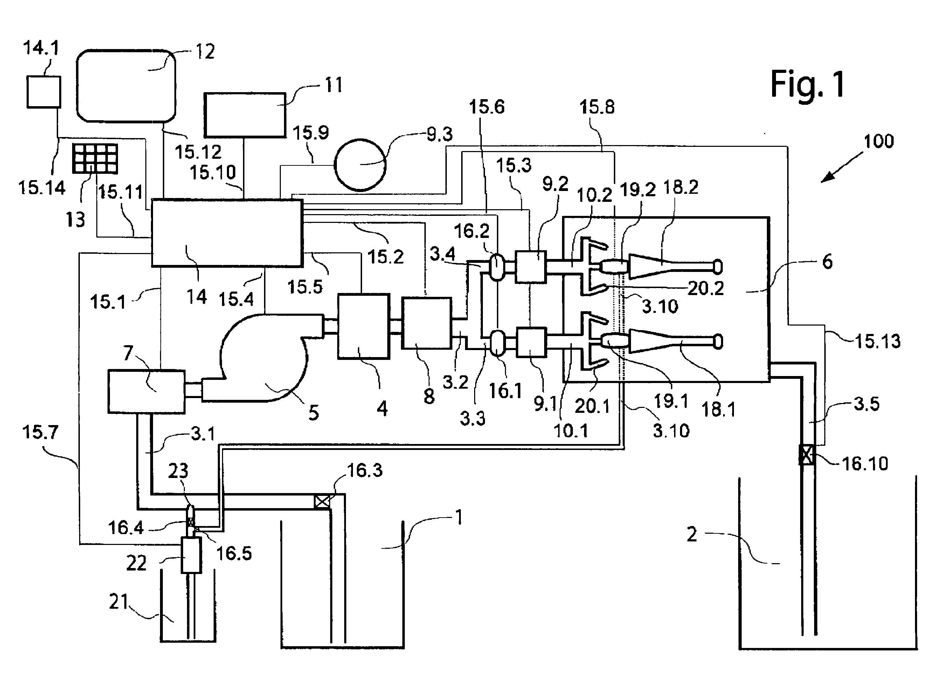

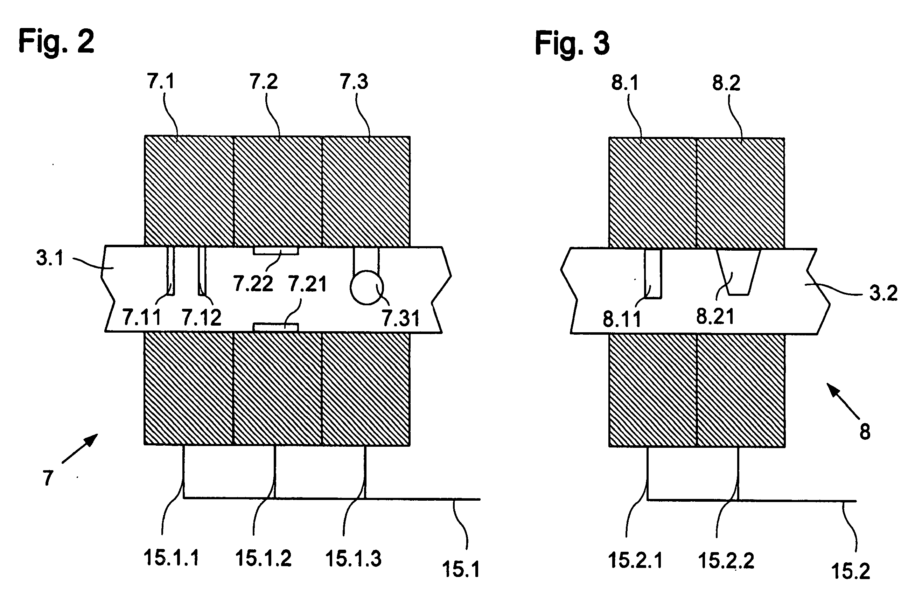

In Fig. 1 ist eine schematische Darstellung einer ersten Reinigungsapparatur 100 dargestellt. Im linken unteren Bereich von Fig. 1 ist dabei ein Flüssigkeitsbehälter 1 für eine Reinigungsflüssigkeit angeordnet. In den Flüssigkeitsbehälter 1 ragt ein Ansaugrohr 3.1, welches zur Entnahme der Reinigungsflüssigkeit aus dem Flüssigkeitsbehälter 1 vorgesehen ist. Das Ansaugrohr 3.1 ist über ein darin integriertes erstes Einlassventil 16.3 verschliessbar und mündet in eine Stoffbeschaffenheits-Sensoreinheit 7 zur Bestimmung der stofflichen Beschaffenheit der Reinigungsflüssigkeit. Der Aufbau der Stoffbeschaffenheits-Sensoreinheit 7 ist in Fig. 2 im Detail gezeigt. Austrittsseitig ist an der Stoffbeschaffenheits-Sensoreinheit 7 über ein kurzes Rohrstück eine Pumpe 5 angeschlossen. Die Pumpe 5 ist, ebenfalls über ein kurzes Rohrstück, ausgangsseitig mit einer Heizeinheit 4 verbunden. Auf die Heizeinheit 4 folgt alsdann eine Zustands-Sensoreinheit 8 zur Bestimmung eines physikalischen Zustands der Reinigungsflüssigkeit. Der Aufbau der Zustands-Sensoreinheit 8 ist in Fig. 3 im Detail gezeigt.

Mit der Pumpe 5 kann die im Flüssigkeitsbehälter 1 vorliegende Reinigungsflüssigkeit angesogen werden, so dass sie durch das Ansaugrohr 3.1 in die Stoffbeschaffenheits-Sensoreinheit 7 geleitet wird. Aus der Stoffbeschaffenheits-Sensoreinheit 7 wird die Reinigungsflüssigkeit sodann durch die Pumpe 5 und die Heizeinheit 4 gefördert und gelangt von da in die Zustands-Sensoreinheit 8 zur Bestimmung des physikalischen Zustands der Reinigungsflüssigkeit.

Austrittseitig ist an der Zustands-Sensoreinheit 8 eine erste Rohrverzweigung 3.2 angeordnet, welche in ein erstes Zufuhrrohr 3.3 und in ein zweites Zufuhrrohr 3.4 mündet. Im ersten Zufuhrrohr 3.3 ist ein erstes Absperrventil 16.1 integriert, welches den Verschluss des ersten Zufuhrrohrs 3.3 ermöglicht. Direkt auf das erste Absperrventil 16.1 folgend mündet das erste Zufuhrrohr 3.3 in ein erstes Durchflussmessgerät 9.1 zur Bestimmung eines fluiddynamischen Parameters der Reinigungsflüssigkeit. Austrittsseitig ist am ersten Durchflussmessgerät 9.1 eine zweite Rohrverzweigung 10.1 mit drei ersten Spritzdüsen 20.1 angeordnet, wobei die drei ersten Spritzdüsen 20.1 in einen Aufnahmeraum 6 für medizintechnische Geräte münden. An der mittleren Spritzdüse der drei ersten Spritzdüsen 20.1 ist zudem ein erstes Verbindungsstück 19.1 angebracht, über welches ein erstes zu reinigendes medizintechnisches Hohlinstrument 18.1 angeschlossen ist. Die mittlere Spritzdüse der drei ersten Spritzdüsen 20.1 kommuniziert dabei mit den Fluidkanälen des ersten medizintechnischen Hohlinstruments 18.1. Die beiden freien Spritzdüsen der ersten drei Spritzdüsen 20.1 sind auf das erste zu reinigende medizintechnische Hohlinstrument 18.1 gerichtet, welches zudem vollständig innerhalb des Aufnahmeraums 6 vorliegt.

Im zweiten Zufuhrrohr 3.4 ist entsprechend ein zweites Absperrventil 16.2 integriert, welches den Verschluss des zweiten Zufuhrrohrs 3.4 ermöglicht. Direkt auf das zweite Absperrventil 16.2 folgend mündet das zweite Zufuhrrohr 3.4 in ein zweites Durchflussmessgerät 9.2. Austrittsseitig ist am zweiten Durchflussmessgerät 9.2 eine dritte Rohrverzweigung 10.2 mit drei weiteren Spritzdüsen 20.2 angeordnet, wobei die drei weiteren Spritzdüsen 20.2 ebenfalls in den Aufnahmeraum 6 für medizintechnische Geräte münden. An der mittleren Spritzdüse der drei weiteren Spritzdüsen 20.2 ist zudem ein zweites Verbindungsstück 19.2 angebracht, über welches ein zweites zu reinigendes medizintechnisches Hohlinstrument 18.2 angeschlossen ist. Die mittlere Spritzdüse der drei weiteren Spritzdüsen 20.2 kommuniziert mit den Fluidkanälen des zweiten medizintechnischen Hohlinstruments 18.2. Die beiden freien Spritzdüsen der drei weiteren Spritzdüsen 20.2 sind auf das zweite zu reinigende medizintechnische Hohlinstrument 18.2 gerichtet, welches ebenfalls vollständig innerhalb des Aufnahmeraums 6 vorliegt.

Die beiden Verbindungsstücke 19.1, 19.2 sind zudem je mit einem in Fig. 1 nicht dargestellten Kupplungssensor ausgestattet, welcher die korrekte Ankoppelung der beiden medizintechnischen Hohlinstrumente 18.1, 18.2 überprüft. Das erste Absperrventil 16.1 wird dabei aufgrund eines Signals des am ersten Verbindungsstücks 19.1 angeordneten Kupplungssensors geöffnet oder geschlossen. Ist das erste medizintechnische Hohlinstrument 18.1 korrekt am ersten Verbindungsstück 19.1 angekoppelt, wird das erste Absperrventil 16.1 geöffnet. Bei falsch angeschlossenem oder fehlendem ersten medizintechnischen Hohlinstrument 18.1 wird das erste Absperrventil 16.1 geschlossen.

Das zweite Absperrventil 16.2 wird in analoger Weise aufgrund des Sensorsignals des am zweiten Verbindungsstück 19.2 angeordneten Kupplungssensors entsprechend gesteuert.

Die beiden Durchflussmessgeräte 9.1, 9.2 sind so ausgebildet, dass sie die ab einem Startzeitpunkt durchgeflossene Menge an Reinigungsflüssigkeit aufsummieren.

Der Aufnahmebereich 6 ist wannenförmig ausgebildet und während dem Betrieb der ersten Reinigungsapparatur 100 im Allgemeinen vollständig mit Reinigungsflüssigkeit gefüllt, so dass die zu reinigenden medizintechnischen Hohlinstrumente 18.1, 18.2 vollständig in die Reinigungsflüssigkeit eingetaucht vorliegen. Aus dem Aufnahmeraum 6 führt ein Abflussrohr 3.5 in ein Auffanggefäss 2, welches der Aufnahme der verbrauchten Reinigungsflüssigkeit dient.

Im Abflussrohr 3.5 ist des Weiteren ein stufenloses Regulierventil 16.10 angeordnet. Damit lässt sich der Füllstand im Aufnahmebereich 6 kontrollieren, indem die aus dem Aufnahmebereich 6 abfliessende Menge an Reinigungsflüssigkeit gesteuert wird. Bei vollständig geöffnetem Regulierventil 16.10 wird der Aufnahmebereich 6 vollständig entleert. Bei geschlossenem Regulierventil 16.10 steigt der Füllstand im Aufnahmebereich 6 kontinuierlich an. Wird das Regulierventil so eingestellt, dass die aus den Aufnahmebereich 6 abfliessende Reinigungsflüssigkeit gerade der durch die Spritzdüsen 19.1, 19.2 in den Aufnahmebereich hinein geförderten Menge an Reinigungsflüssigkeit entspricht, bleibt der Füllstand im Aufnahmebereich 6 konstant.

Während einem Reinigungs- und/oder Desinfektionsvorgang ist die Pumpe 5, ausser bei einer Störung, ständig in Betrieb und fördert kontinuierlich Reinigungsflüssigkeit vom Flüssigkeitsbehälter 1 durch die Aufnahmevorrichtung 6 und die medizinischen Hohlinstrumente 18.1, 18.2 in das Auffanggefäss 2.

Des Weiteren mündet im Bereich zwischen dem ersten Einlassventil 16.3 und der Stoffbeschaffenheits-Sensoreinheit 7 ein Einlassstutzen 23 in das Ansaugrohr 3.1. Der Einlassstutzen 23 ist mit einem zweiten Einlassventil 16.4 verschliessbar. Das zweite Einlassventil 16.4 ist eingangsseitig mit einer universellen Fluidpumpe 22 verbunden. Aus einem weiteren Behälter 21 kann mit der universellen Pumpe 22 beispielsweise ein Reinigungsmittelkonzentrat angesaugt und durch das zweite Einlassventil 16.4 in die Ansaugleitung 3.1 eingebracht werden. Damit kann die stoffliche Beschaffenheit des Reinigungsmittels verändert werden.

Zwischen dem zweiten Einlassventil und der universellen Fluidpumpe 22 zweigt zudem eine Fluidleitung 3.10 ab, welche in die beiden Verbindungsstücke 19.1, 19.2 für die medizintechnischen Hohlinstrumente 18.1, 18.2 mündet, so dass die Fluidleitung 3.10 mit den Fluidkanälen in den beiden medizintechnischen Hohlinstrumenten 18.1, 18.2 kommuniziert. Die Fluidleitung 3.10 lässt sich mit einem dritten Einlassventil 16.5 absperren und öffnen. Damit kann ein beliebiges Fluid über die Fluidleitung 3.10 direkt in die beiden Verbindungsstücke 19.1, 19.2 eingeleitet werden. Als Fluid kann z. B. ein Gas, insbesondere Luft, eingeleitet werden, um z. B. die Fluidkanäle der medizintechnischen Hohlinstrumente 18.1, 18.2 von Reinigungsflüssigkeit zu befreien. Es ist aber auch möglich, anstelle oder zusätzlich zum Gas ein Schmiermittel, z. B. ein ölhaltiges Aerosol einzuleiten, so dass die medizintechnischen Hohlinstrumente innwendig geschmiert werden können.

Zur Steuerung verfügt die Reinigungsapparatur 100 über eine Steuereinheit 14. Diese umfasst einen Mikroprozessor zur Datenverarbeitung und mehrere Schnittstellen zur Ein-und Ausgabe von Messdaten oder Steuersignalen.

Die Steuereinheit 14 ist über eine erste Signalleitung 15.1 mit der Stoffbeschaffenheits-Sensoreinheit 7 zur Bestimmung der stofflichen Beschaffenheit der Reinigungsflüssigkeit verbunden. Ebenso verläuft zwischen der Steuereinheit 14 und der Zustands-Sensoreinheit 8 eine zweite Signalleitung 15.2, und zwischen der Steuereinheit 14 und den Durchflussmessgeräten 9.1, 9.2 ist eine dritte Signalleitung 15.3 angeordnet.

Zwischen der Pumpe 5 und der Steuereinheit ist eine vierte Signalleitung 15.4 angeordnet, während die Heizeinheit 4 über eine fünfte Signalleitung 15.5 mit der Steuereinheit 14 verbunden ist.

Die beiden Absperrventile 16.1, 16.2 in den beiden Zufuhrrohren 3.3, 3.4 sind über eine sechste Signalleitung 15.6 mit der Steuereinheit 14 verbunden und lassen sich daher durch die Steuereinheit 14 öffnen und schliessen.

Ebenso ist die universelle Fluidpumpe 22 über eine siebte Signalleitung 15.7 mit der Steuereinheit 14 verbunden. Damit lässt sich z. B. die Reinigungsflüssigkeit durch eine automatische Zugabe eines Reinigungsmittelkonzentrats modifizieren. Sämtliche Einlassventile 16.3, 16.4, 16.5 können ebenfalls durch die siebte Signalleitung 15.7 betätigt werden.

Die beiden Verbindungsstücke 19.1, 19.2 für die medizinischen Hohlinstrumente 18.1, 18.2 sind über eine achte Signalleitung 15.8 mit der Steuereinheit 14 verbunden, so dass z. B. ein Zustand eines Kopplungssensors in den Verbindungsstücken 19.1, 19.2 durch die Steuereinheit 14 erfasst werden kann. Dadurch lässt sich z. B. mit der Steuereinheit 14 prüfen, ob die medizinischen Hohlinstrumente 18.1, 18.2 korrekt an die Verbindungsstücke 19.1, 19.2 angekoppelt sind.

Des Weiteren ist die Steuereinheit über eine neunte Signalleitung 15.9 mit einer Zeitmesseinrichtung 9.3 verbunden. Die Zeitmesseinrichtung 9.3 lässt sich durch die Steuereinheit auf einen vorbestimmten Startwert, insbesondere auf den Wert Null zurücksetzen und anschliessend starten. Damit kann die seit dem Zurücksetzen verstrichene Zeit durch die Steuereinheit 14 ausgelesen werden. Die Zeitmesseinrichtung 9.3 funktioniert analog zu einer Stoppuhr.

Über eine zehnte Signalleitung 15.10 ist eine Speichereinheit 11 mit der Steuereinheit verbunden. In der Speichereinheit 11 sind in einem ersten Bereich mehrere unterschiedliche und verschiedenen Reinigungsflüssigkeiten und/oder medizintechnischen Geräten zugeordnete Programme zur Steuerung der Reinigungsapparatur abgelegt. In einem zweiten Speicherbereich sind mehrere Vergleichswerte abgelegt, welche zur Beurteilung der durch die Sensoreinheiten 7, 8 bzw. die Durchflussmessgeräte 9.1, 9.2 ermittelten Messwerte benötigt werden. In einem dritten Bereich der Speichereinheit 1 1 sind schliesslich sämtliche von der Steuereinheit 14 erfassten Messsignale und ausgegebenen Steuerbefehle abgelegt. In einem weiteren Speicherbereich der Speichereinheit 11 können zudem Zugangsdaten für verschiedene Benutzer mit Passwörtern abgelegt sein. Ebenso sind die Daten der letzten Überprüfung bzw. Validierung der Reinigungsapparatur und das Datum der nächsten durchzuführenden Überprüfung bzw. Validierung abgelegt. Ebenso kann in einem Speicherbereich der Speichereinheit 11 das aktuelle Datum und die Uhrzeit erfasst werden.

Eine Eingabevorrichtung 13 in Form einer Tastatur ist über eine elfte Signalleitung 15.11 an die Steuereinheit 14 angeschlossen. Entsprechend findet sich bei der Reinigungsapparatur 100 auch eine Ausgabevorrichtung 12 in Form eines Bildschirms, welche über eine zwölfte Signalleitung 15.12 mit der Steuereinheit 14 verbunden ist. Über die Eingabevorrichtung 13 können beispielsweise die in der Speichereinheit 11 abgelegten Programme ausgewählt werden, wobei der Vorgang auf der Ausgabevorrichtung 12 angezeigt wird. Über die Ausgabevorrichtung 12 können dem Benutzer sämtliche Informationen betreffend die Reinigungs- und/oder Desinfektionsvorgang bzw. allfällige Störungen angezeigt werden.

Über eine dreizehnte Steuerleitung 15.13 ist die Steuereinheit 14 zudem mit dem Regulierventil 16.10 verbunden. Das Regulierventil 16.10 lässt sich somit durch die Steuereinheit 14 steuern. Je nach durchzuführendem Reinigungsverfahren, kann die Steuereinheit dabei den Füllstand im Aufnahmebereich 6 kontrollieren. Hierfür ist ein in Fig. 1 nicht dargestellter Füllstandssensor im Aufnahmebereich 6 angeordnet, über welchen die Steuereinheit 14 den aktuellen Füllstand des Aufnahmebereichs 6 ermitteln kann.

Eine vierzehnte Steuerleitung 15.14 verbindet die Steuereinheit 14 des Weiteren mit einer Kommunikationsschnittstelle 14.1, z. B. eine USB-Schnittstelle, welche der Datenübertragung zwischen der Steuereinheit 14 und einem in Fig. 1 nicht dargestellten externen Gerät dient.

Des Weiteren verfügt die Reinigungsapparatur 100 über offensichtlich für den Betrieb notwendige Komponenten, wie z. B. Stromversorgungseinrichtungen, welche in Fig. 1 der Übersichtlichkeit halber aber nicht eingezeichnet sind.

In Fig. 2 ist die Sensoreinheit 7 zur Bestimmung der stofflichen Beschaffenheit der Reinigungsflüssigkeit aus Fig. 1 schematisch im Querschnitt dargestellt. Die Sensoreinheit 7 zur Bestimmung der stofflichen Beschaffenheit der Reinigungsflüssigkeit besteht dabei aus einem Leitfähigkeitssensor 7.1, einem Fotometer 7.2 und einem pH-Sensor 7.3, wobei diese in einer seriellen Anordnung vorliegen.

Beim Leitfähigkeitssensor 7.1 ragen eine erste metallische Elektrode 7.11 und eine zweite metallische Elektrode 7.12 in einem vorgegebenen Abstand und parallel zueinander ausgerichtet in den zentralen Innenraum des Ansaugrohrs 3.1. Die beiden elektrisch leitenden Elektroden 7.11, 7.12 weisen eine definierte Fläche auf und tauchen vollständig in die im Ansaugrohr 3.1 fliessende Reinigungsflüssigkeit ein. Zur Bestimmung der Leitfähigkeit wird der ohmsche Widerstand, bzw. dessen Kehrwert (der elektrische Leitwert) mit Wechselstrom gemessen. Die Leitfähigkeit der Reinigungsflüssigkeit hängt bei gegebener geometrischer Anordnung der Elektroden insbesondere von der Art und Konzentration der in der Reinigungsflüssigkeit vorhandenen Ionen ab. Zur Übermittlung der vom Leitfähigkeitssensor 7.1 ermittelten Messwerte an die Steuereinheit 14 ist der Leitfähigkeitssensor 7.1 an einen ersten elektrischen Kontakt 15.1.1 der ersten Signalleitung 15.1 angeschlossen.

Der Fotometer 7.2 umfasst eine Leuchtdiode 7.2.1 und eine Fotodiode 7.2.2, welche direkt gegenüberliegend an der Innenwand des Ansaugrohrs 3.1 angeordnet sind. Licht, welches von der Leuchtdiode 7.21 ausgesendet wird, passiert die im Ansaugrohr fliessende Reinigungsflüssigkeit und wird von der Fotodiode 7.22 detektiert. Beim Passieren des Lichts durch die Reinigungsflüssigkeit, wird das Licht teilweise von den Molekülen in der Reinigungsflüssigkeit absorbiert, gestreut und an den Grenzflächen reflektiert. Die von der Fotodiode detektierte Lichtintensität hängt im Wesentlichen von der Beschaffenheit der Reinigungsflüssigkeit ab. Zur Übermittlung der vom Fotometer 7.2 ermittelten Messwerte an die Steuereinheit 14 ist der Fotometer 7.2 an einen zweiten elektrischen Kontakt 15.1.2 der ersten Signalleitung 15.1 angeschlossen.

Der pH-Sensor 7.3 umfasst beispielsweise eine an sich bekannte Einstabmesskette 7.31 mit einer Glaselektrode und einer Bezugselektrode in Form einer Silber-Silberchlorid-Halbzelle. Die Einstabmesskette ragt dabei in einen zentralen Bereich im Innern des Ansaugrohrs 3.1 und wird von der fliessenden Reinigungsflüssigkeit umspült. Je nach Beschaffenheit der Reinigungsflüssigkeit wird ein anderer pH-Wert gemessen. Zur Übermittlung der vom pH-Sensor 7.3 ermittelten Messwerte an die Steuereinheit 14 ist der pH-Sensor 7.3 an einen dritten elektrischen Kontakt 15.1.3 der ersten Signalleitung 15.1 angeschlossen.

Es versteht sich, dass der Leitfähigkeitssensor 7.1, der Fotometer 7.2 und der pH-Sensor 7.3 über die zur Durchführung der Messungen erforderlichen elektrischen und/oder elektronischen Komponenten verfügt. Diese wurden der Übersichtlichkeit halber in Fig. 2 nicht dargestellt.

In Fig. 3 ist die Sensoreinheit 8 zur Bestimmung des physikalischen Zustands der Reinigungsflüssigkeit aus Fig. 1 schematisch im Querschnitt dargestellt. Die Sensoreinheit 8 zur Bestimmung des physikalischen Zustands der Reinigungsflüssigkeit besteht aus einem Temperatursensor 8.1 und einem Drucksensor 8.2, welche in serieller Weise angeordnet sind.

Der Temperatursensor 8.1 umfasst beispielsweise ein Thermoelement 8.11 aus zwei unterschiedlichen und an ihren Enden verbundenen Metallen (z. B. Nickel-Chrom/Nickel; K-Typ), welche in einen zentralen Bereich der durch die Sensoreinheit 8 zur Bestimmung des physikalischen Zustands verlaufenden Rohrleitung ragen und von der Reinigungsflüssigkeit umspült werden. Aufgrund der Temperatur der Reinigungsflüssigkeit resultiert eine Thermospannung im Thermoelement 8.11, welche zur Bestimmung der Temperatur herangezogen wird. Zur Übermittlung der vom Temperatursensor 8.1 ermittelten Messwerte an die Steuereinheit 14 ist der Temperatursensor 8.1 an einen ersten elektrischen Kontakt 15.2.1 der zweiten Signalleitung 15.2 angeschlossen.

Der Drucksensor 8.2 umfasst einen piezoelektrischen Kristall 8.21, welcher in einen zentralen Bereich der durch die Sensoreinheit 8 zur Bestimmung des physikalischen Zustands verlaufenden Rohrleitung ragt und von der Reinigungsflüssigkeit umspült wird. Je nach Flüssigkeitsdruck, welcher auf den piezoelektrischen Kristall 8.21 einwirkt, resultiert eine unterschiedliche Ladungstrennung, welche in Form einer Spannung gemessen werden kann. Zur Übermittlung der vom Drucksensor 8.2 ermittelten Messwerte an die Steuereinheit 14 ist der Drucksensor 8.2 an einen zweiten elektrischen Kontakt 15.2.2 der zweiten Signalleitung 15.2 angeschlossen.

Es versteht sich auch hier, dass der Temperatursensor 8.1 und der Drucksensor 8.2 zusätzlich über die zur Durchführung der Messungen erforderlichen elektrischen und/oder elektronischen Komponenten verfügt. Diese wurden der Übersichtlichkeit halber in Fig. 3 nicht dargestellt.

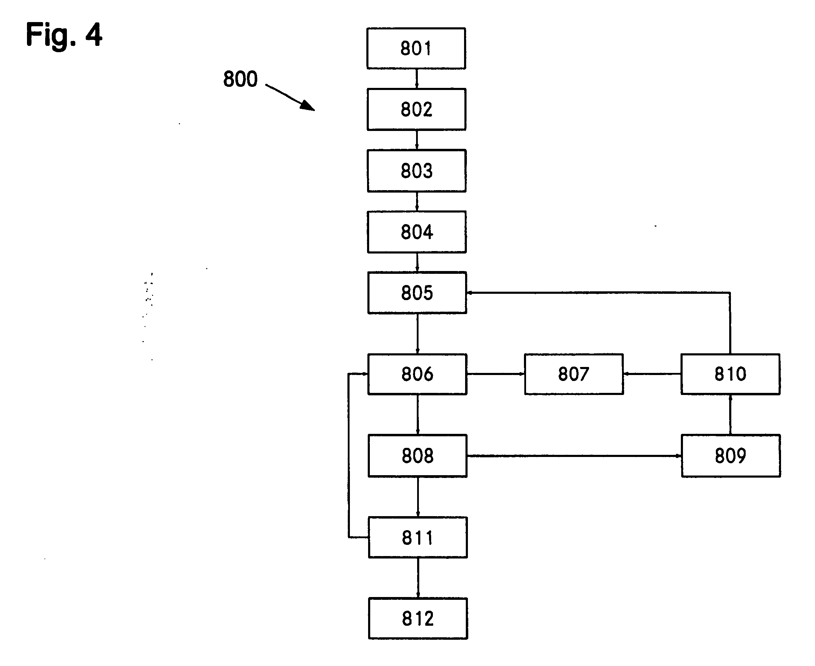

In Fig. 4 ist der Verfahrensablauf 800 bei der Reinigung von medizintechnischen Geräten mit der ersten Reinigungsapparatur 100 dargestellt.

Direkt nach dem Einschalten führt die Reinigungsapparatur 100 im ersten Verfahrensschritt 801 eine Initialisierung des gesamten Systems durch. Hierbei kann die Steuereinheit 14 z. B. die grundsätzliche Funktionsfähigkeit der elektronischen Komponenten der ersten Reinigungsapparatur 100 überprüfen.

Anschliessend folgt der zweite Verfahrensschritt 802, bei welchem auf eine Eingabe des Benutzers gewartet wird. Während dem zweiten Verfahrensschritt wird der Benutzer zuerst die zu reinigenden medizintechnischen Hohlinstrumente 18.1, 18.2 in den wannenförmigen Aufnahmebereich 6 einlegen und über die beiden Verbindungsstücke 19.1, 19.2 an die hierfür vorgesehenen beiden Spritzdüsen 20.1, 20.2 anschliessen. Darauf kann der Benutzer über die Eingabevorrichtung 13 und mit Hilfe der Ausgabevorrichtung 12 das aufgrund des Reinigungsmittels passende und in der Speichereinheit abgelegte Programm auswählen und den Reinigungsvorgang starten.

Im dritten Verfahrensschritt 803 lädt die Steuereinheit 14 die erforderlichen Vergleichswerte, welche zur Beurteilung der durch die Sensoreinheiten 7, 8 bzw. die Durchflussmessgeräte 9.1, 9.2 ermittelten Messwerte benötigt werden, aus der Speichereinheit 11. Anschliessend wird die Pumpe 5 von der Steuereinheit 14 selbsttätig gestartet, so dass die im Flüssigkeitsbehälter 1 vorliegende Reinigungsflüssigkeit angesaugt und in den Aufnahmebereich 6 bzw. durch die medizintechnischen Hohlinstrumente 18.1, 18.2 hindurch in das Auffanggefäss 2 geleitet wird.

Zu Stabilisierungszwecken der ersten Reinigungsapparatur 100, wird im vierten Verfahrensschritt 804 über einen definierten Zeitraum kontinuierlich Reinigungsflüssigkeit durch die erste Reinigungsapparatur 100 gefördert. Dabei werden noch keine Sensorwerte ausgelesen bzw. eine Überprüfung der Reinigungsflüssigkeit findet noch nicht statt. Die Förderung der Reinigungsflüssigkeit wird auch in den nachfolgenden Verfahrensschritten aufrecht erhalten, soweit nichts anderes vermerkt ist.

Im fünften Verfahrensschritt 805 wird die Zeitmesseinrichtung 9.3 zurückgesetzt und die Zeitmesseinrichtung beginnt mit der Erfassung der für den Reinigungsvorgang relevanten Zeit. Ebenso werden die Durchflussmessgeräte 9.1, 9.2 zurückgesetzt bzw. die durchgeflossene Menge an Reinigungsflüssigkeit wird auf Null festgelegt. Die Aufsummierung der durch die Durchflussmessgeräte 9.1, 9.2 geflossenen Menge an Reinigungsflüssigkeit beginnt von Neuem.

Anschliessend folgt der sechste Verfahrensschritt 806, bei welchen die Messwerte der Sensoreinheit 7 zur Bestimmung der stofflichen Beschaffenheit der Reinigungsflüssigkeit von der Steuereinheit 14 erfasst und mit den zugehörigen und vorgängig im zweiten Verfahrenschritt 802 geladenen Vergleichswerten verglichen werden. Die Sensoreinheit 7 zur Bestimmung der stofflichen Beschaffenheit der Reinigungsflüssigkeit umfasst, wie bei Fig. 2 ausgeführt, insgesamt drei unabhängige Sensoren zur Bestimmung des pH-Werts, der elektrischen Leitfähigkeit und der Lichtabsorption der Reinigungsflüssigkeit auf. Wenn einer der drei ermittelten Messwerte der drei Sensoren eine Abweichung vom Vergleichswert aufweist, welche grösser ist, als eine maximal tolerierbare Abweichung, so folgt der siebte Verfahrensschritt 807. Weisen alle drei Messwerte der drei Sensoren Abweichungen vom zugehörigen Vergleichswert auf , welche kleiner sind als die maximal tolerierbare Abweichung, so folgt der achte Verfahrensschritt 808.

Im siebten Verfahrensschritt stoppt die Steuereinheit 14 die Pumpe 5 und bricht den Reinigungsvorgang ab. Dabei wird über die Ausgabevorrichtung 12 eine Fehlermeldung ausgegeben, wobei der Benutzer über die genaue Ursache des Abbruchs informiert wird.

Im achten Verfahrensschritt 808 werden die Messwerte der Sensoreinheit 8 zur Bestimmung des physikalischen Zustands der Reinigungsflüssigkeit von der Steuereinheit 14 erfasst und mit den zugehörigen und vorgängig im zweiten Verfahrenschritt 802 geladenen Vergleichswerten verglichen. Die Sensoreinheit 8 zur Bestimmung des physikalischen Zustands der Reinigungsflüssigkeit umfasst, wie bei Fig. 3 ausgeführt, insgesamt zwei unabhängige Sensoren zur Bestimmung der Temperatur und des Drucks. Falls der Temperatur- und/oder der Druckmesswert eine Abweichung vom zugehörigen Vergleichswert aufweisen, welche grösser ist, als eine maximal tolerierbare Abweichung, so folgt der neunte Verfahrensschritt 809. Weist der Temperatur- und/oder der Druckmesswert eine Abweichung vom zugehörigen Vergleichswert auf, welche kleiner ist als die maximal tolerierbare Abweichung, so folgt der elfte Verfahrensschritt 811.

Im neunten Verfahrensschritt 809 wird die Heizleistung der Heizeinheit 4 und/oder die Pumpleistung der Pumpe 5 angepasst. Ist die durch die Sensoreinheit 8 zur Bestimmung des physikalischen Zustands der Reinigungsflüssigkeit gemessene Temperatur der Reinigungsflüssigkeit zu niedrig, wird die Heizleistung der Heizeinheit 4 durch die Steuereinheit 14 erhöht. Sollte die durch die Sensoreinheit 8 zur Bestimmung des physikalischen Zustands der Reinigungsflüssigkeit gemessene Temperatur der Reinigungsflüssigkeit zu hoch sein, wird die Heizleistung der Heinzeinheit 4 durch die Steuereinheit 14 entsprechend erniedrigt. Hat sich der durch die Sensoreinheit 8 zur Bestimmung des physikalischen Zustands der Reinigungsflüssigkeit gemessene Druck als zu niedrig erwiesen, wird die Pumpleistung der Pumpe 5 durch die Steuereinheit 14 erhöht, während bei zu hohlem Druck die Steuereinheit 14 die Pumpleistung entsprechend reduziert. Heizleistung und Pumpleistung können dabei gleichzeitig angepasst werden. Die Anpassung der Heiz- und/oder Pumpleistung erfolgt z. B. in Abhängigkeit der gemessenen Abweichungen und mit Hilfe vorbestimmter Regelparametem bzw. Regelfunktionen, auf welche die Steuereinheit 14 zurückgreift.

Im auf den neunten Verfahrensschritt 809 folgenden zehnten Verfahrensschritt 810 wird durch die Steuereinheit 14 geprüft, ob die Heizleistung und/oder die Pumpleistung bereits einen maximal oder minimal zulässigen Wert erreicht haben. Sollte dies der Fall sein, ist eine sinnvolle Regelung nicht mehr möglich und es folgt der bereits beschriebene siebte Verfahrenschritt 807, bei welchem der Reinigungsvorgang abgebrochen wird. Falls die Heizleistung und die Pumpleistung beide im Bereich zwischen dem minimal und dem maximal zulässigen Wert liegen, folgt erneut der fünfte Verfahrensschritt 805, wobei die Zeitmesseinrichtung 9.3 erneut zurückgesetzt und wieder neu gestartet wird.

Im elften Verfahrenschritt 811 wird jeder der beiden Messwerte der Durchflussmessgeräte 9.1, 9.2 von der Steuereinheit 14 erfasst und mit dem zugehörigen und vorgängig im zweiten Verfahrenschritt 802 geladenen Vergleichswert verglichen. Falls der Vergleich ergibt, dass die Messwerte der beiden Durchflussmessgeräte 9.1, 9.2 kleiner als der zugehörige Vergleichswert sind, d.h. die zu reinigenden medizintechnischen Hohlinstrumente wurden noch nicht mit der für eine wirksame Reinigung und/oder Desinfektion erforderlichen Menge an Reinigungsflüssigkeit in Kontakt gebracht, folgt erneuet der sechste Verfahrensschritt 806. Ebenso wird der von der Zeiterfassungseinrichtung 9.3 ermittelte Messwert von der Steuereinheit 14 erfasst und mit dem zugehörigen und vorgängig im zweiten Verfahrenschritt 802 geladenen Vergleichswert verglichen. Falls der Vergleich ergibt, dass der Messwert kleiner als der zugehörige Vergleichswert ist, d. h. die zu reinigenden medizintechnischen Hohlinstrumente 18.1, 18.2 wurden noch nicht über eine ausreichende Zeitdauer mit der Reinigungsflüssigkeit in Kontakt gebracht, folgt erneut der sechste Verfahrensschritt 806.

Falls sowohl die von den Durchflussmessgeräten 9.1, 9.2 gemessenen Messwerte als auch der von der Zeitmesseinrichtung 9.3 ermittelte Messwert grösser oder gleich sind als die zugehörigen Vergleichswerte, ist der Reinigungs- bzw. Desinfektionsvorgang erfolgreich abgeschlossen.

Im zwölften Verfahrenschritt 812 wird die Pumpe 5 gestoppt. Anschliessend kann durch die universelle Fluidpumpe 22 und die Fluidleitung 3.10 Luft in die Fluidkanäle der medizintechnischen Hohlinstrumente eingeleitet werden, um die Fluidkanäle von Reinigungsmittel zu befreien. Das Öffnen und Schliessen der Absperr- und Einlassventile, sowie die Betätigung der universellen Fluidpumpe 22 kann ebenfalls automatisch durch die Steuereinheit 14 erfolgen. Der Abschluss des Reinigungsvorgangs kann dem Benutzer anschliessend über die Ausgabevorrichtung 12 mitgeteilt werden.

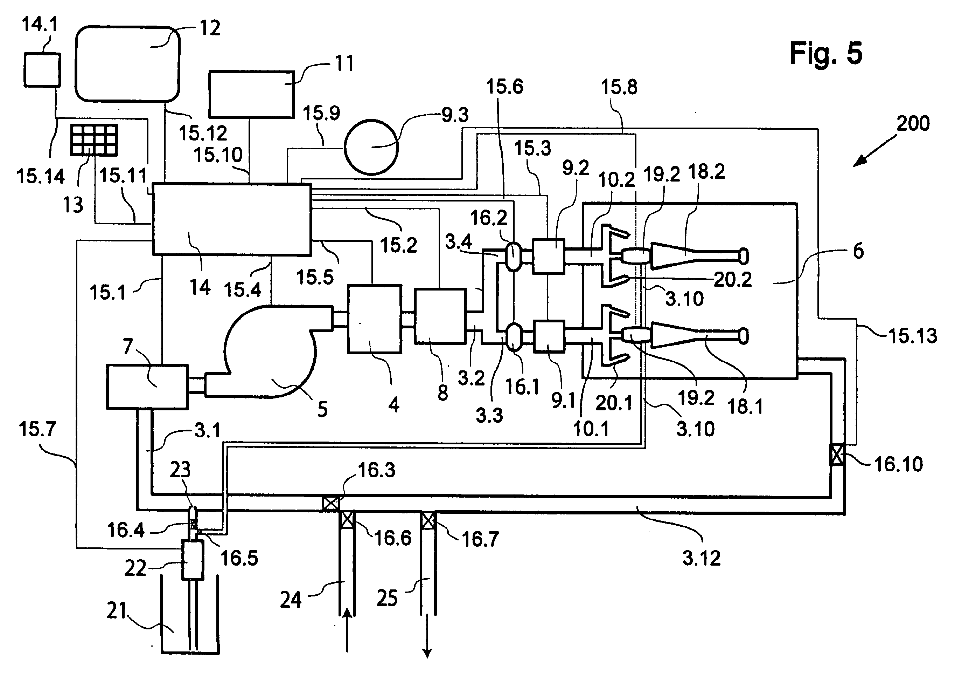

Fig. 5 zeigt eine schematische Darstellung einer zweiten Reinigungsapparatur 200. Die zweite Reinigungsapparatur 200 entspricht im Wesentlichen der ersten Reinigungsapparatur 100. Im Unterschied zur ersten Reinigungsapparatur 100 ist bei der zweiten Reinigungsapparatur 200 aber kein Flüssigkeitsbehälter 1 für eine Reinigungsflüssigkeit angeordnet und ein Auffanggefäss 2 zur Aufnahme der verbrauchten Reinigungsflüssigkeit liegt auch nicht vor. Stattdessen führt bei der zweiten Reinigungsapparatur 200 ein Verbindungsrohr 3.12 aus dem Aufnahmebereich 6 direkt zum ersten Einlassventil 16.3. Somit ist das Verbindungsrohr 3.12 direkt mit dem Ansaugrohr 3.1 verbunden. Reinigungsflüssigkeit, welche aus dem Aufnahmebereich 6 abfliesst, gelangt somit über das Verbindungsrohr 3.12 wieder in das Ansaugrohr 3.1. Die Reinigungsflüssigkeit kann somit in einem geschlossenen Kreislauf zirkulieren und während einem Reinigungs- und/oder Desinfektionsvorgang fortlaufend mit den zu reinigenden medizintechnischen Hohlinstrumenten 18.1, 18.2 in Kontakt gebracht werden. Insbesondere aufgrund der Sensoreinheit 7 zur Bestimmung der stofflichen Beschaffenheit der Reinigungsflüssigkeit kann dabei fortlaufend überprüft werden, ob die Reinigungsflüssigkeit den Anforderungen noch genügt.

Des Weiteren ist bei der zweiten Reinigungsapparatur 200 im Verbindungsrohr 3.12, in einem Bereich zwischen dem ersten Einlassventil 16.3 und dem Aufnahmebereich 6, ein Einlassstutzen 24 und ein Auslassstutzen 25 angeordnet, welche beide mit dem Verbindungsrohr 3.12 kommunizieren. Der Einlassstutzen 24 kann mit einem darin angeordneten vierten Einlassventil 16.6 geschlossen und geöffnet werden. Ebenso kann der Auslassstutzen durch ein Auslassventil 16.7 verschlossen oder geöffnet werden.

Sollte die Reinigungsflüssigkeit beispielsweise mit Fremdstoffen verunreinigt sein, so kann das Auslassventil 16.7 geöffnet werden, so dass die Reinigungsflüssigkeit aus der zweiten Reinigungsapparatur 200 entnommen werden kann. Anschllessend wird das Auslassventil 16.7 wieder geschlossen und das vierte Einlassventil 16.6 wird geöffnet. Sodann kann die zweite Reinigungsapparatur 200 über den Einlassstutzen 25 mit frischer und unverbrauchter Reinigungsflüssigkeit befüllt werden.

Der wannenförmige Aufnahmebereich 6, welcher während dem Betrieb der ersten Reinigungsapparatur 100 im Allgemeinen vollständig mit Reinigungsflüssigkeit gefüllt ist, dient bei der zweiten Reinigungsapparatur 200 zusätzlich als Ausgleichsbecken für die Reinigungsflüssigkeit. Damit wird insbesondere verhindert, dass während dem Betrieb der zweiten Reinigungsapparatur 200 Luft in die flüssigkeitsführenden Komponenten, wie z. B. die verschiedenen Rohre 3.1, 3.12, 3.3, 3.4 und die Spritzdüsen 20.1, 20.2, bzw. in den Flüssigkeitskreislauf gelangt.

Die Ansteuerung der zweiten Reinigungsapparatur 200 erfolgt in analoger Weise, wie bei der ersten Reinigungsapparatur 100.

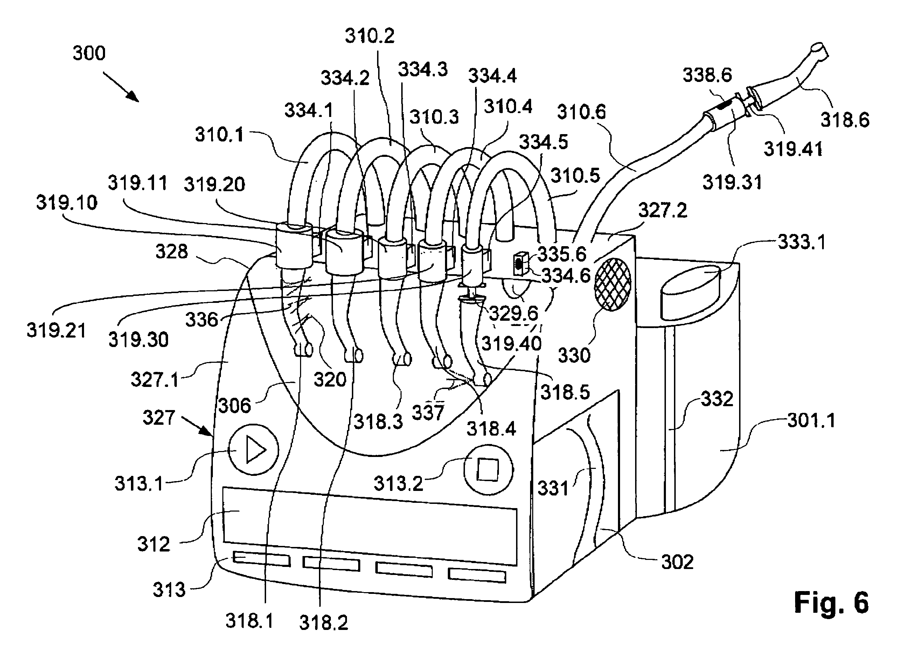

In Fig. 6 ist eine dritte Reinigungsapparatur 300 in einer perspektivischen Darstellung von schräg vorne abgebildet. Die dritte Reinigungsapparatur 300 umfasst ein in einem vorderen Bereich im Wesentlichen quaderförmiges Gehäuse 327, wobei sich die Frontseite 327.1 mit zunehmender Höhe nach hinten wölbt. Im untersten Bereich der Frontseite 327.1 ist eine Tastatur 313 mit vier rechteckförmigen Tasten angebracht. Darüber befindet sich eine Ausgabevorrichtung 312 in Form einer Flüssigkristallanzeige, welche sich über nahezu die gesamte Breite der Frontseite 327.1 erstreckt. Oberhalb der Ausgabevorrichtung 312 ist links an der Frontseite 327.1 eine Starttaste 313.1 angeordnet und rechts an der Frontseite 327.1 ist eine Stopptaste 313.2 angebracht. Die Tastatur 313 mit den vier Tasten, die Starttaste 313.1 und die Stopptaste 313.2 bilden zusammen die Eingabevorrichtung der dritten Reinigungsapparatur 300. In einem oberen Bereich der Frontseite 327.1 ist eine nach unten gerichtete und zulaufende zungenförmige Abdeckhaube 328 aus einem transparenten Material eingebracht. Die Abdeckhaube 328 deckt den im Innern der dritten Reinigungsapparatur 300 liegenden Aufnahmebereich 306 für die medizintechnischen Geräte ab. Die Abdeckhaube 328 kann über ein nicht dargestelltes Scharnier, welches die Abdeckhaube 328 im Bereich der unteren mit der Frontseite 327.1 verbindet, nach vorne aufgeklappt werden. In Fig. 6 ist die Abdeckhaube 328 in der geschlossenen Position gezeigt.

An der horizontalen Oberseite 327.2 des vorderen und im Wesentlichen quaderförmigen Bereichs des Gehäuses 327 sind in einem vorderen Bereich insgesamt sechs gleichartige und quaderförmige Halteblöcke 334.1, 334.2...334.6 in regelmässigen Abständen angeordnet. Der in Fig. 5 vollständig sichtbare sechste Halteblock 334.6 verfügt an seiner Vorderseite über einen Permanentmagneten 335.6. Auch die übrigen fünf Halteblöcke 334.1...334.5 verfügen über entsprechende Permanentmagnete, welche allerdings in Fig. 5 nicht sichtbar sind.

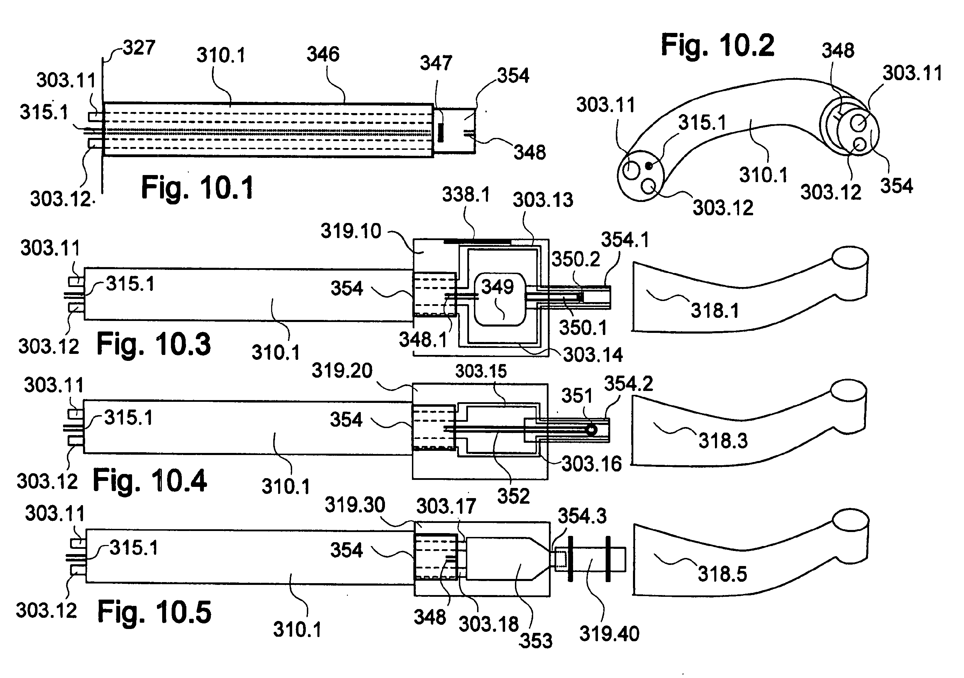

Aus einem hinteren Bereich der horizontalen Oberseite 327.2 des Gehäuses 327 ragen insgesamt sechs flexible Schlauchstücke 310.1, 310.2...310.6 aus dem Innern des Gehäuses 327. Die Schlauchstücke sind in den Fig. 10.1 - 10.5 im Detail gezeigt. Das von links her betrachtet erste Schlauchstück 310.1 ist zum ersten Halteblock 334.1 nach vorne gebogen und an seinem ausserhalb des Gehäuses 327 liegenden Ende mit einem ersten Verbindungsstück 319.10, welches zylinderförmig ausgebildet ist, verbunden. Im Bereich des dem ersten Schlauchstück 310.1 abgewandten Endes ist am ersten Verbindungsstück 319.10 ein erstes medizintechnisches Hohlinstrument 318.1 befestigt. Das erste Verbindungsstück 319.10 ist am ersten Halteblock 334.1 fixiert. Die Fixierung erfolgt dabei über einen in Fig. 5 nicht sichtbaren Gegenmagnet am ersten Verbindungsstück 319.10, welcher mit dem Permanentmagnet des ersten Halteblocks 334.1 wechselwirkt, so dass aufgrund der Magnetkräfte eine stabile Halterung des Verbindungsstücks 319.10 am ersten Halteblock 334.1 resultiert.

Entsprechend ist das von links her betrachtet zweite Schlauchstück 310.2 zum zweiten Halteblock 334.2 nach vorne gebogen und an seinem ausserhalb des Gehäuses 327 liegenden Ende mit einem zweiten Verbindungsstück 319.11, welches zylinderförmig ausgebildet ist, verbunden. Im Bereich des dem zweiten Schlauchstück 310.2 abgewandten Endes ist am zweiten Verbindungsstück 319.11 ein zweites medizintechnisches Hohlinstrument 318.2 befestigt. Das zweite Verbindungsstück 319.11 ist am zweiten Halteblock 334.2 fixiert, wobei die Fixierung wie beim ersten Verbindungsstück 319.10 über einen in Fig. 5 nicht sichtbaren Gegenmagnet erfolgt.

Ebenso ist das von links her betrachtet dritte Schlauchstück 310.3 zum dritten Halteblock 334.3 nach vorne gebogen und an seinem ausserhalb des Gehäuses 327 liegenden Ende mit einem dritten Verbindungsstück 319.20, welches zylinderförmig ausgebildet ist, verbunden. Im Bereich des dem dritten Schlauchstück 310.3 abgewandten Endes ist am dritten Verbindungsstück 319.20 ein drittes medizintechnisches Hohlinstrument 318.3 befestigt. Das dritte Verbindungsstück 319.20 ist am dritten Halteblock 334.3 fixiert, wobei die Fixierung wie beim ersten Verbindungsstück 319.10 über einen in Fig. 6 nicht sichtbaren Gegenmagnet erfolgt.

Das von links her betrachtet vierte Schlauchstück 310.4 ist entsprechend zum vierten Halteblock 334.4 nach vorne gebogen und an seinem ausserhalb des Gehäuses 327 liegenden Ende mit einem vierten Verbindungsstück 319.21, welches zylinderförmig ausgebildet ist, verbunden. Im Bereich des dem vierten Schlauchstück 310.4 abgewandten Endes ist am vierten Verbindungsstück 319.21 ein viertes medizintechnisches Hohlinstrument 318.4 befestigt. Das vierte Verbindungsstück 319.21 ist am vierten Halteblock 334.4 fixiert, wobei die Fixierung wie beim ersten Verbindungsstück 319.10 über einen in Fig. 5 nicht sichtbaren Gegenmagnet erfolgt.

In gleicher Weise ist das von links her betrachtet fünfte Schlauchstück 310.5 zum fünften Halteblock 334.5 nach vorne gebogen und an seinem ausserhalb des Gehäuses 327 liegenden Ende mit einem fünften Verbindungsstück 319.30, welches zylinderförmig ausgebildet ist, verbunden. Im Bereich des dem fünften Schlauchstück 310.5 abgewandten Endes ist am fünften Verbindungsstück 319.30 ein erster Kupplungsadapter 319.40 angeordnet, über welchen ein fünftes medizintechnisches Hohlinstrument 318.5 an das fünfte Verbindungsstück 319.30 angeschlossen ist. Das fünfte Verbindungsstück 319.30 ist am fünften Halteblock 334.5 fixiert, wobei die Fixierung wie beim ersten Verbindungsstück 319.10 über einen in Fig. 5 nicht sichtbaren Gegenmagneten erfolgt.

Die Abdeckhaube weist im Bereich der oberen Kante insgesamt sechs zungenförmige Ausnehmungen auf. Die ersten fünf medizintechnischen Hohlinstrumente 318.1, 318.2...318.5 ragen entsprechend durch die ersten fünf in Fig. 5 nicht sichtbaren Ausnehmungen in der Abdeckhaube 328 nach unten in den Aufnahmebereich 306.

Hinter den in den Aufnahmebereich 306 ragenden fünf ersten medizintechnischen Hohlinstrumente 318.1...318.5 sind zudem in der rückseitigen Wand des Aufnahmebereichs 306 eine Vielzahl von Sprühdüsen 320 angeordnet. Die Sprühdüsen 320 sind der Übersichtlichkeit halber in Fig. 6 lediglich exemplarisch im Bereich des ersten medizintechnischen Hohlinstruments dargestellt. Es versteht sich aber, dass sämtliche Innenwände des Aufnahmebereichs 306 mit einer Vielzahl Sprühdüsen 320 bestückt ist. Die Sprühdüsen 320 erzeugen konische Sprühstrahlen 336 aus Reinigungsflüssigkeit, welche auf die in den Aufnahmebereich 306 hinein ragenden medizintechnischen Hohlinstrumente 318.1...318.5 gerichtet sind und diese im Bereich ihrer äusseren Oberfläche rundherum vollständig benetzen.

Die durch die Innenbereiche bzw. Fluidkanäle der in den Aufnahmebereich 306 hinein ragenden medizintechnischen Hohlinstrumente 318.1...318.5 geleitete Reinigungsflüssigkeit tritt im Bereich der freien Enden in Form von Flüssigkeitsstrahlen 337 aus den medizintechnischen Hohlinstrumenten 318.1...318.5 aus und gelangt in den Aufnahmebereich 306.

Das sechste Schlauchstück 310.6 verläuft schräg nach hinten und verfügt an dem der horizontalen Oberseite 327.2 abgewandten Ende über ein sechstes Verbindungsstück 319.31. In der Mantelfläche des zylindrischen sechsten Verbindungsstücks 319.31 ist ein sechster Gegenmagnet 338.6 angeordnet, welcher zur Befestigung des sechsten Verbindungsstücks 319.31 am sechsten Halteblock 334.6 vorgesehen ist. Am dem sechsten Schlauchstück 310.6 abgewandten Ende des sechsten Verbindungsstücks 319.31 ist ein zweiter Kupplungsadapter 319.41 angeordnet, über welchen ein sechstes medizintechnisches Hohlinstrument 318.6 an das sechste Verbindungsstück 319.31 angeschlossen ist.

Das mit dem sechsten Schlauchstück 310.6 verbundene sechste medizintechnische Hohlinstrument 318.6 kann durch die sechste zungenförmige Ausnehmung 329.6 in der Abdeckhaube 328 in gleicher Weise wie die ersten fünf medizintechnischen Hohlinstrumente 318.1...318.5 in den Aufnahmebereich 306 ragend über den sechsten Halteblock 334.6 befestigt werden.

An der rechten Seite des Gehäuses ist in einem vorderen und unteren Bereich ein Aufnahmegefäss 302 für verbrauchte Reinigungsflüssigkeit in das Innere des dritten Reinigungsapparats 300 eingeschoben. Das Aufnahmegefäss 302 verfügt dabei über einen sich in vertikaler Richtung über die gesamte Höhe des Aufnahmegefässes 302 erstreckenden transparenten Griff 331, welcher innwendig hohl ist und mit dem für die verbrauchte Reinigungsflüssigkeit vorgesehenen Innenbereich des Aufnahmegefässes 302 kommuniziert. Damit lässt sich ein Füllstand der verbrauchten Reinigungsflüssigkeit von aussen direkt ablesen.

Rechts oberhalb des Aufnahmegefässes 302 ist zudem eine kreisrunde Lufteinlassöffnung 330 in die rechte Seite des Gehäuses 327 eingebracht.

In einem hinteren Bereich des dritten Reinigungsapparats 300 weitet sich das Gehäuse 327 in einer runden Form auf. Dabei ist ein in vertikaler Richtung verlaufendes Sichtfenster 332 im Gehäuse 327 eingebracht, welches zur Überprüfung der Füllhöhe des dahinter im Innern des Gehäuses 327 liegenden ersten Flüssigkeitsbehälters 301.1 dient. Im ersten Flüssigkeitsbehälter 301.1 liegt z. B. eine desinfizierende Reinigungsflüssigkeit vor. An der Oberseite des hinteren und aufgeweiteten Bereichs des Gehäuses 327 rechts ist ein erster und oval geformter Deckel 333.1 angeordnet, welcher eine in Fig. 6 nicht sichtbare Befüllöffnung des ersten Flüssigkeitsbehälters 301.1 verschliesst.

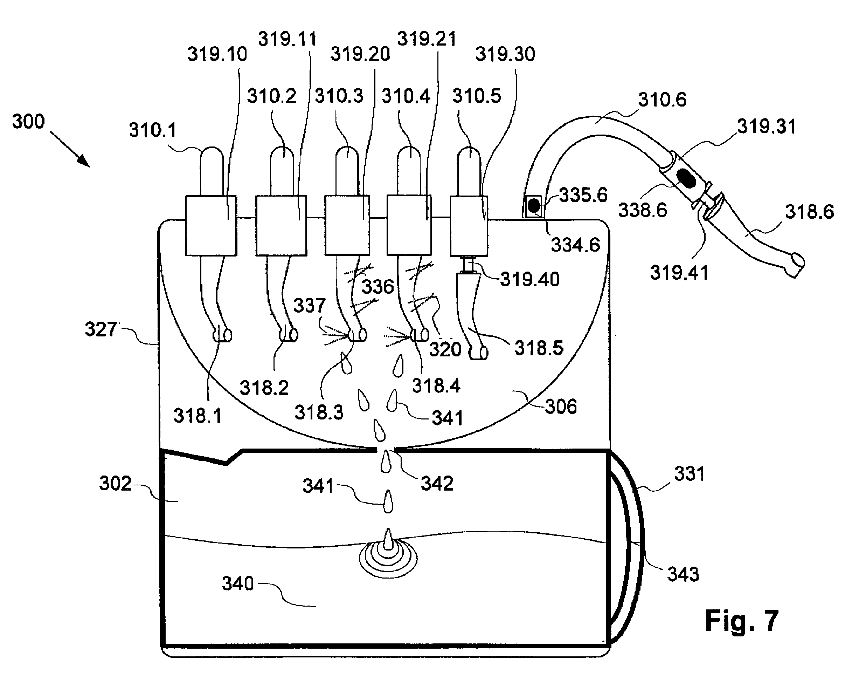

In Fig. 7 ist eine Ansicht von vorne auf die Frontseite 327.1 des dritten Reinigungsapparats 300 gezeigt. Der untere Bereich der Frontseite 327.1 ist dabei aufgeschnitten, so dass das hinter der Flüssigkristallanzeige 312, der Tastatur 313 und den Start-/Stopptasten 313.1, 313.2 liegende Aufnahmegefäss 302 der Reinigungsapparatur 300 sichtbar ist. Die aus den Sprühdüsen 320 und aus den Innenbereichen bzw. den Fluidkanälen der medizintechnischen Hohlinstrumente 318.1...318.5 austretende und verbrauchte Reinigungsflüssigkeit bildet im Aufnahmebereich 306 grössere Tropfen 341, welche aufgrund der Schwerkraft nach unten fliessen und im untersten Punkt des Aufnahmebereichs 6 durch eine an dieser Stelle ausgebildete Abflussöffnung 342 in das darunter liegende Aufnahmegefäss 302 gelangen, wo sie in Form von verbrauchter Reinigungsflüssigkeit 340 gesammelt werden. Der Füllstand 343 der verbrauchten Reinigungsflüssigkeit 340 im sich in vertikaler Richtung über die gesamte Höhe des Aufnahmegefässes 302 erstreckenden transparenten Griff 331 zeigt dabei die Füllhöhe im Aufnahmegefäss 302 an.

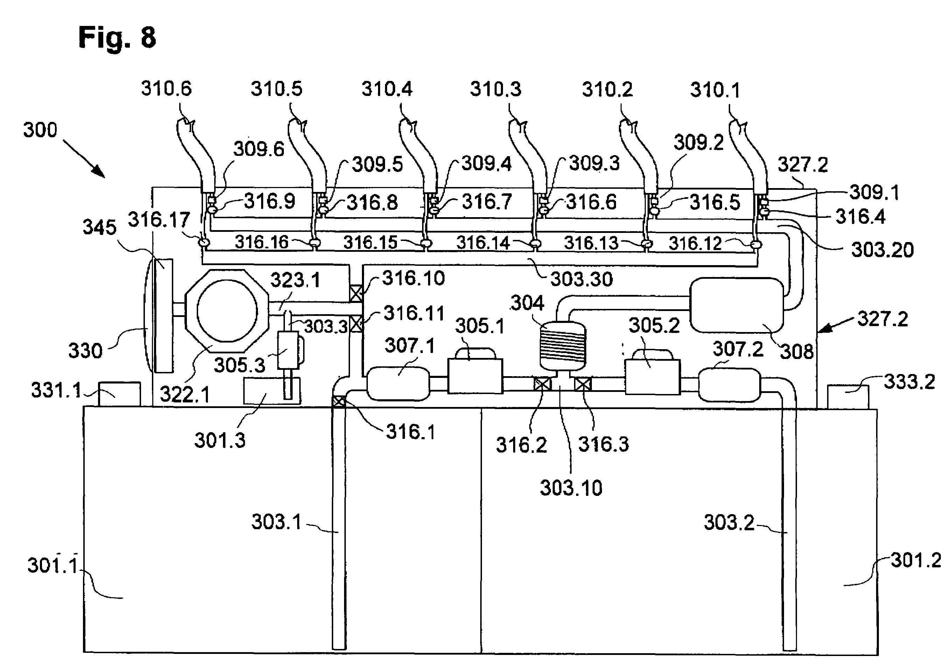

Fig. 8 zeigt die dritte Reinigungsapparatur 300 aus Fig. 6 bei entfernter Rückwand von der Hinterseite. Unten links liegt der erste Flüssigkeitsbehälter 301.1 vor, welcher beispielsweise mit einer ersten schmutzlösenden Reinigungsflüssigkeit befüllt und mit dem ersten Deckel 333.1 verschlossen ist. In den ersten Flüssigkeitsbehälter 301.1 taucht in vertikaler Richtung ein erstes Ansaugrohr 303.1 ein, welches in eine erste Sensoreinheit 307.1 zur Bestimmung der stofflichen Beschaffenheit der ersten schmutzlösenden Reinigungsflüssigkeit mündet. Die erste Sensoreinheit 307.1 zur Bestimmung der stofflichen Beschaffenheit der schmutzlösenden Reinigungsflüssigkeit entspricht im Wesentlichen der in Fig. 2 gezeigten Sensoreinheit 7. Über ein kurzes Rohrstück kommuniziert die erste Sensoreinheit 307.1 zur Bestimmung der stofflichen Beschaffenheit der schmutzlösenden Reinigungsflüssigkeit mit einer ersten Pumpe 305.1, welche die schmutzlösende Reinigungsflüssigkeit aus dem ersten Flüssigkeitsbehälter 301.1 ansaugt. Das erste Ansaugrohr 303.1 verfügt direkt über dem ersten Flüssigkeitsbehälter 301.1 zudem über ein erstes Absperrventil 316.1. Ausgangsseitig kommuniziert die erste Pumpe 305.1 mit einem T-förmigen Rohrstück 303.10, wobei zwischen T-förmigem Rohrstück 303.10 und der ersten Pumpe 305.1 ein zweites Absperrventil 316.2 angeordnet ist.

Rechts neben dem ersten Flüssigkeitsbehälter 301.1 ist ein zweiter Flüssigkeitsbehälter 301.2 angeordnet, welcher beispielsweise mit einer desinfizierenden Reinigungsflüssigkeit befüllt und mit dem zweiten Deckel 333.2 verschlossen ist. In den zweiten Flüssigkeitsbehälter 301.2 taucht in vertikaler Richtung ein zweites Ansaugrohr 303.2 ein, welches in eine zweite Sensoreinheit 307.2 zur Bestimmung der stofflichen Beschaffenheit der zweiten desinfizierenden Reinigungsflüssigkeit mündet. Die zweite Sensoreinheit 307.2 zur Bestimmung der stofflichen Beschaffenheit der desinfizierenden Reinigungsflüssigkeit entspricht ebenfalls im Wesentlichen der in Fig. 2 gezeigten Sensoreinheit 7. Über ein kurzes Rohrstück kommuniziert die zweite Sensoreinheit 307.2 zur Bestimmung der stofflichen Beschaffenheit der desinfizierenden Reinigungsflüssigkeit mit einer zweiten Pumpe 305.2, welche die desinfizierende Reinigungsflüssigkeit aus dem zweiten Flüssigkeitsbehälter 301.2 ansaugt. Ausgangsseitig kommuniziert die zweite Pumpe 305.2 ebenfalls mit dem T-förmigen Rohrstück 303.10, wobei zwischen T-förmigem Rohrstück 303.10 und der zweiten Pumpe 305.2 ein drittes Absperrventil 316.3 angeordnet ist.

Der Auslass des T-förmigen Rohrstücks 303.10 mündet schliesslich in eine Heizeinheit 304, welche wiederum mit einer Sensoreinheit 308 zur Bestimmung des physikalischen Zustands der Reinigungsflüssigkeiten verbunden ist. Die Sensoreinheit 308 zur Bestimmung des physikalischen Zustands der Reinigungsflüssigkeiten entspricht im Wesentlichen der in Fig. 3 gezeigten Sensoreinheit 8. An die Sensoreinheit 308 zur Bestimmung des physikalischen Zustands der Reinigungsflüssigkeiten schliesst ausgangsseitig ein erster Speisekanal 303.20 an. Der erste Speisekanal 303.20 verläuft im Wesentlichen in horizontaler Richtung direkt unterhalb der horizontalen Oberseite 327.2 des vorderen und im Wesentlichen quaderförmigen Bereichs des Gehäuses 327. Von diesem ersten Speisekanal 303.20 zweigt in Fig. 8 auf der rechten Seite ein viertes Absperrventil 316.4 in vertikaler Richtung aus dem Speisekanal 303.20 nach oben ab, wobei das vierte Absperrventil 316.4 direkt in ein erstes Durchflussmessgerät 309.1 mündet. Ausgangsseitig ist das erste Durchflussgerät 309.1 mit dem darüber angeordneten ersten Schlauchstück 310.1 1 verbunden.

Links neben dem vierten Absperrventil 316.4 zweigt in gleicher Weise ein fünftes Absperrventil 316.5 ab, welches direkt in ein zweites Durchflussmessgerät 309.2 mündet. Ausgangsseitig ist das zweite Durchflussgerät 309.2 mit dem darüber angeordneten zweiten Schlauchstück 310.2 verbunden. Links neben dem fünften Absperrventil 316.5 zweigt in gleicher Weise ein sechstes Absperrventil 316.6 ab, welches direkt in ein drittes Durchflussmessgerät 309.3 mündet. Ausgangsseitig ist das dritte Durchflussgerät 309.3 mit dem darüber angeordneten dritten Schlauchstück 310.3 verbunden. Links neben dem sechsten Absperrventil 316.6 zweigt in gleicher Weise ein siebtes Absperrventil 316.7 ab, welches direkt in ein viertes Durchflussmessgerät 309.4 mündet. Ausgangsseitig ist das vierte Durchflussgerät 309.4 mit dem darüber angeordneten vierten Schlauchstück 310.4 verbunden. Links neben dem siebten Absperrventil 316.7 zweigt In gleicher Weise ein achtes Absperrventil 316.8 ab, welches direkt in ein fünftes Durchflussmessgerät 309.5 mündet. Ausgangsseitig ist das fünfte Durchflussgerät 309.5 mit dem darüber angeordneten fünften Schlauchstück 310.5 verbunden. Links neben dem achten Absperrventil 316.8 zweigt in gleicher Weise ein neuntes Absperrventil 316.9 ab, welches direkt in ein sechstes Durchflussmessgerät 309.6 mündet. Ausgangsseitig ist das sechste Durchflussgerät 309.6 mit dem darüber angeordneten sechsten Schlauchstück 310.6 verbunden.

Während einem Reinigungs- und/oder Desinfektionsvorgang ist entweder die erste Pumpe 305.1 oder die zweite Pumpe 305.2, ausser bei einer Störung, ständig in Betrieb und fördert kontinuierlich Reinigungsflüssigkeit aus dem ersten Flüssigkeitsbehälter 301.1 oder aus dem zweiten Flüssigkeitsbehälter 301.2 durch die Aufnahmevorrichtung 306 und die medizinischen Hohlinstrumente 318.1...318.6 in das Aufnahmegefäss 302.

Links oberhalb des ersten Flüssigkeitsbehälters 301.1 ist des Weiteren die Lufteinlassöffnung 330 angeordnet. Auf die Lufteinlassöffnung 330 folgt im Innern des Gehäuses 327 ein Luftfilter 345, welcher mit einer Luftpumpe 322.1 kommuniziert. Ausgangsseitig mündet die Luftpumpe 322.1 in einen Fluidkanal 323.1, welcher sich nach oben und nach unten verzweigt. Der nach oben führende Zweig des Fluidkanals 323.1 ist über ein zehntes Absperrventil 316.10 verschliessbar. Entsprechend ist der nach unten führende Zweig des Fluidkanals 323.1 über ein elftes Absperrventil 316.11 verschliessbar. Das zehnte Absperrventil 316.10 mündet schliesslich in einen zweiten Speisekanal 303.30, welcher direkt unterhalb des ersten Speisekanals 303.20 und parallel zu diesem verläuft. In Fig. 8 auf der rechten Seite zweigt ein zwölftes Absperrventil 316.12 in horizontaler Richtung aus dem zweiten Speisekanal 303.30 nach oben ab. Ausgangsseitig mündet das zwölfte Absperrventil 313.12 im ersten Schlauchstück 310.1.

Links des zwölften Absperrventils 316.12 zweigt ein dreizehntes Absperrventil 316.13 in gleicher Weise aus dem zweiten Speisekanal 303.30 ab und mündet im zweiten Schlauchstück 310.2. Links des dreizehnten Absperrventils 316.13 zweigt ein vierzehntes Absperrventil 316.14 in gleicher Weise aus dem zweiten Speisekanal 303.30 ab und mündet im dritten Schlauchstück 310.3. Links des vierzehnten Absperrventils 316.14 zweigt ein fünfzehntes Absperrventil 316.15 in gleicher Weise aus dem zweiten Speisekanal 303.30 ab und mündet im vierten Schlauchstück 310.4. Links des fünfzehnten Absperrventils 316.15 zweigt ein sechzehntes Absperrventil 316.16 in gleicher Weise aus dem zweiten Speisekanal 303.30 ab und mündet im fünften Schlauchstück 310.5. Links des sechzehnten Absperrventils 316.16 zweigt ein siebzehntes Absperrventil 316.17 in gleicher Weise aus dem zweiten Speisekanal 303.30 ab und mündet im sechsten Schlauchstück 310.6.

Zwischen der Luftpumpe 322.1 und dem zehnten Absperrventil 316.10 bzw. dem elften Absperrventil 316.11 mündet zudem ein Einlassstutzen 303.3 einer Ölpumpe 305.3 in das T-förmige Rohrstück 323.1. Eingangsseitig kommuniziert die Ölpumpe 305.3 zudem mit einem Ölreservoir 301.3, welches mit Schmieröl befüllt ist.

Das am unteren Zweig des T-förmigen Rohrstücks 323.1 angeordnete elfte Absperrventil 316.11 mündet zudem in das Rohrstück zwischen dem ersten Absperrventil 316.1 und der ersten Sensoreinheit 307.1 zur Bestimmung der stofflichen Beschaffenheit der ersten Reinigungsflüssigkeit.

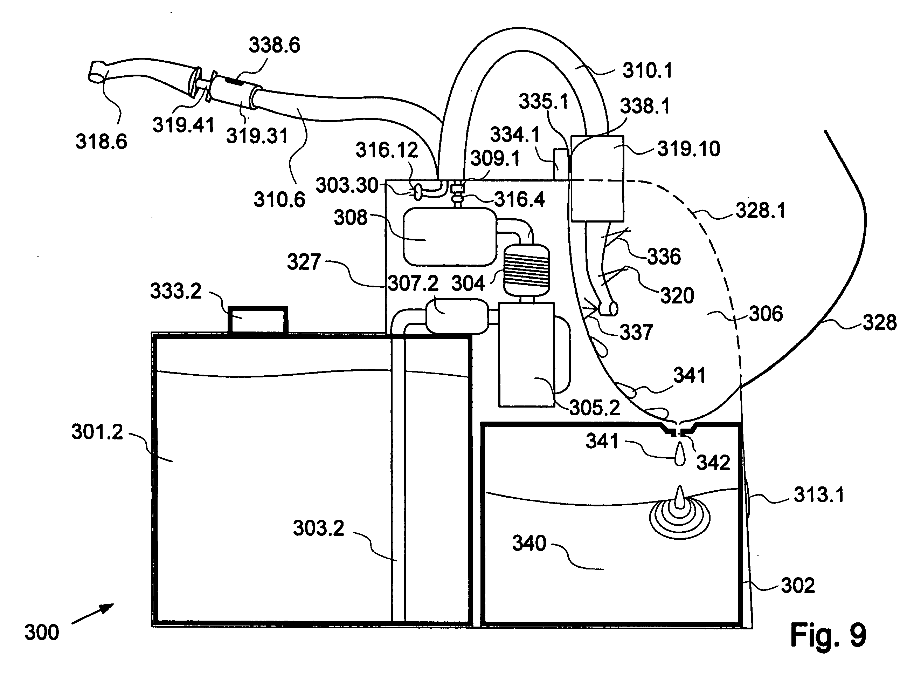

Fig. 9 zeigt die dritte Reinigungsapparatur 300 aus Fig. 6 entsprechend von der linken Seite, mit abmontierter Seitenwand. Rechts unten liegt das Auffanggefäss 302 vor. Links daneben ist der zweite Flüssigkeitsbehälter 301.2 der dritten Reinigungsapparatur 300 angeordnet, welcher dem zweiten Deckel 333.2 verschlossen ist. Von der horizontalen Oberseite 327.1 des Gehäuses 327 ragt das erste Schlauchstück 310.1 halbkreisförmig zum ersten Verbindungsstück 319.10, welches über den ersten Permanentmagneten 335.1 und den ersten Gegenmagneten 338.1 am ersten Halteblock 334.1 befestigt ist.

In Fig. 9 ist die Abdeckhaube 328 in der aufgeklappten Position gezeigt. Die entsprechend geschlossene Position ist durch die gestrichelte Linie 328.1 angedeutet.

Die dritte Reinigungsapparatur 300 verfügt zudem über eine Steuereinheit mit einem Mikroprozessor, einer Speichereinheit und einer Zeitmesseinrichtung. Sämtliche Absperrventile 316.1...316.17, Sensoreinheiten 307.1, 307.2, 308, Durchflussmessgeräte 309.1...309.6, Pumpen 305.1, 305.2, 305.3, 322.1, die Flüssigkristallanzeige 312, die Tastatur 313 und die Start-/Stopptasten 313.1, 313.2 sind über entsprechende Signalleitungen mit der Steuereinheit verbunden. Damit ist eine vollautomatische Steuerung der dritten Reinigungsapparatur 300 möglich. Der Übersichtlichkeit halber wurde in den Fig. 6 - 9 aber auf eine Darstellung der entsprechenden Komponenten verzichtet.

Die Ansteuerung der dritten Reinigungsapparatur 300 erfolgt in analoger Weise, wie bei der ersten Reinigungsapparatur 100. Bei der dritten Reinigungsapparatur 300 können jedoch aufgrund der zwei Flüssigkeitsbehälter 301.1, 301.2 zusätzlich mehrstufige Reinigungs- und/oder Desinfektionsvorgänge mit unterschiedlichen Reinigungsflüssigkeiten durchgeführt werden. Der Einsatz der unterschiedlichen Reinigungsflüssigkeiten kann z. B. durch die Steuereinheit kontrolliert werden und vollautomatisch erfolgen.

In den Fig. 10.1 und 10.2 ist das erste Schlauchstück 310.1 aus Fig. 6-9 im Detail gezeigt. Das erste Schlauchstück 310.1 besteht aus einer äusseren und zylindrischen Ummantelung 346 aus einem Kunststoff. Am rechten Ende ist die äussere Ummantelung 346 stufenartig verjüngt, wobei der verjüngte Bereich eine Andockstelle 354 für die Verbindungsstücke bildet. In einem hinteren Bereich der Mantelfläche der Andockstelle 354 ist zudem eine senkrecht zur Längsrichtung des ersten Schlauchstücks 310.1 laufende und rechteckige Rastkerbe 347 eingebracht. Die Rastkerbe 347 dient zur Aufnahme eines Rastelements eines an der Andockstelle 354 zu befestigenden Verbindungsstücks.

Im Innern der Ummantelung 346 ist ein erster zylindrischer Fluidkanal 303.11 eingebracht, welcher sich vom linken stirnseitigen Ende des Schlauchstücks 310.1 bis zum rechten stirnseitigen Ende bzw. bis zur Stirnseite der Andockstelle 354 erstreckt. Der erste Fluidkanal 303.11 des ersten Schlauchstücks 310.1 ist zum Anschluss an das erste Durchflussmessgerät 309.1 (siehe Fig. 8) vorgesehen und ragt hierfür auf der linken Stirnseite bzw. an der der Andockstelle 354 gegenüberliegenden Stirnseite des ersten Schlauchstücks 310.1 aus der Ummantelung 346. Im angeschlossenen Zustand kommuniziert der erste Fluidkanal 303.11 des ersten Schlauchstücks 310.1 demnach mit dem ersten Durchflussmessgerät 309.1 und das vierte Absperrventil 316.4 mit dem zweiten Speisekanal 303.30 und dient zur Zuleitung der Reinigungsflüssigkeiten in den stirnseitigen Bereich der Andockstelle 354.

Ebenso ist im Innern der Ummantelung 346 ein zweiter zylindrischer Fluidkanal 303.12 eingebracht, welcher sich vom linken stirnseitigen Ende des Schlauchstücks 310.1 bis zum rechten stirnseitigen Ende bzw. bis zur Stirnseite der Andockstelle 354 erstreckt. Der zweite Fluidkanal 303.12 des ersten Schlauchstücks 310.1 ist zum Anschluss an den Auslass des zwölften Absperrventils 316.12 ausgebildet und ragt hierfür auf der linken Stirnseite bzw. an der der Andockstelle 354 gegenüberliegenden Stirnseite des ersten Schlauchstücks 310.1 aus der Ummantelung 346. Im angeschlossenen Zustand kommuniziert der zweite Fluidkanal 303.12 des ersten Schlauchstücks 310.1 demnach mit dem zweiten Speisekanal 303.30 und dient zur Zuleitung von Luft und/oder einem öligen Aerosol in den stirnseitigen Bereich der Andockstelle 354.

Des Weiteren verläuft im Innern der Ummantelung 346 ein zweipoliges elektrisches Kabel 315.1, welches mit einem aussen auf der vorderen Mantelfläche der Andockstelle 354 abgebrachten Kontaktpaar 348 elektrisch verbunden ist. An der der Andockstelle 354 gegenüberliegenden Stirnseite des ersten Schlauchstücks 310.1 ragt das zweipolige elektrische Kabel 315.1 auf der linken Stirnseite aus der Ummantelung 346 heraus und kann an einem nicht dargestellten Stecker im Innern des Gehäuses 327 kontaktiert und mit der Steuereinheit verbunden werden.

Die übrigen fünf in den Fig. 6 - 9 gezeigte Schlauchstücke 310.2...310.6 sind gleich ausgebildet wie das erste Schlauchstück 310.1.

In Fig. 10.3 ist das erste Schlauchstück 310.1 aus den Fig. 10.1 und 10.2 mit dem an der Andockstelle 354 befestigten ersten Verbindungsstück 319.10 gezeigt. Das erste Verbindungsstück 319.10, welches baugleich mit dem zweiten Verbindungsstück 319.11 ist, ist speziell für die Reinigung von medizintechnischen Hohlinstrumenten mit drehbaren mechanischen Teilen ausgelegt.

Das erste Verbindungsstück 319.10 ist im linken Bereich, bzw. im Bereich, welcher dem ersten Schlauchstück 310.1 zugewandt ist, zylindrisch ausgeformt und geht stufenartig in einen rechten und verjüngten zylindrischen Kopplungsbereich 354.1 für ein erstes medizintechnisches Hohlinstrument 318.1 über.

Das erste Verbindungsstück 319.10 weist im Bereich der linken Stirnseite eine zylindrische Öffnung auf, welche form- und kraftschlüssig über die Andockstelle 354 des ersten Schlauchstücks geschoben ist. Das in Fig. 10.3 nicht sichtbare Rastelement an der Innenseite der zylindrischen Öffnung greift dabei in die Rastkerbe 347 an der Andockstelle 354 des ersten Schlauchstücks 310.1 ein und sichert dadurch die Verbindung zusätzlich. Im Bereich der Mantelfläche des ersten Verbindungsstücks 319.10 ist des Weiteren der erste Gegenmagnet 338.1 angeordnet.

Des Weiteren verfügt das erste Verbindungsstück 319.10 über eine erste Fluidleitung 303.13, welche mit dem ersten Fluidkanal 303.11 des ersten Schlauchstücks 310.1 kommuniziert und im Bereich der rechten Stirnseite aus dem Kopplungsbereich 354.1 des ersten Verbindungsstücks 319.10 austritt. Ebenso liegt eine zweite Fluidleitung 303.14 vor, welche mit dem zweiten Fluidkanal 303.12 des ersten Schlauchstücks 310.1 kommuniziert und ebenfalls im Bereich der rechten Stirnseite aus dem Kopplungsbereich 354.1 des ersten Verbindungsstücks 319.10 austritt.

In einem zentralen inneren Bereich des ersten Verbindungsstücks 319.10 liegt zudem ein Elektromotor 349 vor. Linksseitig, bzw. in Richtung der Andockstelle 354, ragt ein elektrisches Anschlusspaar 348.1 aus dem Elektromotor 349, wobei das Anschlusspaar 348.1 mit dem Kontaktpaar 348 der Andockstelle 354 des ersten Schlauchstücks 310.1 elektrisch verbunden ist. Auf der gegenüberliegenden rechten Seite des Elektromotors 349 ragt eine Antriebsachse 350.1 koaxial zur Längsachse des ersten Verbindungsstücks 319.10 aus dem Elektromotor 349 in einen verjüngten rechten Bereich des ersten Verbindungsstücks 319.10. Am stirnseitigen Ende der Antriebsachse 350.1 ist eine Stirnzahnkupplung 350.2 angeordnet.

Die beiden Fluidleitungen 303.13, 303.14 des ersten Verbindungsstücks 319.10 sind so angeordnet, dass sie mit den entsprechenden Kanälen oder Hohlräumen im Innern des über den Kopplungsbereich 354.1 geschobenen ersten medizintechnischen Hohlinstruments 318.1 kommunizieren. Über die Stirnzahnkupplung 350.2 können drehbare Rotoren des ersten medizintechnischen Hohlinstruments 318.1, welche beim Einsatz normalerweise mit Hochdruck angetrieben werden, während dem Reinigungs-und/oder Desinfektionsvorgang rotiert werden, so dass auch die drehbaren Rotoren vollständig gereinigt bzw. desinfiziert werden können. Falls erwünscht, kann aufgrund der elektrischen Leistungsaufnahme des Elektromotors 349 die korrekte Befestigung des ersten medizintechnischen Hohlinstruments 318.1 am ersten Verbindungsstück 319.10 geprüft werden.

In Fig. 10.4 ist das erste Schlauchstück 310.1 mit dem an der Andockstelle 354 befestigten dritten Verbindungsstück 319.20, welches baugleich mit dem vierten Verbindungsstück 319.21 ist, im Detail gezeigt. Das dritte Verbindungsstück ist im linken Bereich, bzw. im Bereich welcher dem ersten Schlauchstück 310.1 zugewandt ist, zylindrisch ausgeformt und geht stufenartig in einen rechten und verjüngten zylindrischen Kopplungsbereich 354.2 für das dritte medizintechnische Hohlinstrument 318.3 über.

Das dritte Verbindungsstück 319.20 weist im Bereich der linken Stirnseite eine zylindrische Öffnung auf, welche form- und kraftschlüssig über die Andockstelle 354 des ersten Schlauchstücks geschoben ist. Das in Fig. 10.4 nicht sichtbare Rastelement an der Innenseite der zylindrischen Öffnung greift dabei in die Rastkerbe 347 an der Andockstelle 354 des ersten Schlauchstücks 310.1 ein und sichert dadurch die Verbindung zusätzlich.

Des Weiteren verfügt das dritte Verbindungsstück 319.20 über eine erste Fluidleitung 303.15, welche mit dem ersten Fluidkanal 303.11 des ersten Schlauchstücks 310.1 kommuniziert und im Bereich der rechten Stirnseite aus dem Kopplungsbereich 354.2 des dritten Verbindungsstücks 319.20 austritt. Ebenso liegt ein zweite Fluidleitung 303.16 vor, welche mit dem zweiten Fluidkanal 303.12 des ersten Schlauchstücks 310.1 kommuniziert und ebenfalls im Bereich der rechten Stirnseite aus dem Kopplungsbereich 354.2 des dritten Verbindungsstücks 319.20 austritt.

Die beiden Fluidleitungen 303.15, 303.16 des dritten Verbindungsstücks 319.20 sind so angeordnet, dass sie mit den entsprechenden Kanälen oder Hohlräumen im Innern des über den Kopplungsbereich 354.2 geschobenen dritten medizintechnischen Hohlinstruments 318.3 kommunizieren.

In einem zentralen Bereich des dritten Verbindungsstücks 319.20 verläuft in Längsrichtung ein zweipoliges elektrisches Anschlusskabel 352, welches links mit dem Kontaktpaar 348 des ersten Schlauchstücks 310.1 elektrisch verbunden ist. Im Innern der Kontaktstelle 354.2 des dritten Verbindungsstücks 319.20 ist das zweipolige elektrische Anschlusskabel 352 mit einem Befestigungssensor 351 verbunden. Über den Befestigungssensor kann festgestellt werden, ob ein drittes medizintechnisches Hohlinstrument 318.3 in der richtigen Art und Weise an der Kontaktstelle 354.2. Der Befestigungssensor 351 ist z. B. als Reed-Kontakt ausgebildet, welcher durch ein im dritten medizintechnischen Hohlinstrument 318.3 angebrachten Magneten geschalten wird.

In Fig. 10.5 ist das erste Schlauchstück 310.1 mit dem an der Andockstelle 354 befestigten fünften Verbindungsstück 319.30, welches baugleich mit dem sechsten Verbindungsstück 319.31 ist, im Detail abgebildet. Das fünfte Verbindungsstück 319.30 dient als universales Verbindungsstück, welches zur Befestigung von speziellen und auf unterschiedliche medizintechnische Hohlinstrumente angepasste Kopplungsadapter ausgelegt ist.

Das fünfte Verbindungsstück ist im linken Bereich, bzw. im Bereich welcher dem ersten Schlauchstück 310.1 zugewandt ist, zylindrisch ausgeformt und geht stufenartig in einen rechten und verjüngten zylindrischen Kopplungsbereich 354.3 für den ersten Kupplungsadapter 319.40 auf, welcher zum Anschluss des fünften medizintechnischen Hohlinstruments 318.5 dient.

Das fünfte Verbindungsstück 319.30 weist im Bereich der linken Stirnseite eine zylindrische Öffnung auf, welche form- und kraftschlüssig über die Andockstelle 354 des ersten Schlauchstücks geschoben ist. Das in Fig. 10.5 nicht sichtbare Rastelement an der Innenseite der zylindrischen Öffnung greift dabei in die Rastkerbe 347 an der Andockstelle 354 des ersten Schlauchstücks 310.1 ein und sichert dadurch die Verbindung zusätzlich.

Des Weiteren verfügt das fünfte Verbindungsstück 319.30 über eine erste Fluidleitung 303.17, welche mit dem ersten Fluidkanal 303.11 des ersten Schlauchstücks 310.1 kommuniziert und in einen Hohlraum 353 im Innern des fünften Verbindungsstücks 319.30 mündet. Ebenso liegt eine zweite Fluidleitung 303.18 vor, welche mit dem zweiten Fluidkanal 303.12 des ersten Schlauchstücks 310.1 kommuniziert und ebenfalls in den Hohlraum 353 des fünften Verbindungsstück 319.30 mündet. Der Hohlraum 353 verjüngt sich auf der rechten Seite konisch und weist auf der rechten Seite, in der Stirnseite des Kopplungsbereichs 354.3, eine Öffnung auf.

Über den zylindrischen Kopplungsbereich 354.3 des fünften Verbindungsstücks 319.30 ist der erste Kopplungsadapter 319.40 geschoben, welcher als zylindrisches Röhrchen ausgebildet ist. Die rechte Seite des Kopplungsadapters 319.40 ist zur Verbindung mit dem fünften medizintechnischen Hohlinstrument ausgelegt.

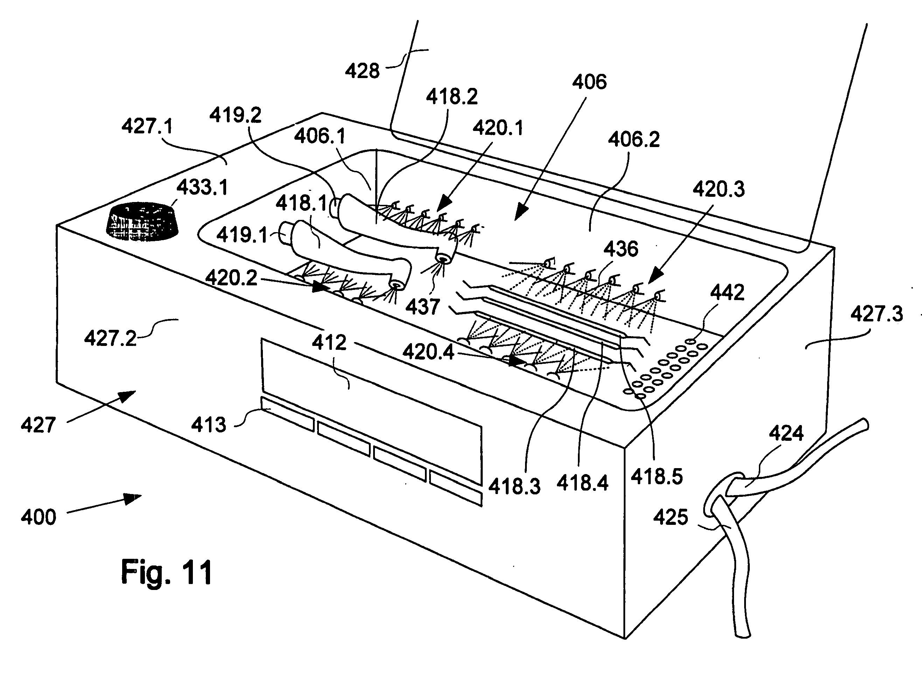

In Fig. 11 ist eine vierte Reinigungsapparatur 400 in einer perspektivischen Ansicht von schräg vorne und oben gezeigt, wobei die vierte Reinigungsapparatur 400 wie die zweite Reinigungsapparatur 200 ein Kreislaufsystem für die Reinigungsflüssigkeit beinhaltet. Die vierte Reinigungsapparatur 400 ist in einem im Wesentlichen quaderförmigen Gehäuse 427 untergebracht und weist einen von oben eingelassenen quaderförmigen Aufnahmebereich 406 für medizintechnische Geräte 418.1, 418.2...418.5 auf. Der Aufnahmebereich 406 ist von oben zugänglich und mit einem transparenten Deckel 428, welcher über ein Scharnier an der Gehäuseoberseite 427.1 befestigt ist, verschliessbar.

Von links ragt aus der linksseitigen Seitenfläche 406.1 des Aufnahmebereichs 406 in horizontaler Richtung ein erstes zylindrisches Verbindungsstück 419.1 auf etwa halber Höhe in den Aufnahmebereich 406 hinein. Am ersten Verbindungsstück 419.1 ist ein erstes medizintechnisches Hohlinstrument 418.1 im linken Bereich des Aufnahmebereichs 406 angeordnet. Auf gleicher Höhe ragt neben dem ersten Verbindungsstück 419.1 in gleicher Weise ein zweites und baugleiches Verbindungsstück 419.2 in den Aufnahmebereich 406 hinein. Entsprechend ist am zweiten Verbindungsstück 419.2 ein zweites medizintechnisches Hohlinstrument 418.2 ebenfalls im linken Bereich des Aufnahmebereichs 406 angeordnet. Die durch die beiden medizintechnischen Holinstrumente 418.1, 418.2 geleitete Reinigungsflüssigkeit tritt in Form von Flüssigkeitsstrahlen 437 in den Aufnahmebereich 406 aus.

An der rechtwinklig zur linksseitigen Begrenzungswand 406.1 verlaufenden hinteren Seitenfläche 406.2 ist linksseitig ein erster Spritzdüsensatz 420.1 auf einer horizontalen Linie angeordnet. Entsprechend ist an der rechtwinklig zur linksseitigen Begrenzungswand 406.1 verlaufenden vorderen Seitenfläche 406.3 linksseitig ein zweiter Spritzdüsensatz 420.2 auf einer horizontalen Linie angeordnet. Die beiden Spritzdüsensätze 420.1, 420.2 erstrecken sich in horizontaler Richtung über die gesamten Längen der beiden medizintechnischen Hohlinstrumente 418.1, 418.2, so dass diese durch die ersten beiden Spritzdüsensätze 420.1, 420.2 seitlich mit Sprühstrahlen 436 aus Reinigungsflüssigkeit besprüht und benetzt werden.

In einem rechten Bereich des Aufnahmebereichs 406 liegen drei Zahnarzthaken 418.3, 418.4, 418.5 auf dem Boden des Aufnahmebereichs 406 auf. An der hinteren Seitenfläche 406.2 ist rechtsseitig ein dritter Spritzdüsensatz 420.3 auf einer horizontalen Linie angeordnet. Ebenso ist an der vorderen Seitenfläche 406.3 rechtsseitig ein vierter Spritzdüsensatz 420.4 auf einer horizontalen Linie angeordnet. Der dritte Spritzdüsensatz 420.3 und der vierte Spritzdüsensatz 420.4 erstrecken sich über die gesamte Länge der drei Zahnarzthaken 418.3, 418.4, 418.5, so dass sie durch den dritten Spritzdüsensatze 420.3 und den vierten Spritzdüsensatze 420.4 seitlich mit Sprühstrahlen 436 aus Reinigungsflüssigkeit besprüht und benetzt werden.

Rechts neben den drei Zahnarzthaken 418.3, 418.4, 418.5 sind mehrere Abflusslöcher 442 für die Reinigungsflüssigkeit im Bodenbereich des Aufnahmebereichs 6 angeordnet.

An der Gehäusevorderseite 427.2 des Gehäuses 427 ist in einem zentralen Bereich eine Eingabevorrichtung 413 in Form von vier Tasten angebracht. Darüber befindet sich als Ausgabevorrichtung 412 eine Flüssigkristallanzeige.

An der rechten Gehäuseseite 427.3 führen eine Einlassleitung 424 und eine Auslassleitung 425 in das Innere des Gehäuses 427.

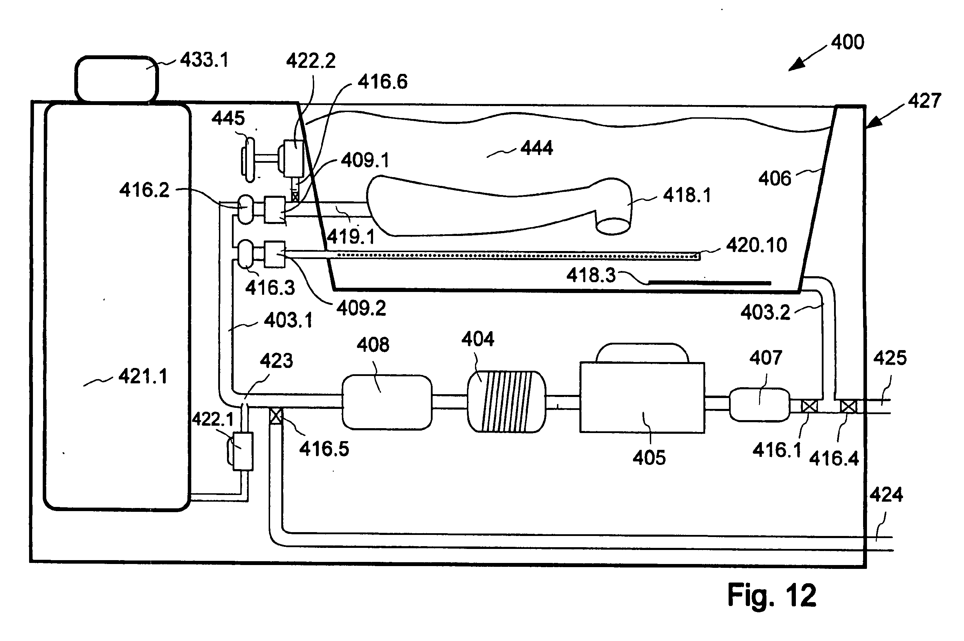

In Fig. 12 ist die vierte Reinigungsapparatur 400 von vorne bei abmontierter Gehäusevorderseite 427.2 abgebildet. Auf der rechten Seite liegt ein erstes Absperrventil 416.1 vor, welches mit einer Sensoreinheit 407 zur Bestimmung der stofflichen Beschaffenheit der Reinigungsflüssigkeit kommuniziert. Die Sensoreinheit 407 zur Bestimmung der stofflichen Beschaffenheit der Reinigungsflüssigkeit ist im Wesentlichen baugleich mit der bei Fig. 2 gezeigten Sensoreinheit 7. Ausgangsseitig ist die Sensoreinheit 407 zur Bestimmung der stofflichen Beschaffenheit der Reinigungsflüssigkeit mit einer Pumpe 405 verbunden, welche wiederum mit einer Heizeinheit 404 kommuniziert. Die Heizeinheit wiederum ist mit ihrem Ausgang an eine Sensoreinheit 408 zur Bestimmung des physikalischen Zustands der Reinigungsflüssigkeit angeschlossen. Die Sensoreinheit 408 zur Bestimmung des physikalischen Zustands der Reinigungsflüssigkeit ist im Wesentlichen baugleich mit der in Fig. 3 gezeigten Sensoreinheit 8.

Von der Sensoreinheit 408 zur Bestimmung des physikalischen Zustands der Reinigungsflüssigkeit führt ein senkrecht nach oben abgebogenes Zufuhrrohr 403.1 in den oberen Bereich des Gehäuses 427. Am oberen Ende mündet das Zufuhrrohr 403.1 in ein nach rechts abzweigendes zweites Absperrventil 416.2. Ausgangsseitig kommuniziert das zweite Absperrventil 416.2 mit einem ersten Durchflussmessgerät 409.1, welches wiederum in das erste Verbindungsstück 419.1 mit dem daran befestigten ersten medizintechnischen Hohlinstrument 418.1 mündet. Das erste Verbindungsstück 419.1 und das daran befestigte erste medizintechnische Hohlinstrument 418.1 ragen dabei in den Aufnahmebereich 406, welcher von der Seite betrachtet eine im Wesentlichen rechteckige Form aufweist. Der Aufnahmebereich 406 ist rechts neben dem ersten Durchflussmessgerät 409.1 und oberhalb der Pumpe 405, der Heizeinheit 404 und der beiden Sensoreinheiten 407, 408 im Gehäuse 427 eingelassen. Hinter dem ersten medizintechnischen Hohlinstrument 418.1 ist wie bei Fig. 11 beschrieben, das zweite medizintechnische Holinstrument 418.2 angeordnet, welches in gleicher Weise mit Reinigungsflüssigkeit versorgt wird, wie das erste medizintechnische Hohlinstrument 418.1. Entsprechend ist auch das zweite Verbindungsstück 419.2 über ein Absperrventil und ein Durchflussmessgerät mit der Zufuhrleitung 403.1 verbunden.

Unterhalb des zweiten Absperrventils 416.2 zweigt ein drittes Absperrventil 416.3 nach rechts aus dem Zufuhrrohr 403.1 ab. Das dritte Absperrventil 416.3 ist ausgangsseitig mit einem zweiten Durchflussmessgerät 409.2 verbunden, welches wiederum in eine Versorgungsleitung 420.10 für die in Fig. 12 nicht sichtbaren zweiten Spritzdüsensatz 420.2 und vierten Spritzdüsensatz 420.4 mündet. Die Versorgungsleitung 420.10 verläuft dabei in horizontaler Richtung und unterhalb des ersten medizintechnischen Hohlinstruments 419.1 ausserhalb des Aufnahmebereichs 406. Eine zweite und in Fig. 11 nicht sichtbare Versorgungsleitung verläuft in gleicher Weise im Bereich der hinteren Seitenwand ausserhalb des Aufnahmebereichs 406 und versorgt den ersten Sprühdüsensatz 420.1 und den dritten Sprühdüsensatz 420.3 mit Reinigungsflüssigkeit. Entsprechend ist auch die zweite Versorgungsleitung über ein Absperrventil und ein Durchflussmessgerät mit der Zufuhrleitung 403.1 verbunden.

An der rechten Seite des Aufnahmebereichs 406 ist zudem eine Abflussleitung 403.2 angeordnet, welche mit ihrem oberen Ende mit den in Fig. 11 nicht sichtbaren Abflusslöchern 422 kommuniziert. An ihrem unteren Ende ist die Abflussleitung 403.2 mit der Eingangsseite des ersten Absperrventils 416.1 verbunden. Somit liegt ein Kreislaufsystem für die Reinigungsflüssigkeit vor. Reinigungsflüssigkeit, welche aus dem Aufnahmebereich 406 abfliesst, kann über das erste Absperrventil 416.1 und die daran angeschlossenen Vorrichtungen die Verbindungsstücke 419.1, 419.2 und die Versorgungsleitungen 420.10 den im Aufnahmebereich 406 vorliegenden medizintechnischen Geräten 418.1...418.5 zugeführt werden. Insbesondere aufgrund der Sensoreinheit 407 zur Bestimmung der stofflichen Beschaffenheit der Reinigungsflüssigkeit kann dabei fortlaufend überprüft werden, ob die Reinigungsflüssigkeit den Anforderungen noch genügt.

Des Weiteren zweigt aus der Abflussleitung 403.2 im Bereich vor dem ersten Absperrventil 416.1 ein viertes Absperrventil 416.4 ab, welches mit der aus dem Gehäuse 427 hinaus führenden Auslassleitung 425 kommuniziert. Unmittelbar nach der Sensoreinheit 408 zur Bestimmung der physikalischen Beschaffenheit der Reinigungsflüssigkeit zweigt ein fünftes Absperrventil 416.5 aus dem Zufuhrrohr 403.1 ab, welches mit der aus dem Gehäuse 427 führenden Einlassleitung 424 verbunden ist.

Sollte die Reinigungsflüssigkeit beispielsweise mit Fremdstoffen verunreinigt sein, so kann das vierte Absperrventil 416.4 geöffnet werden, so dass die Reinigungsflüssigkeit über die Auslassleitung 425 aus der vierten Reinigungsapparatur 400 entnommen werden kann. Anschliessend wird das vierte Absperrventil 416.4 wieder geschlossen und das fünfte Absperrventil 416.5 wird geöffnet. Sodann kann die vierte Reinigungsapparatur 400 über die Einlassleitung 424 mit frischer und unverbrauchter Reinigungsflüssigkeit befüllt werden.

Auf der in Fig. 12 linken Seite des Gehäuses 427 ist ein Flüssigkeitsbehälter 421.1 angeordnet, welcher im Bereich der Gehäuseoberseite 427.1 eine mit einem Deckel 433.1 verschlossene Befüllöffnung aufweist. Der Flüssigkeitsbehälter kommuniziert über ein im Bereich des Bodens des Flüssigkeitsbehälters 421.1 heraus führenden Rohrstück mit einer zweiten Pumpe 422.1. Ausgangsseitig mündet die zweite Pumpe 422.1 über einen Einlassstutzen 423 zwischen dem fünften Absperrventil 416.5 und dem dritten Absperrventil 416.3 in das Zufuhrrohr 403.1. Aus dem Flüssigkeitsbehälter 421.1 kann mit der zweiten Pumpe 422.1 beispielsweise ein Reinigungsmittelkonzentrat angesaugt und durch den Einlassstutzen 423 in das Zufuhrrohr 403.1 eingeleitet werden. Damit kann die stoffliche Beschaffenheit des Reinigungsmittels verändert werden.

Oberhalb des zweiten Absperrventils 416.2 und des ersten Durchflussmessgeräts 406.1 ist des Weiteren eine Luftpumpe 422.2 mit einem einlassseitigen Luftfilter 445 angeordnet. Ausgangsseitig mündet die Luftpumpe 422.2 unmittelbar nach dem ersten Durchflussmessgerät 409.1 über ein sechstes Absperrventil 416.6 in das erste Verbindungsstück 419.1. Über eine in Fig. 12 nicht sichtbare Verbindungsleitung kommuniziert die Luftpumpe ausgangsseitig über ein weiteres Absperrventil in gleicher Weise auch mit dem zweiten Verbindungsstück 419.2. Damit kann Luft direkt in die beiden Verbindungsstücke 419.1, 419.2 eingeleitet werden, um z. B. die Fluidkanäle der medizintechnischen Hohlinstrumente 418.1, 418.2 nach dem Reinigungsvorgang von Reinigungsflüssigkeit zu befreien.

Auch die vierte Reinigungsapparatur 400 verfügt über eine Steuereinheit mit einem Mikroprozessor, einer Speichereinheit und einer Zeitmesseinrichtung. Sämtliche Absperrventile 416.1...416.6, Sensoreinheiten 407, 408, Durchflussmessgeräte 409.1, 409.2, Pumpen 405, 422.1, 422.2, die Ausgabevorrichtung 412 bzw. die Flüssigkristallanzeige und die Tastatur 413 sind über entsprechende Signalleitungen mit der Steuereinheit verbunden. Damit ist eine vollautomatische Steuerung der vierten Reinigungsapparatur 400 möglich. Der Übersichtlichkeit halber wurde in den Fig. 11 und 12 aber auf eine Darstellung der entsprechenden Komponenten verzichtet.

Die Ansteuerung der vierten Reinigungsapparatur 400 erfolgt in analoger Weise, wie bei der ersten Reinigungsapparatur 100.

Während einem Reinigungs- und/oder Desinfektionsvorgang ist die erste Pumpe 405, ausser bei einer Störung, ständig in Betrieb und fördert kontinuierlich Reinigungsflüssigkeit durch die Aufnahmevorrichtung 406 und die medizinischen Hohlinstrumente 418.1, 418.2.

Zur Reinigung der medizintechnischen Geräte 418.1...418.5 wird der Aufnahmebereich 406 in einem ersten Schritt bevorzugt nahezu vollständig mit Reinigungsflüssigkeit gefüllt. Die zu reinigenden medizintechnischen Geräte 418.1...418.5 sind dadurch vollständig in Reinigungsflüssigkeit eingetaucht und werden von dieser umspült. Damit werden insbesondere gut lösliche Verunreinigungen von den zu reinigenden medizintechnischen Geräten 418.1...418.5 entfernt. Sollten noch schwerlösliche Verunreinigungen vorliegen, werden diese zudem aufgeweicht und teilweise angelöst.

In einem nächsten Schritt wird der Aufnahmebereich 406 bevorzugt entleert, so dass die zu reinigenden medizintechnischen Geräte 418.1...418.5 frei liegen. Die Flüssigkeitsteilchen der Sprühstrahlen 436, welche aus den Spritzdüsensätzen 420.1...420.4 austreten, treffen dabei mit hoher kinetischer Energie auf die zu reinigenden medizintechnischen Geräte 418.1...418.5, da in den Bereichen vor den Spritzdüsensätzen 420.1...420.4 keine Reinigungsflüssigkeit vorliegt, welche die Bewegung der Flüssigkeitsteilchen in den Sprühstrahlen 436 abbremsen würde. Dies ermöglicht insbesondere eine effizientere mechanische Entfernung von schwerlöslichen Schmutzpartikeln von den zu reinigenden medizintechnischen Geräten 418.1...418.5.

Für die Desinfektion der medizintechnischen Geräte 418.1...418.5 wird der Aufnahmebereich 406 dann bevorzugt mit Reinigungsflüssigkeit gefüllt, so das die zu reinigenden medizintechnischen Geräte 418.1...418.5 vollständig in die Reinigungsflüssigkeit eintauchen. Damit wird sichergestellt, dass die zu reinigenden medizintechnischen Geräte 418.1...418.5 vollständig mit Reinigungsflüssigkeit benetzt werden.

Der Füllstand im Aufnahmebereich 406 kann z. B. durch Zugabe von Reinigungsflüssigkeit durch die Einlassleitung 424 und das fünfte Absperrventil 416.5 erfolgen. Entsprechend kann der Füllstand im Aufnahmebereich 406 durch Ablassen von Reinigungsflüssigkeit über das vierte Absperrventil und die Auslassleitung 425 erreicht werden.

Es ist aber grundsätzlich auch möglich, während dem gesamten Reinigungs- und/oder Desinfektionsvorgang den Aufnahmebereich 406 nahezu vollständig mit Reinigungsflüssigkeit befüllt zu belassen. Der Aufnahmebereich kann dabei insbesondere auch als Ausgleichsgefäss dienen.

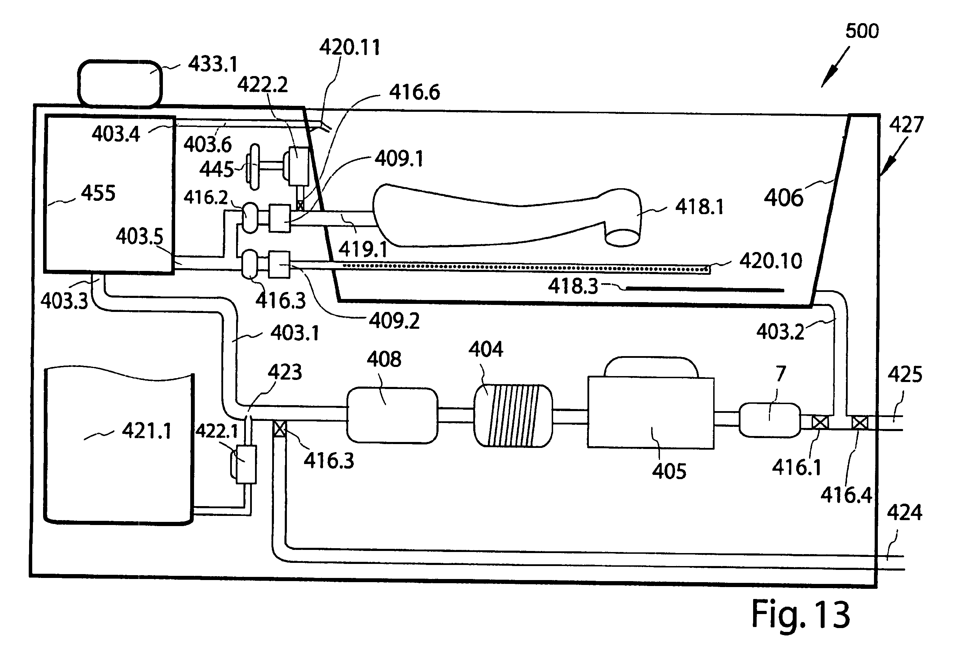

Fig. 13 zeigt eine schematische Darstellung einer fünften Reinigungsapparatur 500. Die fünfte Reinigungsapparatur 500 entspricht im Wesentlichen der vierten Reinigungsapparatur 400. Im Unterschied zur vierten Reinigungsapparatur 400 ist bei der fünften Reinigungsapparatur 500 jedoch im linken und oberen Gehäusebereich neben dem Aufnahmebereich 406 zusätzlich ein Zwischenreservoir 455 für die Reinigungsflüssigkeit angeordnet. Das Zwischenrohr 403.1 mündet dabei direkt in den im Bodenbereich des Zwischenreservoirs 455 angebrachten Einlassstutzen 403.3. Im Bereich des oberen Endes des Zwischenreservoirs 455 ragt ein erster Auslassstutzen 403.4 nach rechts zum Aufnahmebereich 406 aus dem Zwischenreservoir 455 heraus. Der erste Auslassstutzen 403.4 mündet in eine oberste Zufuhrleitung 403.6, welche in vertikaler Richtung in den Aufnahmebereich 406 hinein ragt und am vordersten Ende in eine obere Spritzdüse 420.11 übergeht.

Im Bereich des unteren Endes des Zwischenreservoirs 455 ragt ein zweiter Auslassstutzen 403.5 nach rechts aus dem Zwischenreservoir, welcher mit dem zweiten Absperrventil 416.2 und dem dritten Absperrventil 416.3 über ein T-förmiges Rohrstück kommunizierend verbunden ist. Bei geöffnetem drittem Absperrventil 416.3 ist damit der Aufnahmebereich 406 mit dem Zwischenreservoir 455 kommunizierend verbunden.

Der Bodenbereich des Zwischenreservoirs 455 liegt dabei auf einer Höhe über dem Boden des Aufnahmebereichs 406 und knapp unterhalb der Höhe der Spritzdüsensätze 420.1...420.4 bzw. der Versorgungsleitung 420.10.

Sämtliche Reinigungsflüssigkeit, welche von der Pumpe 405 durch die Heizeinheit 404 und die Sensoreinheit 408 zur Bestimmung des physikalischen Zustands gefördert wird, gelangt daher zuerst in das Zwischenreservoir 455 und von da aus über wenigstens einen der beiden Auslassstutzen 403.4, 403.5 des Zwischenreservoirs 455 in den Aufnahmebereich 406 und/oder in die Verbindungsstücke 419.1, 419.2 .

Bei ausgeschalteter Pumpe 405 oder bei einem Betrieb der Pumpe 405 mit geringer Förderleistung entspricht die Füllhöhe im Zwischenreservoir 455 bei geöffnetem zweitem Absperrventil 416.2 und geöffnetem drittem Absperrventil 416.3 im Wesentlichen der Füllhöhe im Aufnahmebereich 406. Allfällige temporär auftretenden Unterschiede der Füllhöhen zwischen dem Aufnahmebereich 406 und dem Zwischenreservoir 455 werden durch Gravitationskräfte bzw. Druckunterschiede automatisch ausgeglichen. Die gesamte Menge an Reinigungsflüssigkeit in der fünften Reinigungsapparatur wird bevorzugt so gewählt, dass die zu reinigenden medizintechnischen Geräte 418.1...418.5 bei ausgeschalteter Pumpe 405 und geöffnetem zweitem Absperrventil 416.2 und geöffnetem drittem Absperrventil 416.3 vollständig in die Reinigungsflüssigkeit eintauchen.

Zur Durchführung eines Reinigungsprozesses wird die Förderleistung der Pumpe 405 erhöht. Damit steigt die Füllhöhe im Zwischenreservoir 455 auch bei geöffnetem zweitem Absperrventil 416.2 und geöffnetem drittem Absperrventil 416.3 an, während sie im Aufnahmebereich 406 abnimmt. Dies ist dadurch bedingt, dass die in den Aufnahmebereich 406 mündenden Zuleitungen insgesamt eine geringere Querschnittsfläche aufweisen als das Abflussrohr 403.2 und die zur Pumpe 405 führenden Verbindungen. Schlussendlich werden die zu reinigenden medizintechnischen Geräte 418.1...418.5 im Aufnahmebereich 406 freigelegt bzw. die medizintechnischen Geräte 418.1...418.5 sind nicht mehr in die Reinigungsflüssigkeit eingetaucht. Nahezu die gesamte Reinigungsflüssigkeit liegt dabei im Zwischenreservoir 455 vor. Aufgrund des oben angeordneten ersten Auslassstutzen 403.4 kann die im Zwischenreservoir 455 vorliegende Luft während dem Befüllen des Zwischenreservoirs 455 problemlos über die oberste Zufuhrleitung 403.6 und die obere Spritzdüse 420.11 entweichen.

Sobald das Zwischenreservoir 455 vollständig gefüllt ist, wird die Reinigungsflüssigkeit durch beide Auslassstutzen 403.4, 403.5 geleitet und über die Spritzdüsensätze 420.1...420.4 die obere Spritzdüse 420.11 und die Verbindungsstücke 419.1, 419.2 auf und in die zu reinigenden medizintechnischen Geräte 481.1...481.5 gespritzt.

Nach erfolgtem Reinigungsprozess wird die Förderleistung der Pumpe wieder reduziert, womit grundsätzlich mehr Reinigungsflüssigkeit vom Zwischenreservoir 406 in den Aufnahmebereich 406 gelangt als aus diesem heraus gefördert wird. Damit tauchen die zu reinigenden medizintechnischen Geräte 418.1...418.5 mit der Zeit wieder in die Reinigungsflüssigkeit ein. Für den eigentlichen Desinfektionsvorgang wird die Förderleistung der Pumpe 405 dann so eingestellt, dass die Füllhöhe im Aufnahmebereich 406 konstant bleibt und die Reinigungsflüssigkeit durch die fünfte Reinigungsapparatur 500 zirkuliert, so dass insbesondere die zu reinigenden medizintechnischen Geräte 418.1...418.5 eingetaucht vorliegen.

Die vorstehend beschriebenen Ausführungsbeispiele sind lediglich als illustrative Beispiele zu verstehen, welche im Rahmen der Erfindung beliebig abgeändert werden können.

Bei der ersten Reinigungsapparatur 100 kann z. B. auch auf das Auffanggefäss 2 verzichtet werden und stattdessen das Ablaufrohr 3.5 verlängert und mit einem externen Sammelkanister oder einer Abwasserleitung verbunden werden. Ebenso kann das Regulierventil 16.10 in Fig. 1 auch manuell betätigbar ausgebildet sein, womit auf die Steuerleitung zwischen Regulierventil 16.10 und Steuereinheit 14 verzichtet werden kann. Diese Abwandlungen können entsprechend auch bei der dritten Reinigungsapparatur 300 vorgesehen werden.

Bei allen vier Reinigungsapparaturen 100, 200, 300, 400 können auch Kommunikationsschnittstellen angebracht werden, welche eine Datenübertragung von der Reinigungsapparatur zu einem Drittgerät und/oder vom Drittgerät zur Reinigungsapparatur ermöglichen. Das Drittgerät kann z. B. ein externer Computer, eine Datenspeichervorrichtung (USB-Stick, Festplatte) oder ein Drucker sein. Hierfür werden zusätzliche Signalleitungen zwischen Steuereinheit und den besagten Kommunikationsschnittstellen angeordnet. Die Kommunikationsschnittstellen können zur drahtgebundenen Datenübertragung und/oder drahtlosen Datenübertragung mit elektromagnetischer Strahlung ausgebildet sein.

Grundsätzlich können die Reinigungsapparaturen 100, 200, 300, 400 auch durch einen über die Kommunikationsschnittstellen verbundenen externen Computer gesteuert werden. Dies kann z. B. für Wartungsarbeiten vorteilhaft sein. Es ist aber auch möglich, die eigentlichen Reinigungs- und/oder Desinfektionsvorgänge durch den externen Computer zu steuern. In diesem Fall kann auch auf Eingabe- und Ausgabevorrichtungen an der Reinigungsapparatur verzichtet werden.

Bei der in Fig. 2 dargestellten Sensoreinheit 7 zur Bestimmung der stofflichen Beschaffenheit können die Sensoren 7.1, 7.2, 7.3 auch in einer parallelen Anordnung vorliegen, z. B. in drei separaten Rohrabschnitten. Ebenso können auch weniger oder mehr als die drei Sensoren 7.1, 7.2, 7.3 vorliegen.

Entsprechend kann auch die in Fig. 8 abgebildete Sensoreinheit eine parallele Anordnung der zwei Sensoren 8.1, 8.2 aufweisen. Auch hier können zusätzliche Sensoren angebracht werden.

Es ist auch denkbar Sensoreinheiten vorzusehen, welche z. B. zur Bestimmung der Konzentration von Zellen, Blut, Proteinen, Bakterien oder physiologischen Flüssigkeiten in der Reinigungsflüssigkeit ausgebildet sind und welche zusätzlich und/oder anstelle der bereits beschriebenen Sensoreinheiten 7, 8, 307, 407, 307, 308 angebracht werden.

Sämtliche Sensoreinheiten können beispielsweise auch in den Aufnahmebereichen für die medizintechnischen Geräte oder in den Abflussrohren integriert werden, um die Wirksamkeit des Reinigungsvorgangs zu kontrollieren.

Um die vier Reinigungsapparaturen 100, 200, 300, 400 selbst zu reinigen, können diese beispielsweise mit einer speziellen Reinigungsflüssigkeit gespült werden. Mit den Sensoreinheiten 7, 407 zur Bestimmung der stofflichen Beschaffenheit kann dabei der Reinigungsvorgang insbesondere bei der zweiten Reinigungsapparatur 200, der vierten Reinigungsapparatur 400 und der fünften Reinigungsapparatur 500, welche alle über einen geschlossenen Flüssigkeitskreislauf verfügen, überprüft werden. Dabei kann z. B. die Lichtabsorption durch den Fotometer 7.3 des für die Spülung verwendeten Reinigungsmittels kontinuierlich gemessen werden, so dass überprüft werden kann, ob sich die Reinigungsflüssigkeit während dem Reinigungsvorgang verändert bzw. ob während dem Reinigungsvorgang Schmutz und/oder Verunreinigungen von der Reinigungsapparatur in die Reinigungsflüssigkeit gelangen. Hierfür kann aber grundsätzlich auch der Leitfähigkeitsensor 7.1 oder der pH-Sensor 7.3 der Sensoreinheit 7, 407 zur Bestimmung der stofflichen Beschaffenheit verwendet werden.

Die universelle Pumpe 22 in Fig. 1 kann z. B. auch aufgrund eines Signals der Sensoreinheit 7 zur Bestimmung der stofflichen Beschaffenheit automatisch ein Konzentrat aus dem weiteren Flüssigkeitsbehälter 21 in das Ansaugrohr 3.1 einspeisen. Im sechsten Verfahrensschritt 806 des in Fig. 4 dargestellten Reinigungsverfahrens kann in diesem Fall bei einer Abweichung von einem der drei durch die Sensoreinheit 7 ermittelten Messwerte vom zugehörigen Vergleichswert das Konzentrat aus dem weiteren Flüssigkeitsbehälter 21 in das Ansaugrohr 3.1 eingespeist werden. Falls die Beschaffenheit der Reinigungsflüssigkeit durch diese Massnahme ausreichend verbessert werden kann, kann mit dem achten Verfahrensschritt 808 weitergefahren werden, ohne dass der Reinigungsvorgang direkt abgebrochen werden muss.

Eine derartig ausgebildete Steuereinheit, welche aufgrund eines Signals der Sensoreinheit zur Bestimmung der Beschaffenheit der Reinigungsflüssigkeit eine Modifikation der Reinigungsflüssigkeit kontrolliert, kann beispielsweise auch bei der zweiten Reinigungsapparatur 200 oder der vierten Reinigungsapparatur 400 angeordnet sein.

Bei der ersten Reinigungsapparatur 100 ist es auch möglich, sämtliche von der Steuereinheit 14 erfassten Messwerte und/oder gesendeten Steuerbefehle beispielsweise mit einem durch die Zeitmesseinrichtung 9.3 ermittelten Zeitstempel zu versehen und im Datenspeicher 11 abzulegen. Damit kann später genau zurückverfolgt werden, zu welchem Zeitpunkt die Reinigungsflüssigkeit in einem bestimmten Zustand vorlag. Dies erleichtert insbesondere die Fehlersuche.

Bei der zweiten Reinigungsapparatur 200 kann beispielsweise auch ein zusätzliches Ausgleichsgefäss, z. B. im Bereich des Verbindungsrohrs 3.12, vorgesehen werden.

Der Aufnahmebereich 306 der dritten Reinigungsapparatur 300 kann grundsätzlich auch so ausgebildet sein, dass zusätzlich oder anstelle der sechs medizintechnischen Hohlinstrumente 318.1...318.6 auch andere medizintechnische Geräte in den Aufnahme bereich 306 eingelegt und gereinigt werden können, wie dies z. B. bei der vierten Reinigungsapparatur 400 der Fall ist.

Je nach zu reinigenden medizintechnischen Geräten können die flexiblen Schläuche 310.1...310.6 aus Fig. 10.1 und 10.2 auch zusätzliche Fluidkanäle beinhalten. Die Verbindungsstücke 319.10, 319.11, 319.20, 319.21, 319.30, 319.31 weisen dann entsprechend ebenfalls zusätzliche Fluidleitungen auf.

Die flexiblen Schläuche 310.1...310.6 können grundsätzlich auch durch starre Rohrstücke ersetzt werden oder über starre Rohrabschnitte verfügen.

Insbesondere bei der dritten Reinigungsapparatur 300 ist es auch möglich, zu den bestehenden beiden Flüssigkeitsbehältern 301.1, 301.2 noch weitere Flüssigkeitsbehälter mit Pumpen und Sensoreinheiten zur Bestimmung der stofflichen Beschaffenheit vorzusehen. Entsprechend können in diesem Fall die medizintechnischen Geräte mit wenigstens drei verschiedenen Reinigungsflüssigkeiten behandelt werden. Die Reihenfolge der Anwendung der wenigstens drei unterschiedlichen Reinigungsflüssigkeiten kann dann durch die Steuereinheit kontrolliert werden.

Bei der fünften Reinigungsapparatur 500 ist es auch möglich, das Zwischenreservoir 455 so anzuordnen, dass der Bodenbereich in einer Höhe über den zu reinigenden medizintechnischen Geräten 418.1...418.5 zu liegen kommt. Damit kann bei ausgeschalteter Pumpe 405 oder bei einer geringen Förderleistung der Pumpe 405 das Zwischenreservoir 455 zum vollständigen Eintauchen der zu reinigenden medizintechnischen Gräte 418.1...418.5 im Wesentlichen entleert werden.

Zusammenfassend ist festzustellen, dass mehrere Reinigungsapparaturen geschaffen wurden, welche die Einhaltung von Reinigungs- und/oder Desinfektionsrichtlinien bei medizintechnischen Geräten in hohem Masse garantieren und die Validierung von vorgeschriebenen Reinigungs- und/oder Desinfektionsverfahren ermöglichen. Mit den erfindungsgemässen Geräten und Verfahren kann insbesondere sichergestellt werden, dass bei jedem einzelnen Reinigungs- und/oder Desinfektionsvorgang die zu reinigenden medizintechnischen Geräte mit einer genau definierten Reinigungsflüssigkeit und während einer vorgeschriebenen Zeitdauer bei genau definierten Bedingungen, insbesondere bei vorgegebenem Druck und Temperatur, behandelt werden.

Die so durchgeführten Reinigungs- und/oder Desinfektionsvorgänge können zudem im Detail dokumentiert werden. Damit wird eine eigentliche Validierung der Reinigungsvorgänge möglich, wobei die während der Reinigungs- und/oder Desinfektionsvorgänge ermittelten Messdaten der Sensoreinheiten und die an die verschiedenen Komponenten der Reinigungsapparaturen gesendeten Steuerbefehle in gedruckter Form auf Papier und/oder als elektronische Datei auf einem Datenträger abgelegt werden können, so dass beispielsweise eine Qualitätskontrolle ermöglicht wird.

## Patentansprüche

1. Reinigungsapparat (100) zum Reinigen und/oder Desinfizieren wenigstens eines medizintechnischen Geräts (18.1), insbesondere eines zahnmedizinischen Hohlinstruments wie ein Hand- und/oder ein Winkelstück, mit einer Reinigungsflüssigkeit, wobei der Reinigungsapparat (100) wenigstens eine Steuereinheit (14) und eine Zufuhrvorrichtung zur Zuleitung der Reinigungsflüssigkeit in einen Aufnahmebereich (6) und/oder eine Kupplungsvorrichtung (19.1) für das zu reinigende medizintechnische Gerät umfasst und wobei die Zufuhrvorrichtung über wenigstens eine erste Sensoreinheit (8) zur Bestimmung eines physikalischen Zustands der Reinigungsflüssigkeit verfügt, **dadurch gekennzeichnet, dass** die Zufuhrvorrichtung wenigstens eine zweite Sensoreinheit (7) zur Bestimmung der stofflichen Beschaffenheit der Reinigungsflüssigkeit aufweist.

2. Reinigungsapparat (100) nach Anspruch 1, **dadurch gekennzeichnet, dass** die zweite Sensoreinheit (7) zur Bestimmung eines pH-Werts und/oder einer elektrischen Leitfähigkeit und/oder einer Absorption von elektromagnetischer Strahlung der Reinigungsflüssigkeit ausgebildet ist.

3. Reinigungsapparat (100) nach einem der Ansprüche 1 - 2, **dadurch gekennzeichnet, dass** die erste Sensoreinheit (8) zur Bestimmung einer Temperatur und/oder eines Drucks der Reinigungsflüssigkeit ausgebildet ist.

4. Reinigungsapparat (100) nach einem der Ansprüche 1 - 3, **dadurch gekennzeichnet, dass** die Zufuhrvorrichtung über wenigstens eine dritte Sensoreinheit zur Bestimmung eines Strömungsparameters der Reinigungsflüssigkeit verfügt, wobei die dritte Sensoreinheit insbesondere ein Durchflussmessgerät (9.1, 9.2) umfasst.

5. Reinigungsapparat (100) nach einem der Ansprüche 1 - 4, **dadurch gekennzeichnet, dass** die Zufuhrvorrichtung eine Einrichtung (4, 5) zur Veränderung des physikalischen Zustands der Reinigungsflüssigkeit aufweist, insbesondere eine Pumpe (5) und/oder eine Heizung (4).

6. Reinigungsapparat (100) nach Anspruch 5, **dadurch gekennzeichnet, dass** die Steuereinheit (14) derart ausgebildet ist, dass die Einrichtung (4, 5) zur Veränderung des physikalischen Zustands der Reinigungsflüssigkeit aufgrund eines Signals der ersten Sensoreinheit (8) zur Bestimmung des physikalischen Zustands der Reinigungsflüssigkeit steuerbar ist.

7. Reinigungsapparat (100) nach einem der Ansprüche 1 - 6, **dadurch gekennzeichnet, dass** die Steuereinheit (14) über eine Zeitmesseinrichtung (9.3) verfügt und/oder mit einer Zeitmesseinrichtung (9.3) verbunden ist.

8. Reinigungsapparat (100) nach einem der Ansprüche 1 - 7, **dadurch gekennzeichnet, dass** eine Kommunikationsschnittstelle (14.1) angeordnet ist, welche insbesondere zum Datenaustausch mit einem Computer und/oder einem externen Datenspeicher vorgesehen ist.

9. Reinigungsapparat (100) nach einem der Ansprüche 1 - 8, **dadurch gekennzeichnet, dass** die Kupplungsvorrichtung (19.1) für das zu reinigende medizintechnische Gerät (18.1) sowohl nom Reinigungsapparat (100) selbst, als auch von dem zu reinigenden medizintechnischen Gerät (18.1) an-und abkoppelbar ausgebildet ist und dass insbesondere die Kupplungsvorrichtung (19.1) zur Erkennung des angekoppelten medizintechnischen Geräts (18.1) über einen Kupplungssensor verfügt.

10. Reinigungsapparat (100) nach Anspruch 9, **dadurch gekennzeichnet, dass** die Steuereinheit (14) derart ausgebildet ist, dass aufgrund eines Signals des Kupplungssensors die Zuleitung der Reinigungsflüssigkeit zu einem nicht korrekt angekoppelten medizintechnischen Gerät (18.1) unterbunden wird.

11. Reinigungsapparat (100) nach einem der Ansprüche 1 - 10, **dadurch gekennzeichnet, dass** die Kupplungsvorrichtung (19.1) über mehrere Fluidleitungen (303.11, 303.12) verfügt, welche insbesondere zur Kommunikation mit unterschiedlichen Fluidkanälen des zu reinigenden medizintechnischen Geräts vorgesehen sind und dass die Kupplungsvorrichtung (19.1) bevorzugt über einen mechanischen Antrieb (349) zur Bewegung von drehenden Teilen der medizintechnischen Geräte (18.1) während dem Reinigungs- und/oder Desinfektionsvorgang verfügt.

12. Reinigungsapparat (100) nach einem der Ansprüche 1 - 11, **dadurch gekennzeichnet, dass** die Kupplungsvorrichtung (19.1) über eine Anschlussstelle (354.3) für ein Adapterstück (319.40), welches die Kupplungsvorrichtung (19.1) und das zu reinigende medizintechnische Gerät (318.5) miteinander verbindet, verfügt.

13. Reinigungsapparat (100) nach einem der Ansprüche 1-12, **dadurch gekennzeichnet, dass** ein geschlossener Flüssigkeitskreislauf für die Reinigungsflüssigkeit vorliegt, so dass die Reinigungsflüssigkeit während dem Reinigungs- und/oder Desinfektionsvorgang im Flüssigkeitskreislauf zirkulieren kann.

14. Reinigungsapparat (100) nach Anspruch 13, **dadurch gekennzeichnet, dass** zur Kontrolle eines Füllstandes der Reinigungsflüssigkeit im Aufnahmebereich (406) ein Zwischenreservoir (455) im Flüssigkeitskreislauf angeordnet ist.

15. Verfahren zum Reinigen und/oder Desinfizieren eines medizintechnischen Geräts (18.1), insbesondere eines zahnmedizinischen Hohlinstruments wie ein Hand- und/oder ein Winkelstück, mit einem Reinigungsapparat (100), insbesondere mit einem Reinigungsapparat nach einem der Ansprüche 1 - 14, wobei eine Reinigungsflüssigkeit über eine Zufuhrvorrichtung in einen Aufnahmebereich (6) und/oder eine Kupplungsvorrichtung (19.1) für das zu reinigende medizintechnische Gerät (18.1) geleitet wird und über wenigstens eine erste Sensoreinheit (8) der Zufuhrvorrichtung ein physikalischer Zustand der Reinigungsflüssigkeit gemessen und in einer Steuereinheit (14) des Reinigungsapparats (100) erfasst wird und wobei die Reinigungsflüssigkeit mit einer Aussenseite und/oder einer Innenseite des medizintechnischen Geräts (18.1) in Kontakt gebracht wird, **dadurch gekennzeichnet, dass** eine stoffliche Beschaffenheit der Reinigungsflüssigkeit in der Zufuhrvorrichtung durch eine zweite Sensoreinheit (7) gemessen wird.

16. Verfahren nach Anspruch 15, **dadurch gekennzeichnet, dass** ein durch die zweite Sensoreinheit (7) gemessenes Messsignal in der Steuereinheit (14) des Reinigungsapparats (100) mit wenigstens einem in der Steuereinheit (14) abgelegten ersten Vergleichswert verglichen wird.

17. Verfahren nach Anspruch 16, **dadurch gekennzeichnet, dass** die Messung der stofflichen Beschaffenheit der Reinigungsflüssigkeit und der Vergleich mit dem in der Steuereinheit (14) abgelegten ersten Vergleichswert mehrmals, insbesondere kontinuierlich während der Zuleitung der Reinigungsflüssigkeit erfolgt.

18. Verfahren nach einem der Ansprüche 16 - 17, **dadurch gekennzeichnet, dass** die Zufuhrvorrichtung bei einer vorgegebenen Abweichung des durch die zweite Sensoreinheit (7) gemessenen Messsignals vom ersten Vergleichswert die Zuleitung der Reinigungsflüssigkeit in den Aufnahmebereich (6) und/oder in die Kupplungsvorrichtung (19.1) für das zu reinigende medizintechnische Gerät (100) unterbindet.

19. Verfahren nach einem der Ansprüche 16-18, **dadurch gekennzeichnet, dass** durch die erste Sensoreinheit (8) zur Messung des physikalischen Zustands der Reinigungsflüssigkeit eine Temperatur und/oder ein Druck der Reinigungsflüssigkeit gemessen wird.

20. Verfahren nach einem der Ansprüche 16 - 19, **dadurch gekennzeichnet, dass** über wenigstens eine dritte Sensoreinheit (9.1, 9.3) der Zufuhrvorrichtung ein Strömungsparameter der Reinigungsflüssigkeit, insbesondere ein Durchfluss der Reinigungsflüssigkeit, gemessen und von der Steuereinheit (14) der Reinigungsapparatur (100) erfasst wird.

21. Verfahren nach Anspruch 20, **dadurch gekennzeichnet, dass** der gemessene physikalischen Zustand der Reinigungsflüssigkeit in der Steuereinheit (14) mit einem zweiten Vergleichswert und/oder der gemessene Strömungsparameter der Reinigungsflüssigkeit in der Steuereinheit (14) mit einem dritten Vergleichswert verglichen wird, wobei bei einer vorgegebenen Abweichung des gemessenen physikalischen Zustands der Reinigungsflüssigkeit vom zweiten Vergleichswert und/oder bei einer vorgegebenen Abweichung des gemessenen Strömungsparameters der Reinigungsflüssigkeit vom dritten Vergleichswert durch die Steuereinheit (14) ein Betriebszustand einer Vorrichtung zur Veränderung des physikalischen Zustands der Reinigungsflüssigkeit in der Zufuhrvorrichtung, insbesondere eine Pumpe (5) und/oder einer Heizung (4), angepasst wird.

22. Verfahren nach einem der Ansprüche 15 - 21, **dadurch gekennzeichnet, dass** die Aussenseite und/oder die Innenseite des medizintechnischen Geräts (18.1) während einer durch die Steuereinheit (14) kontrollierten und vorbestimmten Zeitdauer mit der Reinigungsflüssigkeit in Kontakt gebracht wird.

## Claims

1. A cleaning device (100) for cleaning and/or disinfecting at least one medical device (18.1), particularly a hollow dental instrument such as a handle and/or angle section, by means of a cleaning fluid, the cleaning device (100) comprising at least one controller (14) and one infeed device for feeding the cleaning fluid into a receptacle area (6) and/or a coupling device (19.1) for the medical device to be cleaned, and the infeed device having at least one first sensor unit (8) for determining a physical state of the cleaning fluid, **characterized in that** the infeed device comprises at least one second sensor unit (7) for determining the material quality of the cleaning fluid.

2. The cleaning device (100) according to claim 1, **characterized in that** the second sensor unit (7) is implemented for determining a pH value and/or an electrical conductivity and/or an absorption of electromagnet radiation for the cleaning fluid.

3. The cleaning device (100) according to one of the claims 1 - 2, **characterized in that** the first sensor unit (8) is implemented for determining a temperature and/or a pressure of the cleaning fluid.

4. The cleaning device (100) according to one of the claims 1 - 3, **characterized in that** the infeed device comprises at least one third sensor unit for determining a flow parameter of the cleaning fluid, wherein the third sensor unit particularly comprises a flow measuring device (9.1, 9.2).

5. The cleaning device (100) according to one of the claims 1 - 4, **characterized in that** the infeed device comprise a device (4, 5) for changing the physical state of the cleaning fluid, particularly a pump (5) and/or a heater (4).

6. The cleaning device (100) according to claim 5, **characterized in that** the controller (14) is implemented such that the device (4, 5) for changing the physical state of the cleaning fluid can be controlled on the basis of a signal from the first sensor unit (8) for determining the physical state of the cleaning fluid.

7. The cleaning device (100) according to one of the claims 1 - 6, **characterized in that** the controller (14) has a time measurement device (9.3) and/or is connected to a time measurement device (9.3).

8. The cleaning device (100) according to one of the claims 1 - 7, **characterized in that** a communications interface (14.1) is provided particularly for data exchange with a computer and/or an external data store.

9. The cleaning device (100) according to one of the claims 1 - 8, **characterized in that** the coupling device (19.1) for the medical device (18.1) to be cleaned is implemented to be able to couple to and decouple from both the cleaning device (100) itself and the medical device (18.1) to be cleaned, and that the coupling device (19.1) in particular has a coupling sensor for detecting the coupled medical device (18.1).

10. The cleaning device (100) according to claim 9, **characterized in that** the controller (14) is implemented such that the feeding of the cleaning fluid to a medical device (18.1) that is not correctly coupled is prevented on the basis of a signal from the coupling sensor.

11. The cleaning device (100) according to one of the claims 1 - 10, **characterized in that** the coupling device (19.1) comprises a plurality of fluid lines (303.11, 303.12) provided particularly for communication with different fluid channels of the medical device to be cleaned, and that the coupling device (19.1) preferably comprises a mechanical drive (349) for moving rotating parts of the medical devices (18.1) during the cleaning and/or disinfection process.

12. The cleaning device (100) according to one of the claims 1 - 11, **characterized in that** the coupling device (19.1) comprises a connection point (354.3) for an adapter piece (319.40) connecting the coupling device (19.1) and the medical device (318.5) to be cleaned to each other.

13. The cleaning device (100) according to one of the claims 1 - 12, **characterized in that** a closed fluid circuit is present for the cleaning fluid, so that the cleaning fluid can circulate in the fluid circuit during the cleaning and/or disinfection process.

14. The cleaning device (100) according to claim 13, **characterized in that** an intermediate reservoir (455) is disposed in the fluid circuit for checking a fill level of the cleaning fluid in the receptacle area (406).

15. A method for cleaning and/or disinfecting a medical device (18.1), particularly a hollow dental instrument such as a handle and/or an angle section, by means of a cleaning device (100), particularly by means of a cleaning device according to one of the claims 1 - 14, a cleaning fluid being fed through an infeed device into a receptacle area (6) and/or a coupling device (19.1) for the medical device (18.1) to be cleaned, and a physical state of the cleaning fluid being measured by at least one first sensor unit (8) of the infeed device and captured in a controller (14) of the cleaning device (100), and wherein the cleaning fluid is brought into contact with an exterior and/or an interior of the medical device (18.1), **characterized in that** a material quality of the cleaning fluid is measured in the infeed device by a second sensor unit (7).

16. The method according to claim 15, **characterized in that** a measurement signal measured by the second sensor unit (7) is compared in the controller (14) of the cleaning device (100) with at least one first comparison value stored in the controller (14).

17. The method according to claim 16, **characterized in that** the measurement of the material quality of the cleaning fluid and the comparison with the first comparison value stored in the controller (14) takes place repeatedly, particularly continuously, during the feeding of the cleaning fluid.

18. The method according to one of the claims 16 - 17, **characterized in that** the infeed device prevents the feeding of the cleaning fluid into the receptacle area (6) and/or into the coupling device (19.1) for the medical device to be cleaned (100) in case of a specified deviation of the measurement signal measured by the second sensor unit (7) from the first comparison value.

19. The method according to one of the claims 16 - 18, **characterized in that** a temperature and/or a pressure of the cleaning fluid is measured by the first sensor unit (8) for measuring the physical state of the cleaning fluid.

20. The method according to one of the claims 16 - 19, **characterized in that** a flow parameter of the cleaning fluid, particularly a flow rate of the cleaning fluid, is measured by means of at least one third sensor unit (9.1, 9.3) of the infeed device and is captured by the controller (14) of the cleaning device (100).

21. The method according to claim 20, **characterized in that** the measured physical state of the cleaning fluid is compared in the controller (14) with a second comparison value and/or the measured flow parameter of the cleaning fluid is compared in the controller (14) with a third comparison value, wherein an operating state of a device for changing the physical state of the cleaning fluid in the infeed device, particularly a pump (5) and/or a heater (4), is adjusted by the controller (14) in the case of a specified deviation of the measured physical state of the cleaning fluid from the second comparison value and/or in the case of a specified deviation of the measured flow parameter of the cleaning fluid from the third comparison value.

22. The method according to one of the claims 15 - 21, **characterized in that** the exterior and/or the interior of the medical device (18.1) is brought into contact with the cleaning fluid during a time period monitored and predetermined by the controller (14).

## Revendications

1. Appareil de nettoyage (100) pour nettoyer et/ou désinfecter au moins un appareil médicotechnique (18.1), en particulier un instrument creux de médecine dentaire tel qu'une pièce à main et/ou un raccord angulaire, avec un liquide de nettoyage, sachant que l'appareil de nettoyage (100) comporte au moins une unité de commande (14) et un dispositif d'alimentation pour l'alimentation en liquide de nettoyage d'une zone de réception (6) et/ou d'un dispositif d'accouplement (19.1) pour l'appareil médicotechnique devant être nettoyé et sachant que le dispositif d'alimentation dispose d'au moins une première unité de capteur (8) pour déterminer un état physique du liquide de nettoyage, **caractérisé en ce que** le dispositif d'alimentation présente au moins une seconde unité de capteur (7) pour déterminer la constitution matérielle du liquide de nettoyage.

2. Appareil de nettoyage (100) selon la revendication 1, **caractérisé en ce que** la seconde unité de capteur (7) se présente sous une forme appropriée pour déterminer une valeur de pH et/ou une conductivité électrique et/ou une absorption de rayonnement électromagnétique du liquide de nettoyage.

3. Appareil de nettoyage (100) selon l'une des revendications 1-2, **caractérisé en ce que** la première unité de capteur (8) se présente sous une forme appropriée pour déterminée une température et/ou une pression du liquide de nettoyage.

4. Appareil de nettoyage (100) selon l'une des revendications 1-3, **caractérisé en ce que** le dispositif d'alimentation comporte au moins une troisième unité de capteur pour déterminer un paramètre d'écoulement du liquide de nettoyage, sachant que la troisième unité de capteur comprend en particulier un débitmètre (9.1,9.2).

5. Appareil de nettoyage (100) selon l'une des revendications 1-4, **caractérisé en ce que** le dispositif d'alimentation comporte un dispositif (4, 5) pour la modification de l'état physique du liquide de nettoyage, en particulier une pompe (5) et/ou un chauffage (4).

6. Appareil de nettoyage (100) selon la revendication 5, **caractérisé en ce que** l'unité de commande (14) se présente sous une forme telle que le dispositif (4, 5) pour la modification de l'état physique du liquide de nettoyage puisse être commande sur la base d'un signal de la première unité de capteur (8) en vue de la détermination de l'état physique du liquide de nettoyage.

7. Appareil de nettoyage (100) selon l'une des revendications 1-6, **caractérisé en ce que** l'unité de commande (14) comporte un chronomètre (9.3) et/ou est reliée à un chronomètre (9.3).

8. Appareil de nettoyage (100) selon l'une des revendications 1-7, **caractérisé en ce qu'**il y est disposé une interface de communication (14.1), qui est prévue en particulier pour l'échange de données avec un ordinateur et/ou une mémoire de données externe.

9. Appareil de nettoyage (100) selon l'une des revendications 1-8, **caractérisé en ce que** le dispositif d'accouplement (19.1) pour l'appareil médicotechnique (18.1) devant être nettoyé se présente sous une forme telle qu'il puisse être accouplé et découplé aussi bien de l'appareil de nettoyage (100) lui-même que de l'appareil médicotechnique (18.1) devant être nettoyé et qu'en particulier, le dispositif d'accouplement (19.1) comporte un capteur d'accouplement pour l'identification de l'appareil médicotechnique (18.1) accouplé.

10. Appareil de nettoyage (100) selon la revendication 9, **caractérisé en ce que** l'unité de commande (14) se présente sous une forme telle que sur la base d'un signal du capteur d'accouplement, l'alimentation en liquide de nettoyage d'un appareil médicotechnique (18.1) incorrectement accouplé soit arrêtée.

11. Appareil de nettoyage (100) selon l'une des revendications 1-10, **caractérisé en ce que** le dispositif d'accouplement (19.1) comporte plusieurs conduits de fluide (303.11, 303.12), qui sont prévus en particulier pour la communication avec différents canaux de fluides de l'appareil médicotechnique devant être nettoyé et que le dispositif d'accouplement (19.1) comporte de préférence un entraînement mécanique (349) pour faire mouvoir des pièces rotatives des appareils médicotechniques (18.1) durant le processus de nettoyage et/ou de désinfection.

12. Appareil de nettoyage (100) selon l'une des revendications 1-11, **caractérisé en ce que** le dispositif d'accouplement (19.1) comporte un point de raccordement (354.3) pour une pièce d'adaptation (319.40), qui relie l'un avec l'autre le dispositif d'accouplement (19.1) et l'appareil médicotechnique (318.5) devant être nettoyé.

13. Appareil de nettoyage (100) selon l'une des revendications 1-12, **caractérisé en ce qu'**il existe un circuit de liquide fermé pour le liquide de nettoyage, si bien que le liquide de nettoyage peut circuler dans le circuit de liquide durant le processus de nettoyage et/ou de désinfection.

14. Appareil de nettoyage (100) selon la revendication 13, **caractérisé en ce qu'**un réservoir intermédiaire (455) est disposé dans le circuit de liquide pour le contrôle d'un niveau de remplissage du liquide de nettoyage dans la zone de réception (406).

15. Procédé pour nettoyer et/ou désinfecter un appareil médicotechnique (18.1), en particulier un instrument creux de médecine dentaire tel qu'une pièce à main et/ou un raccord angulaire, à l'aide d'un appareil de nettoyage (100), en particulier à l'aide d'un appareil de nettoyage selon l'une des revendications 1-14, dans lequel un liquide de nettoyage est acheminé par un dispositif d'alimentation dans une zone de réception (6) et/ou un dispositif d'accouplement (19.1) pour l'appareil médicotechnique (18.1) devant être nettoyé et un état physique du liquide de nettoyage est mesuré par au moins une première unité de capteur (8) du dispositif d'alimentation et enregistré dans une unité de commande (14) de l'appareil de nettoyage (100), et sachant que le liquide de nettoyage est mis en contact avec une face extérieure et/ou une face intérieure de l'appareil médicotechnique (18.1), **caractérisé en ce qu'**une constitution matérielle du liquide de nettoyage est mesurée dans le dispositif d'alimentation par une seconde unité de capteur (7).

16. Procédé selon la revendication 15, **caractérisé en ce qu'**un signal de mesure mesuré par la seconde unité de capteur(7) est comparé dans l'unité de commande (14) de l'appareil de nettoyage (100) avec au moins une première valeur de référence stockée dans l'unité de commande (14).

17. Procédé selon la revendication 16, **caractérisé en ce que** la mesure de la constitution matérielle du liquide de nettoyage et la comparaison avec la première valeur de référence stockée dans l'unité de commande (14) sont effectuées plusieurs fois, en particulier de manière continue durant l'alimentation en liquide de nettoyage.

18. Procédé selon l'une des revendications 16-17, **caractérisé en ce que** le dispositif d'alimentation arrête l'alimentation en liquide de nettoyage de la zone de réception (6) et/ou du dispositif d'accouplement (19.1) pour l'appareil médicotechnique (100) devant être nettoyé en cas d'écart prédéfini du signal de mesure mesuré par la seconde unité de capteur (7) par rapport à la première valeur de référence.

19. Procédé selon l'une des revendications 16-18, **caractérisé en ce que** la première unité de capteur (8) pour la mesure de l'état physique du liquide de nettoyage mesure une température et/ou une pression du liquide de nettoyage.

20. Procédé selon l'une des revendications 16-19, **caractérisé en ce que** via au moins une troisième unité de capteur (9.1, 9.3) du dispositif d'alimentation, un paramètre d'écoulement du liquide de nettoyage, en particulier un débit du liquide de nettoyage, est mesuré et enregistré par l'unité de commande (14) de l'appareil de nettoyage (100).

21. Procédé selon la revendication 20, **caractérisé en ce que** l'état physique mesuré du liquide de nettoyage est comparé dans l'unité de commande (14) avec une seconde valeur de référence et/ou le paramètre d'écoulement mesuré du liquide de nettoyage est comparé dans l'unité de commande (14) avec une troisième valeur de référence, sachant qu'en cas d'écart prédéfini de l'état physique mesuré du liquide de nettoyage par rapport à la seconde valeur de référence et/ou en cas d'écart prédéfini du paramètre d'écoulement mesuré du liquide de nettoyage par rapport à la troisième valeur de référence, un état de fonctionnement d'un dispositif pour la modification de l'état physique du liquide de nettoyage dans le dispositif d'alimentation, en particulier une pompe (5) et/ou un chauffage (4), est adapté par l'unité de commande (14).

22. Procédé selon l'une des revendications 15-21, **caractérisé en ce que** la face extérieure et/ou la face intérieure de l'appareil médicotechnique (18.1) est mise en contact avec le liquide de nettoyage pendant une durée de temps contrôlée et prédéterminée par l'unité de commande (14).
